(19) Europäisches Patentamt
European Patent Office
Office européen des brevets

(11) EP 0 358 609 B1

(12) # EUROPÄISCHE PATENTSCHRIFT

(45) Veröffentlichungstag und Bekanntmachung des Hinweises auf die Patenterteilung: **24.07.1996 Patentblatt 1996/30**

(21) Anmeldenummer: **89810652.1**

(22) Anmeldetag: **01.09.1989**

(51) Int. Cl.$^6$: **C07D 239/42**, C07D 409/12, C07D 405/12, C07D 403/12, C07D 401/12, A01N 43/54

(54) **Schädlingsbekämpfungsmittel**
Parasiticide compounds
Parasiticides

(84) Benannte Vertragsstaaten:
**AT BE CH DE ES FR GB GR IT LI LU NL SE**

(30) Priorität: **09.09.1988 CH 3371/88**
**11.07.1989 CH 2579/89**

(43) Veröffentlichungstag der Anmeldung:
**14.03.1990 Patentblatt 1990/11**

(73) Patentinhaber: **CIBA-GEIGY AG**
**4002 Basel (CH)**

(72) Erfinder:
- **Zondler, Helmut, Dr.**
**CH-4103 Bottmingen (CH)**
- **Hubele, Adolf, Dr.**
**CH-4312 Magden (CH)**

(56) Entgegenhaltungen:
**EP-A- 0 019 450**
**EP-A- 0 182 190**
**EP-A- 0 270 111**
**WO-A-89/07599**
**DE-A- 2 209 470**

- **JOURNAL OF THE CHEMICAL SOCIETY, PERKIN TRANSACTIONS I, Nr. 2, Februar 1988, Seiten 351-357, Royal Society of Chemistry, Letchworth, GB; H. MARLEY et al.: "Synthesis, molecular structure, and reactions of 1H-1,2,4-triazolo[4,3- a]pyrimidinium betaines"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.13.0/3.4

**Beschreibung**

Die vorliegende Erfindung betrifft neue substituierte 2-Aminopyrimidin-Derivate der nachstehenden Formel I. Sie betrifft ferner die Herstellung dieser Substanzen sowie agrochemische Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung von Schädlingen, vor allem von pflanzenschädigenden Mikroorganismen, vorzugsweise Pilzen.

Die erfindungsgemässen Pyrimidin-Verbindungen entsprechend der allgemeinen Formel I

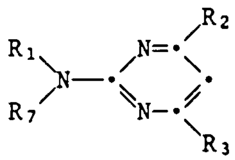

(I)

in welcher bedeuten:

$R_1$ Phenyl oder bis zu 3-fach durch $R_4$ substituiertes Phenyl:

$R_2$ Wasserstoff, $C_1$-$C_5$-Alkyl, mit den Resten $OR_5$ oder $SR_5$ substituiertes $C_1$-$C_5$-Alkyl, $C_3$-$C_6$-Cycloalkyl, mit $C_1$-$C_4$-Alkyl oder Halogen bis zu 3-fach substituiertes $C_3$-$C_4$-Cycloalkyl, $C_2$-$C_5$-Akenyl, $C_2$-$C_5$-Alkinyl oder den Formylrest;

$R_3$ Wasserstoff, $C_1$-$C_4$-Alkyl, mit Halogen, Cyano, oder den Resten $OR_5$ oder $SR_5$ substituiertes $C_1$-$C_4$-Alkyl, $C_3$-$C_6$-Cycloalkyl oder mit $C_1$-$C_4$-Alkyl oder Halogen bis zu 3-fach substituiertes $C_3$-$C_6$-Cycloalkyl;

$R_4$ Halogen, $C_1$-$C_3$-Alkyl, $C_1$-$C_2$-Halogenalkyl, $C_1$-$C_3$-Alkoxy oder $C_1$-$C_3$-Halogenalkoxy;

$R_5$ Wasserstoff, $C_1$-$C_5$-Alkyl, $C_3$-$C_5$-Alkenyl, $C_3$-$C_5$-Alkinyl oder den Rest $(CH_2)_n$-X-$C_1$-$C_3$-Alkyl;

$R_7$ die Gruppe $-NH_2$,

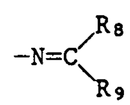 oder 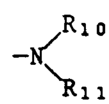 ;

$R_8$ Wasserstoff, $C_1$-$C_3$-Alkyl oder $C_1$-$C_3$-Halogenalkyl;

$R_9$ Wasserstoff, $C_1$-$C_8$-Alkyl, durch Hydroxy, $OR_{12}$, $SR_{12}$ oder $N(R_{12})_2$ substituiertes $C_1$-$C_3$-Alkyl, $C_3$-$C_6$-Cycloalkyl, durch $SR_{12}$ substituiertes Cyclopropyl, $C_3$-$C_{10}$-Alkenyl, $C_1$-$C_3$-Halogenalkyl, 1-, 2- oder 3-Pyridyl,

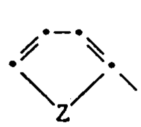 oder 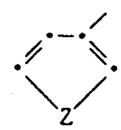 ;

$R_8$ und $R_9$ Zusammen mit dem Kohlenstoffatom im Rest $R_7$ einen gesättigten oder ungesättigten Ring, bestehend aus 4 bis 7 Kohlenstoffatomen;

$R_{10}$ $CH(R_8)R_9$, Phenyl, $C_3$-$C_5$-Alkenyl, $C_3$-$C_5$-Alkinyl oder Cyanoalkyl mit 2 oder 3 Kohlenstoffatomen im Alkylrest;

$R_{11}$ Wasserstoff, $C_1$-$C_5$-Alkyl, $C_3$-$C_5$-Alkenyl, $C_3$-$C_5$-Alkinyl oder Cyanoalkyl mit 2 oder 3 Kohlenstoffatomen im Alkylrest;

$R_{12}$ $CH_3$ oder $C_2H_5$;

X Sauerstoff oder Schwefel;

Z O, S, NH oder $NCH_3$; und

n 1 bis 3;

unter Einschluss ihrer Säureadditionssalze und Metallsalzkomplexe.

Unter Alkyl selbst oder als Bestandteil eines anderen Substituenten, wie Halogenalkyl, Alkoxy oder Halogenalkoxy, sind je nach Anzahl der benannten Kohlenstoffatome beispielsweise zu verstehen Methyl, Ethyl, Propyl, Butyl oder Pentyl sowie ihre Isomeren, wie z.B. Isopropyl, Isobutyl, tert.-Butyl oder sek.-Butyl. Halogen steht für Fluor, Chlor, Brom oder Jod, bevorzugt für Fluor, Chlor oder Brom, Halogenalkyl und Halogenalkoxy bezeichnen einfach bis perhalogenierte Reste, wie z.B. $CHCl_2$, $CH_2F$, $CCl_3$, $CH_2Cl$, $CHF_2$, $CF_3$, $CH_2CH_2Br$, $C_2Cl_5$, $CH_2Br$, CHBrCl usw., vorzugsweise

$CF_3$. Cycloalkyl steht je nach Zahl der genannten Kohlenstoffatome z.B. für Cyclopropyl, Cyclobutyl, Cyclopentyl oder Cyclohexyl.

Die Verbindungen der Formel I sind bei Raumtemperatur stabile Oele, Harze oder Feststoffe, die sich durch wertvolle mikrobizide Eigenschaften auszeichnen. Sie lassen sich auf dem Agrarsektor oder verwandten Gebieten präventiv und kurativ zur Bekämpfung von pflanzenschädigenden Mikroorganismen einsetzen. Die erfindungsgemässen Wirkstoffe der Formel I zeichnen sich bei niedrigen Anwendungskonzentrationen nicht nur durch hervorragende insektizide und fungizide Wirkung, sondern auch durch besonders gute Pflanzenverträglichkeit aus.

Die Erfindung betrifft sowohl die freien Verbindungen der Formel I als auch deren Additionssalze mit anorganischen und organischen Säuren sowie deren Komplexe mit Metallsalzen.

Erfindungsgemässe Salze sind insbesondere Additionssalze mit unbedenklichen anorganischen oder organischen Säuren, beispielsweise Halogenwasserstoffsäuren, z.B. Chlor-, Brom- oder Jodwasserstoffsäure, Schwefelsäure, Phosphorsäure, phosphorige Säure, Salpetersäure, oder organischen Säuren wie Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure, 2-Acetoxybenzoesäure oder 1,2-Naphthalin-disulfonsäure.

Metallsalzkomplexe der Formel I bestehen aus dem zugrundeliegenden organischen Molekül und einem anorganischen oder organischen Metallsalz, z.B. den Halogeniden, Nitraten, Sulfaten, Phosphaten, Acetaten, Trifluoracetaten, Trichloracetaten, Propionaten, Tartraten, Sulfonaten, Salicylaten, Benzoaten usw. der Elements der zweiten Hauptgruppe wie Calcium und Magnesium und der dritten und vierten Hauptgruppe wie Aluminium, Zinn oder Blei sowie der ersten bis achten Nebengruppe wie Chrom, Mangan, Eisen, Kobalt, Nickel, Kupfer, Zink usw. Bevorzugt sind die Nebengruppen-Elemente der 4. Periode. Die Metalle können dabei in den verschiedenen ihnen zukommenden Wertigkeiten vorliegen. Die Metallkomplexe können ein- oder mehrkernig auftreten, d.h. sie können ein oder mehrere organische Molekülanteile als Liganden enthalten.

Wichtige Gruppen von Pflanzenfungiziden sind Verbindungen der Formel I mit folgenden Symbolbedeutungen:

Gruppe 1 (Substituenten)

$R_1$ Phenyl oder bis zu 3-fach durch $R_4$ substituiertes Phenyl;
$R_2$ Wasserstoff, $C_1$-$C_5$-Alkyl, mit den Resten $OR_5$ oder $SR_5$ substituiertes $C_1$-$C_5$-Alkyl, $C_3$-$C_6$-Cycloalkyl mit $C_1$-$C_4$-Alkyl oder Halogen bis zu 3-fach substituiertes $C_3$-$C_6$-Cycloalkyl, $C_2$-$C_5$-Alkenyl, $C_2$-$C_5$-Alkinyl oder den Formylrest;
$R_3$ $C_1$-$C_4$-Alkyl, mit Halogen, Cyano, oder den Resten $OR_5$ oder $SR_5$ substituiertes $C_1$-$C_4$-Alkyl, $C_3$-$C_6$-Cycloalkyl oder mit $C_1$-$C_4$-Alkyl oder Halogen bis zu 3-fach substituiertes $C_3$-$C_6$-Cycloalkyl;
$R_4$ Halogen, $C_1$-$C_3$-Alkyl;
$R_5$ Wasserstoff, $C_1$-$C_5$-Alkyl, $C_3$-$C_5$-Alkenyl, $C_3$-$C_5$-Alkinyl oder den Rest $(CH_2)_n$-X-$C_1$-$C_3$-Alkyl;
$R_9$ Wasserstoff, $C_1$-$C_5$-Alkyl, $C_3$-$C_6$-Cycloalkyl, $C_3$-$C_5$-Alkenyl, $C_1$-$C_3$-Halogenalkyl;
$R_8$ und $R_9$ zusammen mit dem Kohlenstoffatom in Rest $R_7$ einen gesättigten oder ungesättigten Ring bestehend aus 5 oder 6 Kohlenstoffatomen;
$R_7$, $R_{10}$, $R_{11}$, $R_{12}$ und Z besitzen die unter Formel I angegebenen Bedeutungen und Halogen stellt vorzugsweise Fluor, Chlor oder Brom dar.
X Sauerstoff oder Schwefel;
n 1 bis 3.

Gruppe 2 (Substituenten)

$R_1$ Phenyl oder einfach durch Halogen substituiertes Phenyl;
$R_2$ Wasserstoff, $C_1$-$C_5$-Alkyl, mit $OR_5$ substituiertes $C_1$-$C_5$-Alkyl, $C_3$-$C_6$-Cycloalkyl, mit $C_1$-$C_4$-Alkyl oder Halogen bis zu 3-fach substituiertes $C_3$-$C_6$-Cycloalkyl, $C_2$-$C_5$-Alkenyl, $C_2$-$C_5$-Alkinyl oder den Formylrest;
$R_3$ $C_1$-$C_4$-Alkyl, mit Halogen, Cyano oder $OR_5$ substituiertes $C_1$-$C_4$-Alkyl, $C_3$-$C_6$-Cycloalkyl oder mit Methyl substituiertes $C_3$-$C_6$-Cycloalkyl;
$R_5$ Wasserstoff oder $C_1$-$C_2$-Alkyl;
$R_9$ Wasserstoff, $C_1$-$C_5$-Alkyl, $C_3$-$C_6$-Cycloalkyl, $C_3$-$C_5$-Alkenyl, $C_1$-$C_3$-Halogenalkyl;
$R_8$ und $R_9$ zusammen mit dem Kohlenstoffatom in Rest $R_7$ einen gesättigten oder ungesättigten Ring bestehend aus 5 oder 6 Kohlenstoffatomen;
$R_7$, $R_{10}$, $R_{11}$ und $R_{12}$ besitzen die unter Formel I angegebenen Bedeutungen und Halogen stellt vorzugsweise Fluor, Chlor oder Brom dar.

Gruppe 3 (Substituenten)

$R_1$ Phenyl oder bis zu 3-fach durch $R_4$ substituiertes Phenyl;
$R_2$ Wasserstoff, $C_1$-$C_5$-Alkyl, mit den Resten $OR_5$ oder $SR_5$ substituiertes $C_1$-$C_5$-Alkyl, $C_3$-$C_6$-Cycloalkyl, mit $C_1$-$C_4$-Alkyl oder Halogen bis zu 3-fach substituiertes $C_3$-$C_6$-Cycloalkyl, $C_2$-$C_5$-Alkenyl, $C_2$-$C_5$-Alkinyl oder den Formylrest;
$R_3$ $C_1$-$C_4$-Alkyl, mit Halogen, Cyano, oder den Resten $OR_5$ oder $SR_5$ substituiertes $C_1$-$C_4$-Alkyl, $C_3$-$C_6$-Cycloalkyl oder mit $C_1$-$C_4$-Alkyl oder Halogen bis zu 3-fach substituiertes $C_3$-$C_6$-Cycloalkyl;
$R_4$ Halogen, $C_1$-$C_3$-Alkyl, $C_1$-$C_2$-Halogenalkyl, $C_1$-$C_3$-Alkoxy oder $C_1$-$C_3$-Halogenalkoxy;
$R_5$ Wasserstoff, $C_1$-$C_5$-Alkyl, $C_3$-$C_5$-Alkenyl, $C_3$-$C_5$-Alkinyl oder den Rest $(CH_2)_n$-X-$C_1$-$C_3$-Alkyl;
$R_7$ -$NH_2$;
X Sauerstoff oder Schwefel;
n 1-3; unter Einschluss ihrer Säureadditionssalze und Metallsalzkomplexe.

Gruppe 4 (Substituenten)

$R_1$ Phenyl oder bis zu 3-fach durch $R_4$ substituiertes Phenyl;
$R_2$ Wasserstoff, $C_1$-$C_5$-Alkyl, mit den Resten $OR_5$ oder $SR_5$ substituiertes $C_1$-$C_5$-Alkyl, $C_3$-$C_6$-Cycloalkyl, mit $C_1$-$C_4$-Alkyl oder Halogen bis zu 3-fach substituiertes $C_3$-$C_6$-Cycloalkyl, $C_2$-$C_5$-Alkenyl, $C_2$-$C_5$-Alkinyl oder den Formylrest;
$R_3$ $C_1$-$C_4$-Alkyl, mit Halogen, Cyano, oder den Resten $OR_5$ oder $SR_5$ substituiertes $C_1$-$C_4$-Alkyl, $C_3$-$C_6$-Cycloalkyl oder mit $C_1$-$C_4$-Alkyl oder Halogen bis zu 3-fach substituiertes $C_3$-$C_6$-Cycloalkyl;
$R_4$ Halogen;
$R_5$ Wasserstoff, $C_1$-$C_5$-Alkyl, $C_3$-$C_5$-Alkenyl, $C_3$-$C_5$-Alkinyl oder den Rest $(CH_2)_n$-X-$C_1$-$C_3$-Alkyl;
X Sauerstoff oder Schwefel;
n 1-3.

Gruppe 5 (Substituenten)

$R_1$ Phenyl oder bis zu 3-fach durch Halogen substituiertes Phenyl;
$R_2$ Wasserstoff, $C_1$-$C_5$-Alkyl, mit den Resten $OR_5$ oder $SR_5$ substituiertes $C_1$-$C_5$-Alkyl, $C_3$-$C_6$-Cycloalkyl mit $C_1$-$C_4$-Alkyl oder Halogen bis zu 3-fach substituiertes $C_3$-$C_6$-Cycloalkyl, $C_2$-$C_5$-Alkenyl, $C_2$-$C_5$-Alkinyl oder den Formylrest;
$R_3$ $C_1$-$C_4$-Alkyl, mit Halogen, Cyano, oder den Resten $OR_5$ oder $SR_5$ substituiertes $C_1$-$C_4$-Alkyl, $C_3$-$C_6$-Cycloalkyl oder mit $C_1$-$C_4$-Alkyl oder Halogen bis zu 3-fach substituiertes $C_3$-$C_6$-Cycloalkyl;
$R_5$ Wasserstoff, $C_1$-$C_5$-Alkyl, $C_3$-$C_5$-Alkenyl, $C_3$-$C_5$-Alkinyl oder den Rest $(CH_2)_n$-X-$C_1$-$C_3$-Alkyl;
X Sauerstoff oder Schwefel;
n 1-3.

Gruppe 6 (Substituenten)

$R_1$ Phenyl oder einfach durch Chlor oder Fluor substituiertes Phenyl;
$R_2$ $C_1$-$C_5$-Alkyl, mit $OR_5$ substituiertes $C_1$-$C_2$-Alkyl, $C_3$-$C_6$-Cycloalkyl mit $C_1$-$C_4$-Alkyl oder Halogen bis zu 3-fach substituiertes $C_3$-$C_6$-Cycloalkyl, $C_2$-$C_5$-Alkenyl, $C_2$-$C_5$-Alkinyl oder den Formylrest;
$R_3$ $C_1$-$C_4$-Alkyl, $C_1$-$C_4$-Halogenalkyl, $C_3$-$C_6$-Cycloalkyl oder mit Methyl substituiertes $C_3$-$C_6$-Cycloalkyl;
$R_5$ Wasserstoff oder $C_1$-$C_2$-Alkyl.

Besonders bevorzugt sind solche Verbindungen der Gruppen 5 und 6, in denen $R_3$ bedeutet: Methyl, Fluormethyl, Chlormethyl, Brommethyl, $C_3$-$C_6$-Cycloalkyl oder Methoxymethyl.

Folgende Gruppen von Einzelsubstanzen sind bevorzugt:

Gruppe 1 (Verbindungen)

N-(4-Fluormethyl-6-cyclopropylpyrimidyl-2)-N-phenyl-hydrazin;
N-(4-Methyl-6-cyclopropylpyrimidyl-2)-N-m-fluorphenyl-hydrazin;
N-(4-Methyl-6-cyclopropylpyrimidyl-2)-N-p-fluorphenyl-hydrazin;

Gruppe 2 (Verbindungen)

N-(4-Methyl-6-cyclopropylpyrimidyl-2)-N-phenyl-hydrazin;
N-(4,6-Dimethylpyrimidyl-2)-N-phenyl-hydrazin;
N-(4-Methyl-6-methoxymethylpyrimidyl-2)-N-phenyl-hydrazin;

Gruppe 3 (Verbindungen)

N-(4,6-Dimethylpyrimidyl-2)-N-phenyl-propionaldehydhydrazon;
N-(4,6-Dimethylpyrimidyl-2)-N-phenyl-isobutyraldehydhydrazon;
N-(4-Methyl-6-methoxymethylpyrimidyl-2)-N-phenyl-isobutyraldehydhydrazon;
N-(4-Methyl-6-methoxymethylpyrimidyl-2)-N-phenyl-propionaldehydhydrazon;
N-(4-Methyl-6-cyclopropylpyrimidyl-2)-N-phenyl-propionaldehydhydrazon;
N-(4-Methyl-6-cyclopropylpyrimidyl-2)-N-phenyl-n-butyraldehydhydrazon;
N-(4-Methyl-6-cyclopropylpyrimidyl-2)-N-phenyl-isobutyraldehydhydrazon;
N-(4-Methyl-6-cyclopropylpyrimidyl-2)-N-phenyl-trichloracetaldehydhydrazon;
N-(4-Methyl-6-cyclopropylpyrimidyl-2)-N-p-fluorphenyl-acetaldehydhydrazon;
N-(4-Methyl-6-cyclopropylpyrimidyl-2)-N-p-fluorphenyl-isobutyraldehydhydrazon;
N-(4-Methyl-6-cyclopropylpyrimidyl-2)-N-m-fluorphenyl-isobutyraldehydhydrazon;
N-(4,6-Dimethylpyrimidyl-2)-N-phenyl-N′-methylhydrazin;
N-(4,6-Dimethylpyrimidyl-2)-N-phenyl-N′-dimethylhydrazin;
N-(4,6-Dimethylpyrimidyl-2)-N-phenyl-N′-n-propylhydrazin;
N-(4,6-Dimethylpyrimidyl-2)-N-phenyl-N′-isobutylhydrazin;
N-(4-Methyl-6-methoxymethylpyrimidyl-2)-N-phenyl-N′-methylhydrazin;
N-(4-Methyl-6-methoxymethylpyrimidyl-2)-N-phenyl-N′-n-propylhydrazin;
N-(4-Methyl-6-methoxymethylpyrimidyl-2)-N-phenyl-N′-dimethylhydrazin;
N-(4-Methyl-6-cyclopropylpyrimidyl-2)-N-phenyl-N′-methylhydrazin;
N-(4-Methyl-6-cyclopropylpyrimidyl-2)-N-phenyl-N′-isobutylhydrazin;
N-(4-Methyl-6-cyclopropylpyrimidyl-2)-N-phenyl-N′-dimethylhydrazin;
N-(4-Methyl-6-cyclopropylpyrimidyl-2)-N-phenyl-N′-diäthylhydrazin;
N-(4-Methyl-6-cyclopropylpyrimidyl-2)-N-phenyl-N′-methyl-N′-äthylhydrazin;
N-(4-Methyl-6-cyclopropylpyrimidyl-2)-N-p-fluorphenyl-N′-äthylhydrazin;
N-(4-Methyl-6-methoxymethylpyrimidyl-2)-N-m-fluorphenyl-N′-isopropylhydrazin;
Die Verbindungen der Formel I werden wie folgt hergestellt:

Verfahren (a)

Umsetzung eines Pyrimidinderivates der Formel II

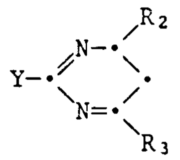

(II)

mit einem Phenylhydrazinderivat der Formel III

$$R_1\text{-NH-NH-R} \qquad \text{(III)}$$

in Gegenwart einer Base in aprotischen Lösungsmitteln bei Temperaturen von -50° bis 150°C, bevorzugt -30° bis 80°C, wobei Y Halogen, bevorzugt Chlor, den Rest $SO_2R_6$ oder $N^{\oplus}(CH_3)_3$ darstellt, $R_6$ $C_1$-$C_4$-Alkyl, Phenyl oder mit Methyl oder Chlor substituiertes Phenyl bedeutet und R die Bedeutungen von $R_{10}$ und $R_{11}$ hat und diese sowie $R_1$-$R_3$ den unter der Formel I angegebenen Definitionen entsprechen.

Verfahren (b)

Umsetzung eines Pyrimidinhydrazinderivates der Formel IV mit einem Aldehyd oder Keton der Formel V zu Verbindungen der Formel VII unter Abspaltung von Wasser

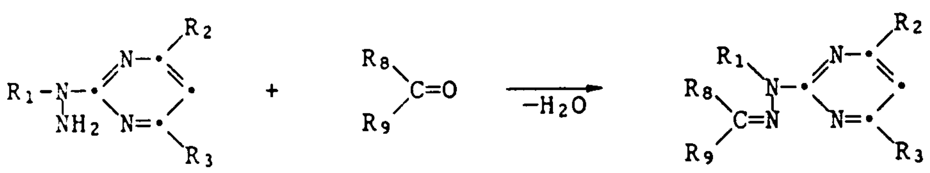


(IV) (V) (VII)

in einem beliebigen Lösungsmittel in Gegenwart einer Säure bei Temperaturen von -20° bis 120°C, bevorzugt 10° bis 50°C, wobei $R_1$-$R_3$ und $R_8$ und $R_9$ die unter Formel I angegebenen Bedeutungen besitzen.

Zur Entfernung des Wassers aus dem Reaktionsgemisch kann die azeotrope Destillation oder der Einsatz von Molekularsieben angewendet werden. Ferner können Trocknungsmittel, wie z.B. $CaCl_2$ oder $Na_2SO_4$, eingesetzt werden. Bei der Umsetzung des Derivates (IV) mit einem Aldehyd kann häufig auf die Entfernung des Wassers aus dem Reaktionsgemisch verzichtet werden.

<u>Verfahren (c)</u>

Reduktion eines Hydrazonderivates der Formel VII

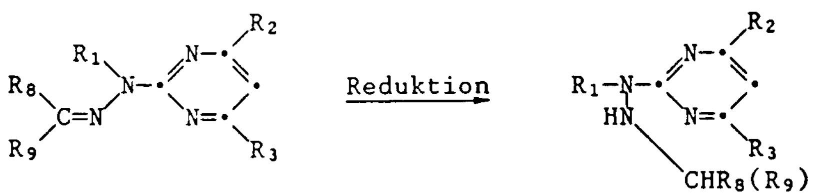


(VII) (VIII)

mittels eines Reduktionsmittels, wie z.B. der Borhydrid-Etherate, $NaBH_4$, $NaCNBH_3$ oder $LiAlH_4$, in einem inerten Lösungsmittel, wie z.B. geeignete Alkohole, Tetrahydrofuran, Dioxan, Essigsäureethylester oder Toluol bei Temperaturen von 0° bis 50°C oder durch katalytische Hydrierung an Katalysatoren, wie z.B. Nickel, Platin, Palladium oder Rhodium.

<u>Verfahren (d)</u>

Reduktive Alkylierung eines Pyrimidinhydrazinderivates der Formel IV

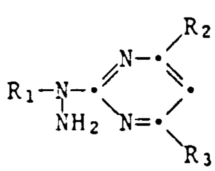

(IV)

mit einem Aldehyd oder Keton der Formel V

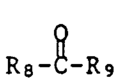

(V)

in Gegenwart eines Reduktionsmittels, wie z.B. Borhydrid-Etherate, $NaBH_4$, $NaCNBH_3$ oder $LiAlH_4$, in einem inerten Lösungsmittel, wie z.B. geeignete Alkohole, Tetrahydrofuran, Dioxan, Essigsäureethylester oder Toluol, bei Temperaturen von 0° bis 50°C, vorzugsweise 10° bis 40°C.

Verfahren (e)

Alkylierung eines Pyrimidinhydrazins der Formel IV oder VIII mit einem Alkylhalogenid $R_oHal$

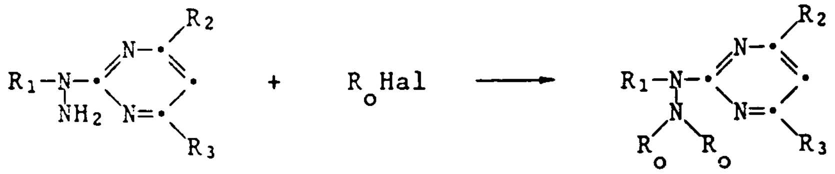

(IV) (IX)

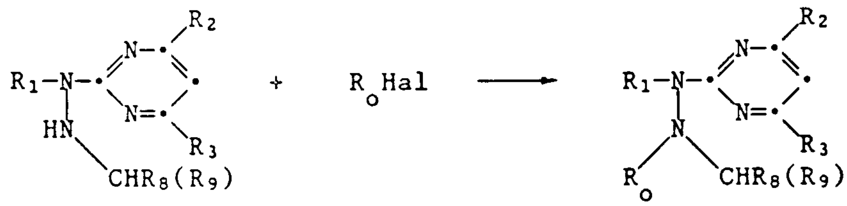

(VIII) (X)

in einem inerten Lösungsmittel in Gegenwart einer Base bei Temperaturen von 0°-60°C, vorzugsweise von 10°-40°C, wobei $R_o$ $C_1$-$C_4$-Alkyl darstellt. Als weitere Alkylierungsmittel eignen sich Dialkylsulfate.

In den vorstehend beschriebenen Verfahren (a-e) stellen $R_1$-$R_9$ die unter Formel I angegebenen Bedeutungen dar.

In den beschriebenen Verfahren können - soweit erforderlich - sowohl anorganische als auch organische Basen verwendet werden, so z.B. folgende: die Hydroxide, Oxide oder Karbonate von Lithium, Natrium, Kalium, Magnesium, Calcium, Strontium und Barium oder auch Hydride, wie z.B. Natriumhydrid, und Alkoholate, wie z.B. Kalium-tert.-Butylat, sowie tertiäre Amine wie Triethylamin, Triethylendiamin oder Pyridin.

Als Reaktionsmedien in Anpassung an die jeweiligen Reaktionsbedingungen können z.B. folgende Lösungs- und Verdünnungsmittel verwendet werden: aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; sowie Gemische solcher Lösungsmittel untereinander.

Die Pyrimidinderivate der Formel II, bei denen Y Halogen bedeutet, lassen sich nach bekannten Methoden (vgl. D.J. Brown, The Pyrimidines, Interscience Publishers, 1962) herstellen.

Ein häufig benutzter Syntheseweg besteht in der Kondensation von Harnstoff mit β-Diketonen zu 2-Hydroxypyrimidinen, die anschliessend zu 2-Halogenpyrimidinen wie folgt umgesetzt werden.

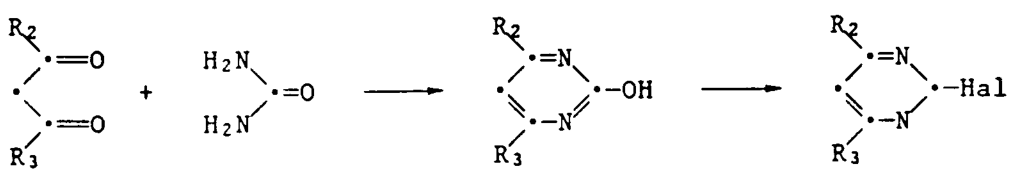

Als Halogenierungsmittel können insbesondere Phosphoroxychlorid oder Phosphoroxybromid verwendet werden.

Eine weitere Möglichkeit die 2-Halogenpyrimidine der Formel II zu erhalten besteht darin, sie über die 2-Aminopyrimidine herzustellen. Die 2-Aminopyrimidine werden nach bekannten Methoden (vgl. D.J. Brown, The Pyrimidines, Interscience Publishers, 1962) gewonnen, anschliessend diazotiert und nach dem Sandmeyer-Verfahren in die 2-Halogenpyrimidine überführt. Die 2-Aminopyrimidine erhält man z.B. durch Kondensation von β-Diketonen mit Guanidin in folgender Weise.

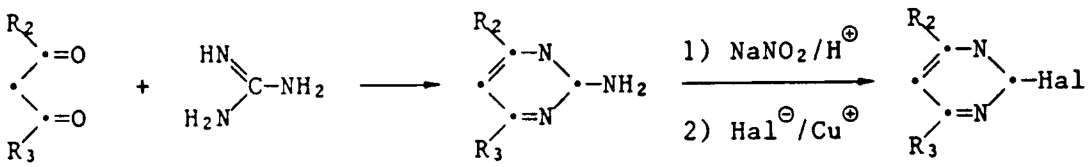


Die Pyrimidinderivate der Formel II, bei denen Y $SO_2$-$C_1$-$C_4$-Alkyl oder $SO_2$-Aryl bedeutet, erhält man nach bekannten Methoden durch Oxydation der entsprechenden Alkyl- bzw. Arylmercaptopyrimidine, deren Herstellung ebenfalls bekannt ist (vgl. D.J. Brown, The Pyrimidines, Interscience Publishers, 1962).

Pyrimidine der Formel II, in denen der Rest $R_3$ die Bedeutung von Halogenalkyl hat, lassen sich ausser, wie vorhergehend beschrieben, durch Kondensation der entsprechenden Diketone auch durch Umsetzung der Hydroxyalkylderivate mit Phosphorhalogenid oder Thionylhalogenid in Gegenwart von tertiären Basen in inerten Lösungsmitteln herstellen.

Zwischenprodukte der Formel II

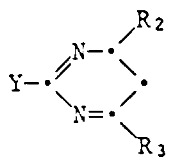 (II),

worin Y Halogen oder $SO_2$-$R_6$ und $R_6$ $C_1$-$C_4$-Alkyl oder Aryl darstellt und $R_2$ und $R_3$ die unter Formel I angegebenen Bedeutungen haben, sind teilweise bekannt.

Die Hydrazinderivate der Formel III sind bekannt oder lassen sich nach dem Fachmann bekannten Methoden herstellen.

2-Anilinopyrimidin-Derivate mit fungiziden Eigenschaften sind in EP-A-270111 beschrieben. Phenylhydrazon-Derivate, die in der Landwirtschaft gegen Mikroorganismen eingesetzt werden, sind aus EP-A-019450 bekannt.

Ueberraschenderweise wurde nun gefunden, dass Verbindungen der Formel I ein für praktische Bedürfnisse sehr günstiges- biozides Spektrum zur Bekämpfung von phytopathogenen Mikroorganismen, insbesondere Fungi, aufweisen. Sie besitzen sehr vorteilhafte kurative, präventive und insbesondere systemische Eigenschaften und werden zum Schutz von zahlreichen Kulturpflanzen eingesetzt. Mit den Wirkstoffen der Formel I können an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile z.B. von phytopathogenen Mikroorganismen verschont bleiben.

Verbindungen der Formel I sind z.B. gegen die den folgenden Klassen angehörenden phytopathogenen Pilze wirksam: Fungi imperfecti (insbesondere Botrytis, ferner Pyricularia, Helminthosporium, Fusarium, Septoria, Cercospora und Alternaria); Basidiomyceten (z.B. Rhizoctonia, Hemileia, Puccinia). Darüber hinaus wirken sie gegen die Klasse der Ascomyceten (z.B. Venturia und Erysiphe, Podosphaera, Monilinia, Uncinula) und der Oomyceten (z.B. Phytophthora, Pythium, Plasmopara). Die Verbindungen der Formel I können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Verbindungen der Formel I sind darüber hinaus gegen Schadinsekten wirksam, z.B. gegen Getreide-Schädlinge, insbesondere Reisschädlinge.

Die Erfindung betrifft auch die Mittel, die als Wirkstoffkomponente Verbindungen der Formel I enthalten, insbesondere pflanzenschützende Mittel, sowie deren Verwendung auf dem Agrarsektor oder verwandten Gebieten.

Darüber hinaus schliesst die vorliegende Erfindung auch die Herstellung dieser Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der neuen Verbindungen der Formel I bzw. der neuen Mittel auszeichnet.

Als Zielkulturen für den hierin offenbarten pflanzenschützenden Einsatz gelten im Rahmen dieser Erfindung beispielsweise folgende Pflanzenarten: Getreide (Weizen, Gerste, Roggen, Hafer, Reis, Mais, Sorghum und verwandte Species); Rüben (Zucker- und Futterrüben); Kern-, Stein und Beerenobst (Aepfel, Birnen, Pflaumen, Pfirsiche, Mandeln, Kirschen, Erd-, Him- und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Kampfer) oder Pflanzen wie Tabak, Nüsse, Kaffee, Zuckerrohr, Tee, Pfeffer, Weinreben, Hopfen, Bananen- und Naturkautschukgewächse sowie Zierpflanzen.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Bei Wasserreiskulturen kann man solche Granulate dem überfluteten Reis-Feld zudosieren. Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet.

Die Verbindungen der Formel I werden dabei in unveränderter Form oder vorzugsweise zusammen mit den in der Formulierungstechnik üblichen Hilfsmitteln eingesetzt. Dafür werden sie zweckmässigerweise z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha, bevorzugt 100 g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorilonit oder Attapulgit. Zur Verbesserung der physikalischen, Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien, z.B. Calcit oder Sand, in Frage. Darüber hinaus kann eine Vielzahl von vorgranulierten Materialien anorganischer Natur wie insbesondere Dolomit oder zerkleinerte Pflanzenrückstände verwendet werden.

Als oberflächenaktive Verbindungen kommen je nach Art des zu formulierenden Wirkstoffs der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na-oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-Methyllaurinsalze zu erwähnen.

Häufiger werden jedoch sog. synthetische Tenside verwendet, insbesondere Alkansulfonate, Fettalkoholsulfate, sulfonierte Benzimidazolderivate oder Alkylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind wasserlösliche 20 bis 250 Aethylenglykoläthergruppen und 10 bis 100 Propylenglykolethergruppen enthaltende Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheiten 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ammoniumbromid.

Weitere in der Formulierungstechnik gebräuchlichen Tenside sind dem Fachmann bekannt oder können der einschlägigen Fachliteratur entnommen werden.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-%, insbesondere 99,9 bis 5 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung, ohne dieselbe einzuschränken.

## 1. Herstellungsbeispiele

Beispiel 1.1: 2-Hydroxy-4-methyl-6-cyclopropyl-pyrimidin-hydrochlorid (Ausgangsprodukt)

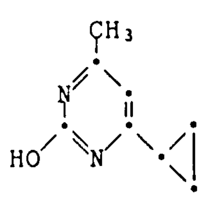

• HCl

Man löst 6,0 g (0,10 Mol) Harnstoff und 12,6 g (0,10 Mol) Cyclopropylbutan-1,3-dion bei Raumtemperatur (~20°C) in 35 ml Ethanol und 15 ml 32 %iger wässeriger Salzsäure. Nach 10-tägigem Stehen bei Raumtemperatur wird die Lösung am Rotationsverdampfer bei einer Badtemperatur von maximal 45°C eingeengt. Der Rückstand wird in 20 ml Ethanol gelöst; nach kurzer Zeit beginnt sich das Produkt in Form des Hydrochlorids abzuscheiden. Man fügt unter Rühren langsam 20 ml Diethylether hinzu, trennt das Produkt vom Lösungsmittel durch Saugfiltration ab, wäscht es mit einem Gemisch aus Diethylether und Ethanol und trocknet es bei 60°C im Vakuum. Man erhält 7,14 g (38,2 % d.Th.) 2-Hydroxy-4-methyl-6-cyclopropyl-pyrimidin-hydrochlorid. Durch Einengen des Filtrats erhält man nach Umkristallisation aus 10 ml Ethanol und 20 ml Diethylether weitere 5,48 g (29,2 % d.Th.) Substanz.

| Analyse: $C_8H_{10}N_2O \cdot HCl$ (MG: 186,64) | | |
|---|---|---|
| | % ber. | % gefunden |
| C | 51,48 | 51,47 |
| H | 5,94 | 5,97 |
| N | 15,01 | 15,15 |
| Cl | 18,99 | 18,89 |

Beispiel 1.2: 2-Chlor-4-methyl-6-cyclopropyl-pyrimidin-hydrochlorid (Ausgangsprodukt)

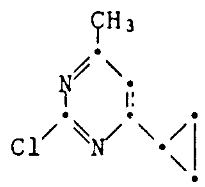

52,8 g (0,24 Mol) 2-Hydroxy-4-methyl-6-cyclopropylpyrimidin-hydrochlorid werden in ein Gemisch von 100 ml Phosphoroxychlorid und 117 g (0,79 Mol) Diethylanilin eingetragen und gerührt; die exotherme Reaktion kommt langsam in Gang, wobei die Temperatur von Raumtemperatur auf 63°C steigt. Anschliessend wird im Oelbad 2 Stunden bei 100-110°C Innentemperatur erhitzt. Nach Abkühlung auf Raumtemperatur wird das Gemisch unter Rühren in eine Mischung aus Eiswasser und Methylenchlorid gegossen. Nach einer Std. trennt man die organische Phase im Scheidetrichter ab und wäscht sie mit $NaHCO_3$-Lösung neutral. Nach Entfernung des Lösungsmittels erhält man 116,4 g Rohprodukt bestehend aus 2-Chlor-4-methyl-6-cyclopropyl-pyrimidin und Diethylanilin. Die chromatographische Trennung mittels Kieselgel und einem Gemisch aus 25 % Essigsäureethylester und 75 % Hexan als Laufmittel ergibt 35,7 g (89,4 % d.Th.) reines 2-Chlor-4-methyl-6-cyclopropylpyrimidin als farbloses Oel.

Beispiel 1.3: 2-Amino-4-diethoxymethyl-6-cyclopropylpyrimidin (Ausgangsprodukt)

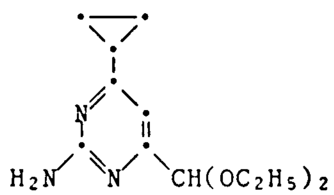

74,8 g (0,42 Mol) Guanidincarbonat und 74,1 g (0,35 Mol) 4-Cyclopropyl-2,4-dioxo-butyraldehyddiethylacetal werden in 250 ml Ethanol 10 Stunden gekocht. Sodann wird am Rotationsverdampfer eingeengt und der Rückstand mit Wasser und Essigsäureethylester extrahiert. Nach dem Abdampfen des Essigsäureethylesters bleiben 79,2 g Rohprodukt zurück, dessen Umkristallisation aus Hexan 70,4 g (85,8 % d. Theorie) Reinsubstanz ergibt. Smp. 77-78°C.

Beispiel 1.4: 2-Chlor-4-formyl-6-cyclopropylpyrimidin (Ausgangsprodukt)

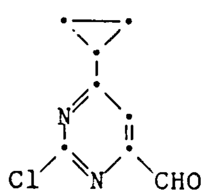

70,3 g (0,30 Mol) 2-Amino-4-diethoxymethyl-6-cyclopropylpyrimidin werden in 340 ml 32 %iger wässriger Salzsäure gelöst und mit Trockeneis auf -25°C gekühlt. Sodann lässt man bei -20 bis -25°C eine Lösung von 40,9 g (0,59 Mol) Natriumnitrit in 80 ml Wasser langsam zutropfen, wobei sich Stickstoff entwickelt und ein Festprodukt abscheidet. Nach 2 Stunden wird die Kühlung entfernt. Man lässt die Mischung auf Raumtemperatur kommen und extrahiert mit Essigsäureethylester. Trocknen des Extrakts mit Natriumsulfat und Entfernung des Lösungsmittels führt zu 21,9 g Rohprodukt als Oel. Die weitere Reinigung mittels Säulenchromatographie (Kieselgel, Laufmittel 30 Teile Essigsäureethylester und 70 Teile Hexan) ergibt 16,1 g Reinsubstanz als farblose Flüssigkeit. Brechungsindex $n_D^{25}$ = 1,5603.

| Analyse: $C_8H_7ClN_2O$ (MG: 182,61) | | |
|---|---|---|
| | % ber. | % gefunden |
| C | 52,6 | 52,6 |
| H | 3,9 | 4,1 |
| N | 15.3 | 14.8 |
| Cl | 19,4 | 18,7 |

Beispiel 1.5: 2-Chlor-4-hydroxymethyl-6-cyclopropylpyrimidin (Ausgangsprodukt)

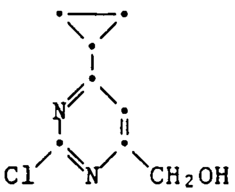

15,4 g (0,084 Mol) 2-Chlor-4-formyl-6-cyclopropylpyrimidin werden in 125 ml Methanol gelöst und durch Zugabe von 1,6 g Natriumborhydryid reduziert. Einengen, Extraktion mit Essigsäureethylester und Entfernung des Lösungsmittels am Rotationsverdampfer ergibt 14,5 g Rohprodukt, das aus einem Gemisch von 20 ml Toluol und 20 ml Cyclohexan umkristallisiert wird. Die Reinausbeute beträgt 13,7 g (88,4 % d. Theorie); Smp. 102-104°C.

| Analyse: $C_8H_9ClN_2O$ (MG: 184,63) | | |
|---|---|---|
| | % ber. | % gefunden |
| C | 52,04 | 52,05 |
| H | 4,91 | 4,90 |
| N | 15,17 | 15,27 |
| Cl | 19,20 | 19,28 |

Beispiel 1.6: Mesylat des 2-Chlor-4-hydroxymethyl-6-cyclopropylpyrimidins (Ausgangsprodukt)

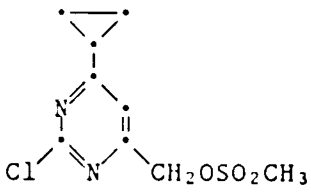

9.5 g (0,05 Mol) 4-Hydroxymethylpyrimidin und 5,7 g Triethylamin werden in 150 ml Tetrahydrofuran vorgelegt und unter Kühlung tropfenweise mit einer Lösung von 6,5 g Methansulfonsäurechlorid in 30 ml Tetrahydrofuran versetzt. Es scheidet sich sofort Triethylaminhydrochlorid ab, das abgesaugt wird. Einengen ergibt 14,7 g Rohprodukt das über Kieselgel chromatographiert wird (25 Teile Essigsäureethylester und 75 Teile Hexan). Man erhält 13,6 g Reinsubstanz. Smp. 64-66°C.

| Analyse: $C_9H_{11}ClN_2O_3S$ (MG: 262,71) | | |
|---|---|---|
| | % ber. | % gefunden |
| C | 41,15 | 41,32 |
| H | 4,22 | 4,33 |
| N | 10,66 | 10,56 |
| S | 12,20 | 12,16 |

Beispiel 1.7: 2-Chlor-4-fluormethyl-6-cyclopropylpyrimidin (Ausgangsprodukt)

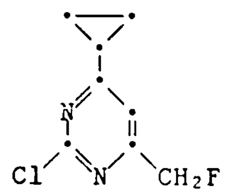

13,4 g (0,05 Mol) Mesylat werden in 70 ml Propionitril mit 9,4 g (0,16 Mol) Kaliumfluorid und 0,8 ml 18-Crown-6 als Katalysator 5 Stunden am Rückfluss gekocht. Die Entfernung des Lösungsmittels und nachfolgende Extraktion mit Wasser und Essigsäureethylester ergibt ein Rohprodukt, das durch Säulenchromatographie (Kieselgel; 15 Teile Essigsäureethylester und 85 Teile Hexan) gereinigt wird. Die Reinausbeute beträgt 7,5 g (78,6 % d. Theorie); Smp. 37-39°C.

| Analyse: $C_8H_8ClFN_2$(MG: 186,62) | | |
|---|---|---|
| | % ber. | % gefunden |
| C | 51,49 | 51,73 |
| H | 4,32 | 4,45 |
| N | 15,01 | 14,90 |
| F | 10,18 | 10,26 |
| Cl | 19,00 | 18,50 |

Beispiel 1.8: 2-Chlor-4-methyl-6-(2-methylcyclopropyl)-pyrimidin (Ausgangsprodukt)

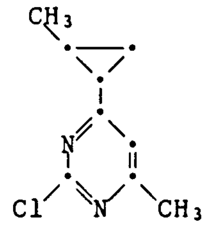

76,4 g (0,47 Mol) 2-Amino-4-methyl-6-(2-methylcyclopropyl)-pyrimidin, hergestellt durch Kochen von Guanidincarbonat mit Acetyl-methyl-2-methylcyclopropyl-keton in Ethanol, werden in 536 g 32 %iger Salzsäure gelöst und auf -25°C gekühlt. Danach setzt man 2 g Kupferpulver zu und tropft bei -25°C eine Lösung von 71,4 g (1,03 Mol) Natariumnitrit in 200 ml Wasser innerhalb 3 Stunden zu, wobei sich Stickstoff und nitrose Gase entwickeln. Danach lässt man das Gemisch auf Raumtemperatur kommen, extrahiert mit Essigsäureethylester, wäscht die Extrakte mit Wasser und trocknet sie mit Natriumsulfat. Nach Entfernung des Lösungsmittels bleiben 27,7 g Rohprodukt als Rückstand, der chromatographisch auf Kieselgel mit einem Gemisch aus 20 Teilen Essigsäureethylester und 80 Teilen Hexan als Laufmittel gereinigt wird. Man erhält 32,2 g Reinsubstanz; Berechnungsindex $n_D^{24}$ = 1,5334.

| Analyse: $C_9H_{11}ClN_2$ | | |
|---|---|---|
| | % ber. | % gefunden |
| C | 59,18 | 59,16 |
| H | 6,07 | 6,15 |
| N | 15,34 | 15,25 |
| Cl | 19,41 | 19,20 |

Beispiel 1.9: 2-(α-Phenylhydrazino)-4,6-dimethyl-pyrimidin (Verb. 1.12)

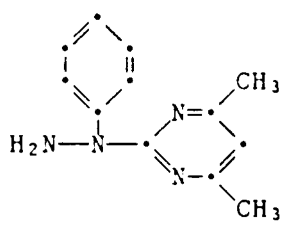

4,77 g (0,033 Mol) Phenylhydrazinhydrochlorid werden unter Stickstoff in 60 ml Tetrahydrofuran suspendiert und mit 7,41 g (0,066 Mol) K-tert.-Butylat versetzt. Sodann lässt man bei 25°-35°C eine Lösung von 5,59 g (0,030 Mol) 2-Methylsulfonyl-4,6-dimethylpyrimidin in 15 ml Tetrahydrofuran zutropfen. Nach 2 Stunden wird das Gemisch mit Essigsäureethylester und Wasser unter Zusatz von wenig Essigsäure bei pH6 extrahiert. Nach den Trocknen der organischen Phase mit Natriumsulfat und dem Entfernen des Lösungsmittels am Rotationsverdampfer erhält man 5,88 g Rohprodukt. Die chromatographische Reinigung mittels Kieselgel und einem Gemisch aus 35 Teilen Essigsäureethylester und 65 Teilen Hexan ergibt 2,89 g Reinsubstanz, die nach Umkristallisation aus n-Hexan bei 41-43°C schmilzt.

Beispiel 1.10: 2-(α-Phenylhydrazino)-4,6-dimethylpyrimidin (Verb. 1.12)

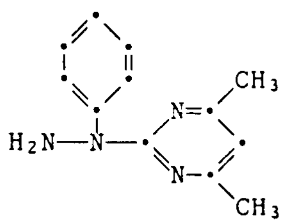

man suspendiert 7,06 g (0,035 Mol) 2-Trimethyl-ammonium-4,6-dimethylpyrimidinchlorid[1] und 5,78 g (0,04 Mol) Phenylhydrazin-Hydrochlorid in 50 ml Tetrahydofuran und tropft unter Stickstoffatmosphäre eine Lösung von 5,04 g (0,045 Mol) Kalium-tert.Butylat in 25 ml Tetrahydofuran zu. Die exotherme Reaktion wird durch Kühlung auf 5°-10°C gehalten.

[1] (W. KLötzer, Monatshefte f. Chemie 87, 131 (1956))

Nachdem sich das Gemisch auf 20°C erwärmt hat wird mit Essigsäureethylester und Wasser extrahiert und der Extrakt mit Natriumsulfat getrocknet. Nach dem Einengen erhält man 5,9 g Rohprodukt, das durch Säulenchromatographie (Kieselgel, Laufmittel Gemisch aus 25 Teilen Essigester und 75 Teilen Hexan) gereinigt wird. Man erhält 3,94 g Reinsubstanz.

Beispiel 1.11: N-(4-Methyl-6-cyclopropylpyrimidinyl-2)-N-phenyl-hydrazin (Verb. 1.4)

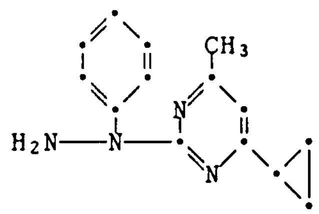

2,20 g (0,013 Mol) 2-Chlor-4-methyl-6-cyclopropylpyrimidin und 1,62 g (0,015 Mol) Phenylhydrazin werden in 20 ml Tetrahydrofuran gelöst. Zu dieser Lösung wird unter Kühlung bei 20-25°C eine Lösung von 2,02 g (0,018 Mol) Kalium-tert.-butylat in 20 ml Tetrahydrofuran zugetropft. Nach 30 Minuten lässt sich mittels Dünnschichtchromatographie kein Ausgangspyrimidin mehr nachweisen. Anschliessend wird das Gemisch mit Wasser und Essigsäureethylester extrahiert. Nach der Entfernung des Essigsäureethylesters am Rotationsverdampfer erhält man 3,13 g Rohprodukt, das durch Säulenchromatographie an Kieselgel (Laufmittel: 35 % Essigsäureethylester/65 % Hexan) gereinigt wird. Man erhält 2,83 g Reinsubstanz (90,2 % d.Th.), die aus einem Gemisch von 8 ml n-Hexan und 1 ml Cyclohexan umkristallisiert werden, woraus 1,62 g Substanz (Smp. 46°C) gewonnen werden. Einengen der Mutterlauge und erneute Umkristallisation ergibt weitere 0,42 g Produkt (Smp. 45-46°C). Die Gesamtausbeute an umkristallisiertem Produkt beträgt 2,04 g (65,3 % d.Th.).

| Analyse: $C_{14}H_{16}N_4$ (MG: 240,31) | | |
|---|---|---|
| | % ber. | % gefunden |
| C | 69,98 | 69,81 |
| H | 6,71 | 6,77 |
| N | 23,32 | 23,49 |

Beispiel 1.12: N-(4-methyl-6-cyclopropylpyrimidinyl-2)-N-phenyl-isobutyraldehydhydrazon (Verb. 3.26)

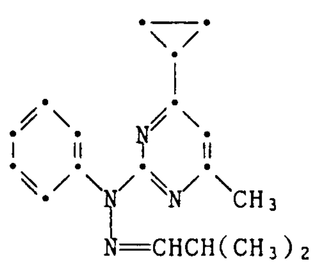

6,25 g (0,026 Mol) N-(4-Methyl-6-cyclopropylpyrimidinyl-2)-N-phenylhydrazin und 2,25 g (0,031 Mol) Isobutyraldehyd werden in 30 ml Methanol gelöst, wobei eine leicht exotherme Reaktion einsetzt. Nach zweistündigem Stehen bei Raumtemperatur wird das Lösungsmittel am Rotationsverdampfer entfernt. Man erhält 7,8 g Rohprodukt in Form eines dickflüssigen Oels. Davon werden 3,2 g durch Säulenchromatographie an Kieselgel gereinigt (Laufmittel: 72 % Hexan/18 % Essigsäureethylester/10 % Methanol). Die Reinausbeute beträgt 2,92 g Substanz mit Smp. 53-55°C. Dies entspricht einer Ausbeute von 93 % der Theorie bei Umrechnung auf die Reinigung des gesamten Rohprodukts.

| Analyse: $C_{18}H_{22}N_4$ (MG: 294,40) | | |
|---|---|---|
| | % ber. | % gefunden |
| C | 73,44 | 73,25 |
| H | 7,53 | 7,64 |
| N | 19,03 | 18,92 |

Beispiel 1.13: N-(4-Methyl-6-cyclopropylpyrimidinyl-2)-N-phenyl-N'-isobutylhydrazin (Verb. 4.87)

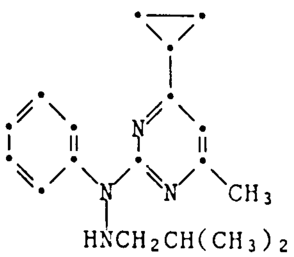

8,55 g (0,029 Mol) N-(4-Methyl-6-cyclopropylpyrimidinyl-2)-N-phenyl-isobutyraldehydhydrazon werden in 30 ml Methanol und 2 ml Eisessig gelöst. Danach werden unter Rühren 2,14 g (0,029 Mol) Natriumcyanborhydrid in Portionen zugegeben. Die Reaktion verläuft exotherm; man hält die Temperatur durch Kühlung auf 10-15°C. Nach 1 Stunde arbeitet man den Ansatz durch Extraktion mit Essigsäureethylester und Wasser auf, engt die organische Phase am Rotationsverdampfer ein und erhält 8,5 g Rohprodukt. Die Reinigung mittels Säulenchromatographie an Kieselgel (Laufmittel: 85 % Hexan/15 % Essigsäureethylester) ergibt 7,6 g (89 % d. Theorie) Oel, das einen Brechungsindex von $n_D^{25}$ = 1,5733 aufweist.

| Analyse: $C_{18}H_{24}N_4$ (MG: 296,42) | | |
|---|---|---|
| | % ber. | % gefunden |
| C | 72,94 | 72,90 |
| H | 8,16 | 8,21 |
| N | 18,90 | 18,83 |

Beispiel 1.14: N-(4-Methyl-6-methoxymethylpyrimidinyl-2)-N-phenyl-N'-methylhydrazin (Verb. 4.17)

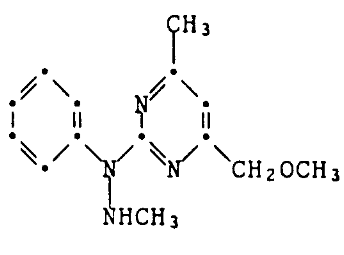

Man löst 3,70 g (0,033 Mol) Kalium-tert.-Butylat in 25 ml mit Molekularsieben getrocknetem Tetrahydrofuran, fügt 3,67 g (0,03 Mol) N-Methyl-N'-phenylhydrazin[2] hinzu und tropft dazu unter Stickstoff bei - 20°C eine Lösung von 4,22 g

[2] K. Kratzl, Monatshefte f. Chemie 89, 83 (1958)

(0,025 Mol) 2-Chlor-4-methyl-6-methoxymethylpyrimidin in 30 ml wasserfreiem Tetrahydrofuran. Dabei entsteht eine gelbbraune Suspension, die man allmählich auf Raumtemperatur kommen lässt. Nach 4 Stunden wird mit Wasser und Essigsäureethylester extrahiert, das Rohprodukt durch Abdampfen des Lösungsmittels isoliert und mittels Säulenchromatographie an Kieselgel gereinigt (Laufmittel: 65 % Hexan/ 35 % Essigsäureethylester). Man erhält das Reinprodukt als Oel, das einen Brechungsindex von $n_D^{24}$ = 1,5793 aufweist.

| Analyse: $C_{14}H_{18}N_4O$ (MG: 258,33) | | |
|---|---|---|
| | % ber. | % gefunden |
| C | 65,09 | 65,08 |
| H | 7,02 | 7,09 |
| N | 21,69 | 21,05 |

Beispiel 1.15: N-(4,6-Dimethylpyrimidinyl-2)-N-phenyl-N'-methylhydrazin (Verb. 4.1).

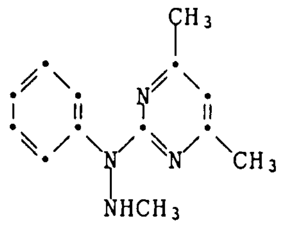

4,03 g (0,02 Mol) 2-Trimethylammonium-4,6-dimethylpyrimidin-hydrochlorid[3] werden mit 3,05 g (0,025 Mol) N-Methyl-N'-phenylhydrazin unter Stickstoff in 30 ml wasserfreiem Tetrahydrofuran gerührt und bei Raumtemperatur tropfenweise mit einer Lösung von 3,36 g (0,03 Mol) Kalium-tert.-Butylat in 15 ml Tetrahydrofuran versetzt. Man lässt über Nacht bei Raumtemperatur ausreagieren, extrahiert mit Wasser und Essigsäureethylester, entfernt das Lösungsmittel und reinigt das Rohprodukt durch Säulenchromatographie an Kieselgel (Laufmittel: 70 % Hexan/30 % Essigsäureethylester). Man erhält 3,09 g Substanz als Oel.

| Analyse: $C_{13}H_{16}N_4$ | | |
|---|---|---|
| | % ber. | % gefunden |
| C | 68,40 | 68,01 |
| H | 7,07 | 7,09 |
| N | 24,54 | 24,13 |

Beispiel 1.16: N-(4,6-Dimethylpyrimidinyl-2)-N-phenyl-N'-dimethylhydrazin (Verb. 4.13).

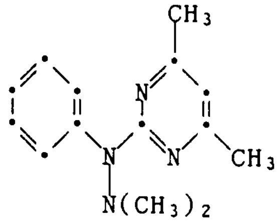

[3] W. Klötzer, Monatshefte f. Chemie 87, 131 (1956)

3,42 g (0,016 Mol) N-(4,6-Dimethylpyrimidinyl-2)-N-phenylhydrazin werden in 20 ml Methanol zusammen mit 3,28 g (0,042 Mol) 38%igem Formaldehyd und 2 ml Eisessig gelöst und bei ca. 5°C portionsweise mit 1,33 g (0,018 Mol) Natriumcyanborhydrid versetzt. Die Reaktion verläuft exotherm und ist nach einer Stunde beendet. Man extrahiert mit Essigsäureethylester und Wasser, entfernt das Lösungsmittel am Rotationsverdampfer und erhält 3,25 g Rohprodukt. Die chromatographische Reinigung mittels Kieselgel und einem Gemisch aus 76 % Hexan, 19 % Essigsäureethylester und 5 % Methanol ergibt 1,80 g (46,4 % d. Theorie) Reinsubstanz als Oel; $n_D^{25}$ = 1,5673.

Beispiel 1.17: N-(4-Methyl-6-cyclopropylpyrimidinyl-2)-N-phenyl-N′-methyl-N′-isobutylhydrazin (Verb. 4.102)

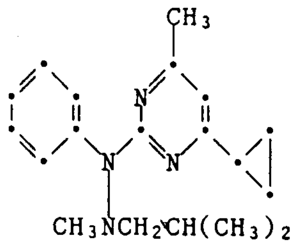

Man löst 4,55 g 0,0153 Mol) N-(4-Methyl-6-cyclopropylpyrimidinyl-2)-N-phenyl-N′-isobutylhydrazin mit 1,45 g (0,0184 Mol) 38%igem Formaldehyd in 25 ml Methanol und 2 ml Eisessig, und fügt bei 10°C in Portionen 1,24 g (0,0168 Mol) Natriumcyanborhydrid zu. Die Reaktion verläuft exotherm, und das Gemisch wird nach einer Stunde mit Wasser und Essigsäureethylester extrahiert. Nach der Entfernung des Lösungsmittels erhält man 4,95 g Rohprodukt, das durch Säulenchromatographie an Kieselgel gereinigt wird (Laufmittel: 85 % Hexan/15 % Essigsäureethylester). Die Ausbeute beträgt 4,4 g eines Oels; Brechungsindex $n_D^{30}$ = 1,5613.

| Analyse: $C_{19}H_{26}N_4$ (MG: 310,45) | | |
|---|---|---|
| | % ber. | % gefunden |
| C | 73,51 | 73,94 |
| H | 8,44 | 8,58 |
| N | 18,05 | 17,93 |

Tabelle 1: Verbindungen der Formel

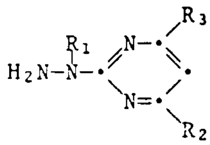

| Verb. Nr. | $R_1$ | $R_2$ | $R_3$ | physikalische Konstante |
|---|---|---|---|---|
| 1.1 | $C_6H_5$ | H | $CH_3$ | |
| 1.2 | $4-CF_3C_6H_4$ | $CH_3$ | $CH_3$ | |
| 1.3 | $4-F-C_6H_4$ | H | H | Smp.129-131°C |
| 1.4 | $C_6H_5$ | $CH_3$ | 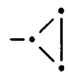 | Smp. 45-46°C |
| 1.5 | $C_6H_5$ | $C(CH_3)_3$ | $CH_2OCH_3$ | |
| 1.6 | $C_6H_5$ | $CH_2OCH_3$ | 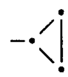 | Smp. 37-38°C |
| 1.7 | $C_6H_5$ | $CH_3$ | $CH_2OCH_3$ | Smp. 60-61°C |
| 1.8 | $C_6H_5$ | $CH_2OC_2H_5$ | $C(CH_3)_3$ | |
| 1.9 | $C_6H_5$ | $CH_2OCH(CH_3)C_2H_5$ | $CH_3$ | |
| 1.10 | $3,4-(C_2H_5O)_2-C_6H_3$ | $CH_3$ | $CH_2OC_2H_5$ | |
| 1.11 | $C_6H_5$ | $CH_3$ | $CH_2OC_2H_5$ | |
| 1.12 | $C_6H_5$ | $CH_3$ | $CH_3$ | Smp. 41-43°C |
| 1.13 | $4-CH_3O-C_6H_4$ | $C(CH_3)_3$ | $CH_2OC_2H_5$ | |
| 1.14 | $C_6H_5$ | $CH_3$ | $CHCl_2$ | |
| 1.15 | $3,5-Cl_2-C_6H_3$ | $CH_3$ | $CH_2OC_2H_5$ | |
| 1.16 | $3,5-Cl_2-C_6H_3$ | $CH_3$ | $CH_3$ | Smp.154-156°C |
| 1.17 | $3,5-Cl_2-C_6H_3$ | $C(CH_3)_3$ | $CH_2OC_2H_5$ | |
| 1.18 | $3,5-Cl_2-C_6H_3$ | 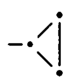 | $CH_2OCH_3$ | |
| 1.19 | $3,4-(C_2H_5O)_2-C_6H_3$ | $CH_2OC_2H_5$ | $C(CH_3)_3$ | |
| 1.20 | $4-CH_3O-C_6H_4$ | $CH_3$ | $CH_3$ | Smp. 90-92°C |
| 1.21 | $4-CH_3O-C_6H_4$ | $CH_2OC_2H_5$ | $CH_3$ | |
| 1.22 | $3,4-(C_2H_5O)_2-C_6H_3$ | 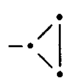 | $CH_2OCH_3$ | |
| 1.23 | $C_6H_5$ | $CH_3$ | $CH_2OCH_2CH=CH_2$ | |
| 1.24 | $4-CH_3-C_6H_4$ | $CH_3$ | $CH_3$ | $n_D^{30}$ 1,604 |

Fortsetzung: Tabelle 1

| Verb. Nr. | $R_1$ | $R_2$ | $R_3$ | physikalische Konstante |
|---|---|---|---|---|
| 1.25 | $4-OCH_3-C_6H_4$ | 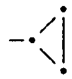 | $CH_2OCH_3$ | |
| 1.26 | $3,5-Cl_2-C_6H_3$ | $CH_3$ | $CH_2OCH(CH_3)C_2H_5$ | |
| 1.27 | $4-OCH_3$ | $CH_3$ | $CH_2OCH(CH_3)C_2H_5$ | |
| 1.28 | $2-Br-C_6H_4$ | $CH_3$ | $CH_3$ | Smp. 55-57°C |
| 1.29 | $3,5-Cl_2-C_6H_3$ | $CH_2OCH_2CH=CH_2$ | $CH_3$ | |
| 1.30 | $4-CH_3O-C_6H_4$ | $C(CH_3)_3$ | $CH_2OCH_3$ | |
| 1.31 | $3,5-Cl_2-C_6H_3$ | $CH_3$ | $CHCl_2$ | |
| 1.32 | $3-Cl-C_6H_4$ | $CH_3$ | $CH_3$ | Smp. 47-48°C |
| 1.33 | $3,4-(C_2H_5O)_2-C_6H_3$ | $CH_3$ | $CH_2OCH(CH_3)C_2H_5$ | |
| 1.34 | $4-CH_3O-C_6H_4$ | $CH_3$ | $CH_2OCH_3$ | |
| 1.35 | $3,4-(C_2H_5O)_2-C_6H_3$ | $C(CH_3)_3$ | $CH_2OCH_3$ | |
| 1.36 | $3,5-Cl_2-C_6H_3$ | $CH_3$ | $CH_2OCH_3$ | |
| 1.37 | $2,4,6-Cl_3-C_6H_2$ | $CH_3$ | $CH_3$ | Smp.150-152°C |
| 1.38 | $3,4-(C_2H_5O)_2-C_6H_3$ | $CH_3$ | $CH_2OCH_3$ | |
| 1.39 | $3,5-Cl_2-C_6H_3$ | $CH_2OCH_2C{\equiv}CH$ | $CH_3$ | |
| 1.40 | $C_6H_5$ | $CH_2OCH_2C{\equiv}CH$ | $CH_3$ | |
| 1.41 | $4-CH_3O-C_6H_4$ | $CH_2OCH_2C{\equiv}CH$ | $CH_3$ | |
| 1.42 | $3,5-(CF_3)_2-C_6H_3$ | $CH_3$ | $CH_3$ | Smp. 88-90°C |
| 1.43 | $C_6H_5$ | $CH_2OCH_3$ | $CH_2OCH_3$ | |
| 1.44 | $C_6H_5$ | $CH_2SCH_3$ | $CH_3$ | |
| 1.45 | $C_6H_5$ | $CH_3$ | $CH(OCH_3)_2$ | |
| 1.46 | $C_6H_5$ | $CH_3$ | $CH(OC_2H_5)_2$ | |

Fortsetzung: Tabelle 1

| Verb. Nr. | $R_1$ | $R_2$ | $R_3$ | physikalische Konstante |
|---|---|---|---|---|
| 1.47 | $C_6H_5$ | 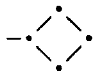 | $CH_3$ | |
| 1.48 | 4-Br-$C_6H_4$ | $CH_3$ | $CH_3$ | Smp. 92-93°C |
| 1.49 | $C_6H_5$ | $CH_3$ | Cyclo-$C_6H_{11}$ | |
| 1.50 | $C_6H_5$ | $CH_3$ | $CF_2Cl$ | |
| 1.51 | $C_6H_5$ | 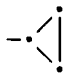 | $CF_3$ | |
| 1.52 | $C_6H_5$ | 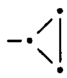 | 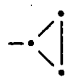 | |
| 1.53 | $C_6H_5$ | 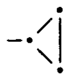 | $CF_2Cl$ | |
| 1.54 | 3-$CH_3$-$C_6H_4$ | $CH_3$ | $CH_3$ | Smp. 72-73°C |
| 1.55 | $C_6H_5$ | $C_2H_5$ | 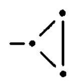 | $n_D^{25}$ 1,6063 |
| 1.56 | $C_6H_5$ | $CH_3$ | 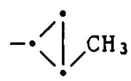 | $n_D^{30}$ 1,6072 |
| 1.57 | $C_6H_5$ | $CH(CH_3)_2$ | 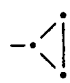 | |
| 1.58 | $C_6H_5$ | $CH_2OCH_3$ | $CF_3$ | |
| 1.59 | $C_6H_5$ | 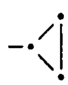 | $CH(OCH_3)_2$ | |
| 1.60 | $C_6H_5$ | $CH_2OCH_3$ | $CH_2CH(CH_3)_2$ | |
| 1.61 | $C_6H_5$ | CHO | 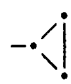 | |
| 1.62 | $C_6H_5$ | $CH_2OCH_3$ | 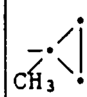 | |
| 1.63 | $C_6H_5$ | $CH_2CH_2CH_3$ | $CH_2OCH_3$ | |
| 1.64 | $C_6H_5$ | $CH(CH_3)_2$ | $CH_2OCH_3$ | |
| 1.65 | 4-F-$C_6H_4$ | $CH_3$ | 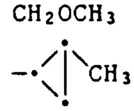 | $n_D^{25}$ 1,5883 |
| 1.66 | $C_6H_5$ | $CH_2OH$ | 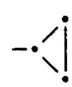 | |

Fortsetzung: Tabelle 1

| Verb. Nr. | $R_1$ | $R_2$ | $R_3$ | physikalische Konstante |
|---|---|---|---|---|
| 1.67 | $C_6H_5$ | $(CH_2)_3CH_3$ | $CH_2OCH_3$ | |
| 1.68 | $C_6H_5$ | $C_2H_5$ | $CH_2OCH_3$ | $n_D^{24}$ 1,5923 |
| 1.69 | $C_6H_5$ | $CH_2OCH_3$ | 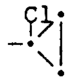 | |
| 1.70 | $C_6H_5$ | $CH_2OCH_3$ | $CF_2Cl$ | |
| 1.71 | $C_6H_5$ | 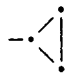 | $CH_2Cl$ | |
| 1.72 | $C_6H_5$ | $CH_2OCH_3$ | $CH(OCH_3)_2$ | |
| 1.73 | $4-F-C_6H_4$ | $CH_3$ | $CH_3$ | Smp. 78-79°C |
| 1.74 | $4-Cl-C_6H_4$ | $CH_3$ | $CH_3$ | Smp. 101-102°C |
| 1.75 | $4-F-C_6H_4$ | $CH_3$ | $C_2H_5$ | $n_D^{25}$ 1,5723 |
| 1.76 | $C_6H_5$ | 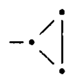 | $CHCl_2$ | |
| 1.77 | $C_6H_5$ | 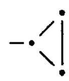 | $CH_2Br$ | |
| 1.78 | $C_6H_5$ | $CH_2OCH_3$ | $CF_2CF_3$ | |
| 1.79 | $C_6H_5$ | 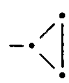 | $CH_2F$ | $n_D^{32}$ 1,6150 |
| 1.80 | $C_6H_5$ | $CH(CH_3)C_2H_5$ | $CH_2OCH_3$ | |
| 1.81 | $C_6H_5$ | CHO | $CH_2OCH_3$ | |
| 1.82 | $C_6H_5$ | $(CH_2)_3CH_3$ | 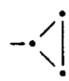 | |
| 1.83 | $C_6H_5$ | $(CH_2)_2CH_3$ | 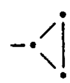 | |
| 1.84 | $C_6H_5$ | 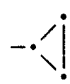 | $CH(CH_3)C_2H_5$ | |
| 1.85 | $C_6H_5$ | 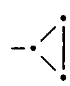 | $CH_2CH(CH_3)_2$ | |
| 1.86 | $C_6H_5$ | 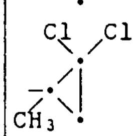 | 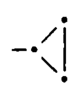 | |
| 1.87 | $4-CF_3-C_6H_4$ | $CH_3$ | $CH_2OCH_3$ | |
| 1.88 | $4-Br-C_6H_4$ | $CH_3$ | $CH_2OCH_3$ | |

Fortsetzung: Tabelle 1

| Verb. Nr. | $R_1$ | $R_2$ | $R_3$ | physikalische Konstante |
|---|---|---|---|---|
| 1.89 | $C_6H_5$ | $CH_2OH$ | $CH_2OCH_3$ | |
| 1.90 | $C_6H_5$ | 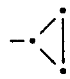 | $CF_2CF_3$ | |
| 1.91 | $C_6H_5$ | $CH_2OCH_3$ | $CH_2Cl$ | |
| 1.92 | $4\text{-}CH_3\text{-}C_6H_4$ | $CH_2OCH_3$ | $CH_3$ | |
| 1.93 | $C_6H_5$ | $CH(OC_2H_5)_2$ | 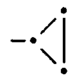 | $n_D^{25}$ 1,5763 |
| 1.94 | $2\text{-}CF_3\text{-}C_6H_4$ | $CH_3$ | $CH_2OCH_3$ | |
| 1.95 | $C_6H_5$ | $CH_3$ | 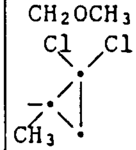 | |
| 1.96 | $C_6H_5$ | $CH_3$ | CHO | |
| 1.97 | $C_6H_5$ | 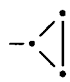 | 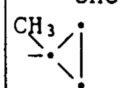 | |
| 1.98 | $4\text{-}Cl\text{-}C_6H_4$ | $CH_3$ | $CH_2OCH_3$ | |
| 1.99 | $4\text{-}Cl\text{-}3\text{-}CF_3\text{-}C_6H_3$ | $CH_3$ | $CH_2OCH_3$ | |
| 1.100 | $3\text{-}CH_3\text{-}C_6H_4$ | $CH_3$ | $CH_2OCH_3$ | |
| 1.101 | $3\text{-}F\text{-}C_6H_4$ | $CH_3$ | $CH(OC_2H_5)_2$ | |
| 1.102 | $3\text{-}CF_3\text{-}C_6H_4$ | $CH_2OCH_3$ | $CH_3$ | |
| 1.103 | $3\text{-}Cl\text{-}C_6H_4$ | $CH_2OCH_3$ | $CH_3$ | |
| 1.104 | $3\text{-}F\text{-}C_6H_4$ | $CH_2OCH_3$ | $CH_3$ | $n_D^{24}$ 1,5863 |
| 1.105 | $4\text{-}F\text{-}C_6H_4$ | $CH_2OCH_3$ | $CH_3$ | Smp. 70-72°C |
| 1.106 | $4\text{-}Cl\text{-}C_6H_4$ | $CH_3$ | 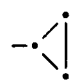 | |

Fortsetzung: Tabelle 1

| Verb. Nr. | $R_1$ | $R_2$ | $R_3$ | physikalische Konstante |
|---|---|---|---|---|
| 1.107 | $C_6H_5$ | $CH_3$ | $CH_2OH$ | |
| 1.108 | 4-F-$C_6H_4$ | $CH_3$ | 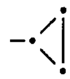 | Smp. 93-95°C |
| 1.109 | $C_6H_5$ | $CH_2OCH_3$ | $CH_2Br$ | |
| 1.110 | $C_6H_5$ | $CH_3$ | $CH_2Cl$ | |
| 1.111 | 4-Cl-3-$CF_3$-$C_6H_3$ | 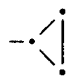 | $CH_3$ | |
| 1.112 | $C_6H_5$ | $CH_2OCH_3$ | $CH_2F$ | |
| 1.113 | $C_6H_5$ | 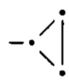 | 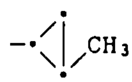 | |
| 1.114 | $C_6H_5$ | $CH_3$ | $CH_2Br$ | |
| 1.115 | $C_6H_5$ | $CH_3$ | $CH_2F$ | |
| 1.116 | $C_6H_5$ | $C(CH_3)_3$ | $C(CH_3)_3$ | |
| 1.117 | $C_6H_5$ | $(CH_2)_4CH_3$ | $CH_3$ | |
| 1.118 | $C_6H_5$ | $C_2H_5$ | $C_2H_5$ | $n_D^{25}$ 1,5923 |
| 1.119 | $C_6H_5$ | $CH_3$ | $C_2H_5$ | $n_D^{30}$ 1,5953 |
| 1.120 | $C_6H_5$ | $CH_3$ | $C\equiv CH$ | |
| 1.121 | $C_6H_5$ | $CH_3$ | $C\equiv CCH_3$ | |
| 1.122 | 3-F-$C_6H_4$ | $CH_3$ | 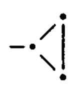 | Smp. 41-42°C |
| 1.123 | 3-F-$C_6H_4$ | 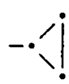 | $CH_2F$ | |
| 1.124 | 3-F-$C_6H_4$ | $CH_3$ | 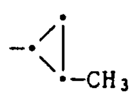 | $n_D^{25}$ 1,5943 |
| 1.125 | $C_6H_5$ | H | 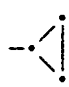 | |
| 1.126 | $C_6H_5$ | $CH_2CH_2CH(CH_3)_2$ | $CH_3$ | |

Fortsetzung: Tabelle 1

| Verb. Nr. | $R_1$ | $R_2$ | $R_3$ | physikalische Konstante |
|---|---|---|---|---|
| 1.127 | 4-F-$C_6H_4$ | $C_2H_5$ | $C_2H_5$ | $n_D^{25}$ 1,5743 |
| 1.128 | 4-F-$C_6H_4$ | $CH_2OCH_3$ | 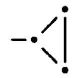 | Smp. 56-57°C |
| 1.129 | 3-F-$C_6H_4$ | $CH_3$ | $CH_3$ | Smp. 60-62°C |
| 1.130 | 3-F-$C_6H_4$ | $CH_3$ | $C_2H_5$ | $n_D^{30}$ 1,5873 |
| 1.131 | 3-F-$C_6H_4$ | $CH_2OCH_3$ | 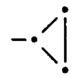 | $n_D^{25}$ 1,5873 |
| 1.132 | $C_6H_5$ | $CH_3$ | $CF_3$ | $n_D^{24}$ 1,5543 |
| 1.133 | 4-F-$C_6H_4$ | $CH_2OCH_3$ | $C_2H_5$ | $n_D^{24}$ 1,5733 |
| 1.134 | 2-F-$C_6H_4$ | $CH_3$ | $CH_3$ | $n_D^{23}$ 1,5863 |
| 1.135 | 2-F-$C_6H_4$ | $CH_3$ | $CH_2OCH_3$ | $n_D^{23}$ 1,5773 |
| 1.136 | 2-F-$C_6H_4$ | $CH_3$ | 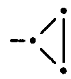 | $n_D^{37}$ 1,5811 |
| 1.137 | 4-F-$C_6H_4$ | $C_2H_5$ | 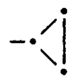 | $n_D^{22}$ 1,5813 |
| 1.138 | 3-F-$C_6H_4$ | $C_2H_5$ | 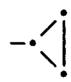 | $n_D^{23}$ 1,5894 |
| 1.139 | $C_6H_5$ | $CH_2CH_2CH_3$ | $CH_2CH_2CH_3$ | $n_D^{23}$ 1,5743 |
| 1.140 | 3-F-$C_6H_4$ | $CH_2CH_2CH_3$ | $CH_2CH_2CH_3$ | $n_D^{23}$ 1,5633 |
| 1.141 | 2-F-$C_6H_4$ | 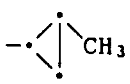 | $CH_3$ | $n_D^{24}$ 1,5854 |
| 1.142 | $C_6H_5$ | H | H | Smp. 75-77°C |

Tabelle 2: Verbindungen der Formel

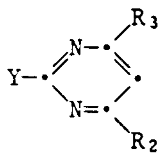

| Verb. Nr. | $R_2$ | $R_3$ | Y | physikalische Konstante |
|---|---|---|---|---|
| 2.1 | CHO | 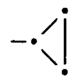 | Cl | $n_D^{25}$ 1,5603 |
| 2.2 | t-butyl | $CH_2OC_2H_5$ | Cl | |
| 2.3 | $CH_3$ | $CH_2OCH(CH_3)C_2H_5$ | Cl | |
| 2.4 | $CH_3$ | $CH_2OC_2H_5$ | Cl | |
| 2.5 | $CH_3$ | $CHCl_2$ | Cl | |
| 2.6 | $CH_3$ | $CH_2OCH_2CH=CH_2$ | Cl | |
| 2.7 | $CH_3$ | $CH_2OCH_2C\equiv CH$ | Cl | |
| 2.8 | $CH_2OCH_3$ | $CH_2OCH_3$ | Cl | |
| 2.9 | $CH_2OCH_3$ | 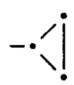 | Cl | $n_D^{25}$ 1,5344 |
| 2.10 | $CH_3$ | $CH(OCH_3)_2$ | Cl | |
| 2.11 | $CH_3$ | $CH(OC_2H_5)_2$ | Cl | |
| 2.12 | $CH_3$ | $CF_2Cl$ | Cl | |
| 2.13 | $CH_2OCH_3$ | $CF_3$ | Cl | |
| 2.14 | $CH(OC_2H_5)_2$ | 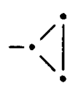 | Cl | Oel |
| 2.15 | n-propyl | $CH_2OCH_3$ | Cl | |
| 2.16 | i-propyl | $CH_2OCH_3$ | Cl | |
| 2.17 | n-butyl | $CH_2OCH_3$ | Cl | |
| 2.18 | $CH_2OCH_3$ | $C_2H_5$ | Cl | $n_D^{24}$ 1,5064 |
| 2.19 | $CH_2OCH_3$ | $CH(OCH_3)_2$ | Cl | |
| 2.20 | $CH_2OCH_3$ | $CF_2CF_3$ | Cl | |
| 2.21 | $CH_2OH$ | $CH_2OCH_3$ | Cl | |
| 2.22 | $CH_2OCH_3$ | $CH_2Cl$ | Cl | |
| 2.23 | $CH_2OCH_3$ | $CH_2F$ | Cl | |
| 2.24 | $CH_3$ | $CH_2OCH_3$ | $SO_2CH_3$ | |

Tabelle 3: Verbindungen der Formel

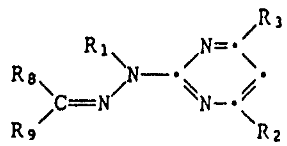

| Verb. Nr. | $R_1$ | $R_2$ | $R_3$ | $R_8$ | $R_9$ | physikal. Konstante |
|---|---|---|---|---|---|---|
| 3.2 | $C_6H_5$ | $CH_3$ | $CF_3$ | H | $C_2H_5$ | |
| 3.3 | $C_6H_5$ | $CH_3$ | $CH_3$ | H | $CH_3$ | Smp.117-118°C |
| 3.4 | $C_6H_5$ | $CH_3$ | cyclo-$C_3H_5$ | H | $CH_3$ | Smp.137-138°C |
| 3.5 | $C_6H_5$ | $CH_3$ | cyclo-$C_3H_5$ | H | $C_2H_5$ | Smp.103-105°C |
| 3.6 | $C_6H_5$ | $CH_3$ | $CH_3$ | H | $C_2H_5$ | Smp. 46-49°C |
| 3.7 | $C_6H_5$ | $CH_3$ | $CH_2OCH_3$ | H | $C_2H_5$ | Smp. 51-53°C |
| 3.8 | $C_6H_5$ | $CH_3$ | cyclo-$C_3H_5$ | H | n-$C_3H_7$ | $n_D^{25}$ 1,5862 |
| 3.9 | $C_6H_5$ | $CH_3$ | $C_2H_5$ | $CH_3$ | $CH_3$ | |
| 3.10 | 3-F-$C_6H_4$ | $CH_3$ | $CH_2OCH_3$ | $CH_3$ | $CH_3$ | $n_D^{24}$ 1,5678 |
| 3.11 | $C_6H_5$ | $CH_3$ | $CH_3$ | $C_2H_5$ | $CH_3$ | $n_D^{23}$ 1,5830 |
| 3.12 | 4-$NO_2$-$C_6H_4$ | $C_2H_5$ | $C_2H_5$ | H | n-$C_3H_7$ | |
| 3.14 | $C_6H_5$ | $CH_3$ | cyclo-$C_3H_5$ | $CH_3$ | $CH_3$ | $n_D^{22}$ 1,5982 |
| 3.15 | $C_6H_5$ | $CH_3$ | cyclo-$C_3H_5$ | $CH_3$ | 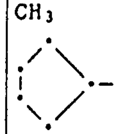 | |
| 3.16 | $C_6H_5$ | $CH_3$ | cyclo-$C_3H_5$ | $CH_3$ | $CH_2OCH_3$ | |

Tabelle 3: (Fortsetzung)

| Verb. | $R_1$ | $R_2$ | $R_3$ | $R_8$ | $R_9$ | physikal. Konstante |
|---|---|---|---|---|---|---|
| 3.17 | $4-CF_3O-C_6H_4$ | $CH_3$ | cyclo-$C_3H_5$ | H | $(CH_3)_2CH$ | |
| 3.18 | $4-CF_3O-C_6H_4$ | $CH_3$ | cyclo-$C_3H_5$ | H | $CH_3$ | |
| 3.19 | $3-F-C_6H_4$ | $CH_3$ | $CH_2OCH_3$ | H | $C(CH_3)_3$ | Smp.104-106°C |
| 3.20 | $3-F-C_6H_4$ | $CH_3$ | $CH_2OCH_3$ | H | $C_2H_5$ | Smp. 95-97°C |
| 3.21 | $4-CH_3O-C_6H_4$ | $CH_3$ | $CH_3$ | H | $C_2H_5$ | Smp. 81-82°C |
| 3.22 | $4-CH_3-C_6H_4$ | $(CH_3)_3C$ | $CH_3$ | H | $n-C_3H_7$ | |
| 3.23 | $4-CH_3-C_6H_4$ | $n-C_3H_7$ | $n-C_3H_7$ | H | $C_2H_5$ | |
| 3.24 | $C_6H_5$ | $C_2H_5$ | $C_2H_5$ | H | $C_2H_5$ | |
| 3.25 | $C_6H_5$ | $CH_3$ | $CH_3$ | H | $(CH_3)_2CH$ | Smp. 83-84°C |
| 3.26 | $C_6H_5$ | $CH_3$ | cyclo-$C_3H_5$ | H | $(CH_3)_2CH$ | Smp. 53-55°C |
| 3.27 | $C_6H_5$ | $CH_3-C{\equiv}C$ | cyclo-$C_3H_5$ | H | $CH_3$ | |
| 3.29 | $4F-C_6H_4$ | $CH_3$ | cyclo-$C_3H_5$ | $CH_3$ | $CF_3$ | |
| 3.30 | $4-(CH_3)_2CH-C_6H_4$ | $CH_3$ | $CH_3$ | $C_2H_5$ | $CH_3$ | |
| 3.31 | $C_6H_5$ | $CH_3$ | $CH_3$ | $-(CH_2)_5-$ | | |
| 3.32 | $2-F-C_6H_4$ | $CH_3$ | $n-C_3H_7$ | $-(CH_2)_4-$ | | |
| 3.33 | $4-J-C_6H_4$ | $CH_3$ | $CH_3$ | $C_2H_5$ | H | |
| 3.34 | $4-J-C_6H_4$ | cyclo-$C_3H_5$ | $CH_3$ | $(CH_3)_2CH$ | H | |
| 3.36 | $C_6H_5$ | cyclo-$C_3H_5$ | $CH_3$ | $CF_3$ | H | |
| 3.38 | $C_6H_5$ | $C(CH_3)_3$ | $C(CH_3)_3$ | $(CH_3)_2CH$ | H | |
| 3.39 | $C_6H_5$ | $C(CH_3)_3$ | $C(CH_3)_3$ | $C_2H_5$ | $CH_3$ | |
| 3.40 | $C_6H_5$ | $CH_3$ | cyclo-$C_3H_5$ | H | $CCl_3$ | Smp.139-141°C |

Fortsetzung: (Tabelle 3)

| Verb. | $R_1$ | $R_2$ | $R_3$ | $R_8$ | $R_9$ | physikal. Konstante |
|---|---|---|---|---|---|---|
| 3.48 | $4-(CH_3)_2CH-C_6H_4$ | $CH_3$ | $CH_3$ | $-(CH_2)_3-$ | | |
| 3.49 | $4-Br-C_6H_4$ | $CH_3$ | $CH_3$ | H | 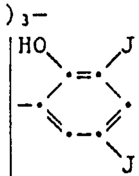 | Smp. 255-256°C |
| 3.50 | $4-F-C_6H_4$ | $cyclo-C_3H_5$ | $CH_3$ | $CH_3$ | H | Smp. 162-164°C |
| 3.51 | $4-F-C_6H_4$ | $cyclo-C_3H_5$ | $CH_3$ | $CH_3$ | $CH_3$ | |
| 3.52 | $4-F-C_6H_4$ | $cyclo-C_3H_5$ | $CH_3$ | $(CH_3)_2CH$ | H | Smp. 58-60°C |
| 3.57 | $C_6H_5$ | $CH_3$ | $CH_3$ | H | $CH_2=CH-$ | |
| 3.58 | $C_6H_5$ | $CH_3$ | $CH_3$ | $-CH=CHCH_2CH_2CH_2-$ | | |
| 3.59 | $C_6H_5$ | $CH_3$ | $cyclo-C_3H_5$ | $-CH=CHCH_2CH_2-$ | | |
| 3.60 | $C_6H_5$ | $CH_3$ | $CH_3$ | $-(CH_2)_4-$ | | |

Tabelle 3: (Fortsetzung)

| Verb. | $R_1$ | $R_2$ | $R_3$ | $R_8$ | $R_9$ | physikal. Konstante |
|---|---|---|---|---|---|---|
| 3.61 | $C_6H_5$ | $CH_3$ | cyclo-$C_3H_5$ | $CH_3$ | $CF_3$ | |
| 3.62 | $C_6H_5$ | $CH_3$ | cyclo-$C_3H_5$ | $CF_3$ | $CF_3$ | |
| 3.63 | 3,5-$(CF_3)_2$-$C_6H_3$ | $CH_3$ | $CH_3$ | $C_2H_5$ | H | $n_D^{25}$ 1,4990 |
| 3.64 | $C_6H_5$ | $CH_3$ | cyclo-$C_3H_5$ | H | 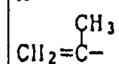 | |
| 3.65 | $C_6H_5$ | $CH_3$ | cyclo-$C_3H_5$ | H | 2-Pyridyl | |
| 3.66 | $C_6H_5$ | $CH_3$ | cyclo-$C_3H_5$ | H | 3-Pyridyl | |
| 3.67 | $C_6H_5$ | $CH_3$ | cyclo-$C_3H_5$ | H | 4-Pyridyl | |
| 3.68 | $C_6H_5$ | $CH_3$ | $CH_2OCH_3$ | H | 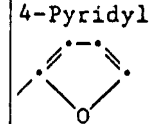 | Smp.155-156°C |
| 3.69 | $C_6H_5$ | $CH_3$ | $CH_3$ | H | 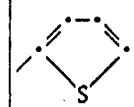 | Smp.177-179°C |
| 3.74 | $C_6H_5$ | $CH_3$ | $CH_2OCH_3$ | $CH_3$ | $CH_2N(CH_3)_2$ | |
| 3.75 | $C_6H_5$ | $CH_3$ | cyclo-$C_3H_5$ | H | $CHCl_2$ | |
| 3.76 | $C_6H_5$ | $CH_3$ | cyclo-$C_3H_5$ | H | $CBr_3$ | |

Tabelle 3: (Fortsetzung)

| Verb. | $R_1$ | $R_2$ | $R_3$ | $R_8$ | $R_9$ | physikal. Konstante |
|---|---|---|---|---|---|---|
| 3.78 | 3-F-$C_6H_4$ | $CH_3OCH_2$ | $CH_3$ | H | $(CH_3)_2CH$ | Smp. 83-84°C |
| 3.79 | $C_6H_5$ | $CH_3OCH_2$ | $CH_3$ | H | $(CH_3)_2CH$ | $n_D^{35}$ 1,5673 |
| 3.81 | 4-$CF_3$-$C_6H_4$ | $CH_3$ | $CH_3$ | $CH_3$ | $CH_3$ | |
| 3.82 | 4-$CF_3$-$C_6H_4$ | $CH_3$ | $CH_3$ | H | $C_2H_5$ | |
| 3.84 | $C_6H_5$ | $CH_3$-C≡C | cyclo-$C_3H_5$ | H | $C_2H_5$ | |
| 3.86 | 3-F-$C_6H_4$ | cyclo-$C_3H_5$ | $CH_3$ | H | 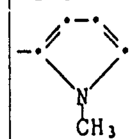 | Smp.111-112°C |
| 3.87 | $C_6H_5$ | $CH_2OCH_3$ | $CH_3$ | $CH_3$ | H | Smp.112-114°C |
| 3.89 | 3-F-$C_6H_4$ | $CH_3$ | cyclo-$C_3H_5$ | H | $CH_3$ | Smp.114-116°C |
| 3.90 | 3-F-$C_6H_4$ | $CH_3$ | cyclo-$C_3H_5$ | H | $C_2H_5$ | |
| 3.91 | 3-F-$C_6H_4$ | $CH_3$ | cyclo-$C_3H_5$ | H | n-$C_3H_7$ | Smp. 46-47°C |
| 3.92 | 3-F-$C_6H_4$ | $CH_3$ | cyclo-$C_3H_5$ | H | $(CH_3)_2CH$ | Smp. 45-46°C |

Tabelle 3: (Fortsetzung)

| Verb. | $R_1$ | $R_2$ | $R_3$ | $R_8$ | $R_9$ | physikal. Konstante |
|---|---|---|---|---|---|---|
| 3.93 | $C_6H_5$ | $CH_3$ | cyclo-$C_3H_5$ | H | $-CH=CH_2$ | |
| 3.94 | 4-F-$C_6H_4$ | $CH_3$ | cyclo-$C_3H_5$ | $C_2H_5$ | H | Smp. 79-80°C |
| 3.95 | 4-F-$C_6H_4$ | $CH_3$ | cyclo-$C_3H_5$ | n-$C_3H_7$ | H | Smp. 80-81°C |
| 3.96 | $C_6H_5$ | $CH_3$ | cyclo-$C_3H_5$ | H | 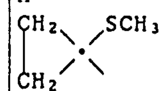 | |
| 3.97 | $C_6H_5$ | $CH_3$ | cyclo-$C_3H_5$ | $C_2H_5$ | $CH_3$ | |
| 3.98 | 3-F-$C_6H_4$ | $CH_3$ | cyclo-$C_3H_5$ | $CH_3$ | $CH_3$ | |
| 3.99 | 3-F-$C_6H_4$ | $CH_3$ | cyclo-$C_3H_5$ | $CH_3$ | $C_2H_5$ | |
| 3.100 | 3-F-$C_6H_4$ | $CH_3$ | cyclo-$C_3H_5$ | $CH_3$ | $CF_3$ | |
| 3.101 | 4-F-$C_6H_4$ | $CH_3$ | cyclo-$C_3H_5$ | $CCl_3$ | H | |
| 3.104 | 3-F-$C_6H_4$ | $CH_2OCH_3$ | $CH_3$ | H | 2-Pyridyl | |
| 3.108 | $C_6H_5$ | cyclo-$C_3H_5$ | $CH_3$ | H | 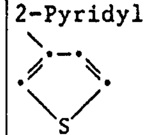 | Smp. 90-92°C |
| 3.109 | $C_6H_5$ | cyclo-$C_3H_5$ | $CH_3$ | H | cyclo-$C_3H_5$ | |
| 3.112 | $C_6H_5$ | $CH_3$ | $CH_3$ | $CH_3$ | $(CH_2)_7CH_3$ | |

Tabelle 3: (Fortsetzung)

| Verb. | $R_1$ | $R_2$ | $R_3$ | $R_8$ | $R_9$ | physikal. Konstante |
|---|---|---|---|---|---|---|
| 3.116 | 3-F-$C_6H_4$ | $CH_3$ | $CH_2OCH_3$ | $CH_3$ | H | Smp.144-145°C |
| 3.117 | $C_6H_5$ | $CH_3$ | $CH_2OCH_3$ | H | n-$C_3H_7$ | $n_D^{25}$ 1,5756 |
| 3.118 | $C_6H_5$ | $CH_3$ | $CH_3$ | H | $C(CH_3)_3$ | Smp. 73-74°C |
| 3.119 | $C_6H_5$ | $CH_3$ | $CH_2OCH_3$ | H | $C(CH_3)_3$ | Smp.100-101°C |
| 3.120 | $C_6H_5$ | $CH_3$ | $CH_3$ | H | n-$C_3H_7$ | $n_D^{25}$ 1,5860 |
| 3.121 | 3-F-$C_6H_4$ | $CH_3$ | $CH_2OCH_3$ | H | n-$C_3H_7$ | Smp. 47-48°C |
| 3.122 | 4-F-$C_6H_4$ | $CH_3$ | 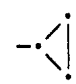 | H | $C(CH_3)_3$ | Smp.127-128°C |

Tabelle 4: Verbindungen der Formel

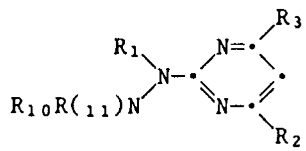

| Verb. | $R_1$ | $R_2$ | $R_3$ | $R_{10}$ | $R_{11}$ | physikal. Konstante |
|---|---|---|---|---|---|---|
| 4.1 | $C_6H_5$ | $CH_3$ | $CH_3$ | H | $CH_3$ | $n_D^{25}$ 1,5963 |
| 4.2 | $C_6H_5$ | $CH_3$ | $CH_3$ | H | $C_2H_5$ | |
| 4.3 | $C_6H_5$ | $CH_3$ | $CH_3$ | H | $CH_2CH=CH_2$ | |
| 4.4 | $C_6H_5$ | $CH_3$ | $CH_3$ | H | $n-C_3H_7$ | $n_D^{24}$ 1,5740 |
| 4.5 | $C_6H_5$ | cyclo-$C_3H_5$ | $CH_3$ | H | $CH_2C \equiv CH$ | |
| 4.6 | $4-CH_3O-C_6H_4$ | $CH_3$ | $CH_3$ | H | $n-C_5H_{11}$ | |
| 4.7 | $C_6H_5$ | $CH_3$ | cyclo-$C_3H_5$ | 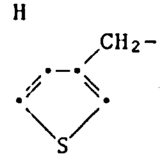 | H | $n_D^{25}$ 1,6160 |
| 4.8 | $3,4-(CH_3)_2-C_6H_3$ | $CH_3$ | $CH_3$ | 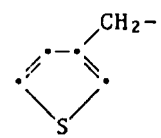 | H | |

Tabelle 4: (Fortsetzung)

| Verb. | $R_1$ | $R_2$ | $R_3$ | $R_{10}$ | $R_{11}$ | physikal. Konstante |
|---|---|---|---|---|---|---|
| 4.9 | $4-CF_3-C_6H_4$ | $CH_3$ | $CH_3$ | 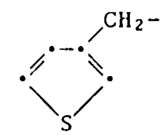 | $CH_3$ | |
| 4.10 | $3-F-C_6H_4$ | $CH_3$ | cyclo-$C_3H_5$ | n-$C_3H_7$ | n-$C_3H_7$ | $n_D^{27}$ 1,5510 |
| 4.11 | $C_6H_5$ | $CH_2OCH_3$ | $CH_3$ | $CH_2C(CH_3)_3$ | H | |
| 4.12 | $C_6H_5$ | $CH_2OCH_3$ | $CH_3$ | 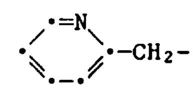 | H | |
| 4.13 | $C_6H_5$ | $CH_3$ | $CH_3$ | $CH_3$ | $CH_3$ | $n_D^{25}$ 1,5673 |
| 4.14 | $C_6H_5$ | $CH_3$ | $CH_3$ | $C_6H_5CH_2-$ | H | Smp. 56-57°C |
| 4.15 | $C_6H_5$ | $CH_3$ | cyclo-$C_3H_5$ | 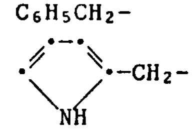 | H | |
| 4.16 | $4-F-C_6H_4$ | $CH_3$ | cyclo-$C_3H_5$ | 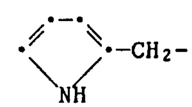 | H | Smp. 83-84°C |
| 4.17 | $C_6H_5$ | $CH_2OCH_3$ | $CH_3$ | H | $CH_3$ | $n_D^{24}$ 1,5793 |
| 4.18 | $C_6H_5$ | $CH_2OCH_3$ | $CH_3$ | $CH_3CH=CHCH_2-$ | H | |
| 4.19 | $C_6H_5$ | $CH_2OCH_3$ | $CH_3$ | $CF_3CH_2$ | H | |
| 4.20 | $C_6H_5$ | $CH_2OCH_3$ | $CH_3$ | n-$C_3H_7$ | H | $n_D^{25}$ 1,5635 |

Tabelle 4: (Fortsetzung)

| Verb. | $R_1$ | $R_2$ | $R_3$ | $R_{10}$ | $R_{11}$ | physikal. Konstante |
|---|---|---|---|---|---|---|
| 4.21 | $C_6H_5$ | $CH_2OCH_3$ | $CH_3$ | 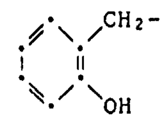 | H | |
| 4.22 | 4-F-$C_6H_4$ | $CH_3$ | cyclo-$C_3H_5$ | $C_2H_5$ | H | $n_D^{23}$ 1,5693 |
| 4.23 | 3-Br-$C_6H_4$ | $CH_3$ | cyclo-$C_3H_5$ | n-$C_4H_9$ | $CH_3$ | |
| 4.24 | 2-Cl-4-$CF_3$-$C_6H_3$ | $CH_3$ | $CH_3$ | $CH_3$ | $C_2H_5$ | |
| 4.25 | $C_6H_5$ | $C_2H_5$ | $CH_3$ | H | $CH_3$ | |
| 4.26 | $C_6H_5$ | n-$C_3H_5$ | $CH_3$ | H | $CH_3$ | |
| 4.27 | $C_6H_5$ | $CH(CH_3)_2$ | $CH(CH_3)_2$ | H | $CH_3$ | |
| 4.28 | $C_6H_5$ | $CH_3$ | $C(CH_3)_3$ | H | $CH_3$ | |
| 4.29 | $C_6H_5$ | $C_2H_5$ | $C_2H_5$ | H | $CH_3$ | |
| 4.31 | 4-F-$C_6H_4$ | $CH_3$ | $CH_3$ | $C_2H_5$ | $C_2H_5$ | $n_D^{25}$ 1,5533 |
| 4.32 | 4-F-$C_6H_4$ | $CH_3$ | $CH_3$ | $CH_3$ | H | Smp. 57-59°C |
| 4.33 | $C_6H_5$ | $CH_3$ | $CH_3$ | $CH_2CCl_3$ | H | |
| 4.34 | $C_6H_5$ | $CH_3$ | $CH_3$ | 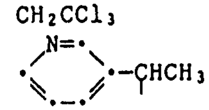 | H | |
| 4.35 | $C_6H_5$ | $CH_3$ | $CH_3$ | $(CH_3)_2CH$ | H | |
| 4.36 | $C_6H_5$ | cyclo-$C_3H_5$ | $CH_3$ | $(CH_3)_2CH$ | H | $n_D^{24}$ 1,5772 |
| 4.37 | $C_6H_5$ | cyclo-$C_3H_5$ | $CH_3$ | $C_2H_5(CH_3)CH$- | H | |
| 4.38 | $C_6H_5$ | $CH_3$ | $CH_3$ | $Br_3CCH_2$- | H | |
| 4.39 | $C_6H_5$ | $CH_3$ | $CH_3$ | $CH_2CH_2CN$ | H | |
| 4.40 | $C_6H_5$ | $CH_3$ | $CH_3$ | $CH_2CH_2CN$ | $CH_2CH_2CN$ | |
| 4.41 | $C_6H_5$ | $CH_3$ | cyclo-$C_3H_5$ | $CH_2CH_2CN$ | $CH_2CH_2CN$ | |

Tabelle 4: (Fortsetzung)

| Verb. | $R_1$ | $R_2$ | $R_3$ | $R_{10}$ | $R_{11}$ | physikal. Konstante |
|---|---|---|---|---|---|---|
| 4.42 | $C_6H_5$ | $CH_3$ | cyclo-$C_3H_5$ | $CH_2CH_2CN$ | H | |
| 4.43 | $C_6H_5$ | $CH_3$ | cyclo-$C_3H_5$ | cyclo-$C_6H_{11}$ | H | Smp. 75-77°C |
| 4.44 | $C_6H_5$ | $CH_3$ | cyclo-$C_3H_5$ | $C_2H_5$ | H | $n_D^{25}$ 1,5830 |
| 4.45 | $C_6H_5$ | $CH_3$ | cyclo-$C_3H_5$ | $CH_3$ | H | $n_D^{29}$ 1,5983 |
| 4.46 | 3-F-$C_6H_4$ | $CH_3$ | cyclo-$C_3H_5$ | $CH_3$ | H | $n_D^{36}$ 1,5842 |
| 4.47 | 3-F-$C_6H_4$ | $CH_3$ | cyclo-$C_3H_5$ | $C_2H_5$ | H | $n_D^{23}$ 1,5778 |
| 4.48 | 4-F-$C_6H_4$ | $CH_3$ | cyclo-$C_3H_5$ | $C_2H_5(CH_3)CH$ | H | |
| 4.52 | $C_6H_5$ | $CH_2OCH_3$ | $CH_3$ | $CH_3OCH_2(CH_3)CH-$ | H | $n_D^{24}$ 1,5565 |
| 4.53 | $C_6H_5$ | $CH_2OCH_3$ | $CH_3$ | $(CH_3)_2NCH_2(CH_3)CH-$ | H | |
| 4.54 | 3-F-$C_6H_4$ | $CH_2OCH_3$ | $CH_3$ | H | $(CH_3)CHCH_2$ | |
| 4.55 | 3-F-$C_6H_4$ | $CH_2OCH_3$ | $CH_3$ | H | n-$C_3H_7$ | |
| 4.56 | 3-F-$C_6H_4$ | $CH_2OCH_3$ | $CH_3$ | $(CH_3)_2CH$ | H | $n_D^{23}$ 1,5450 |
| 4.57 | 4-J-$C_6H_4$ | $CH_3$ | $CH_3$ | 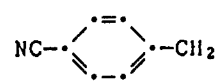 | H | |
| 4.59 | $C_6H_5$ | $CH_3$ | cyclo-$C_3H_5$ | 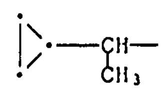 | H | |

Tabelle 4: (Fortsetzung)

| Verb. | $R_1$ | $R_2$ | $R_3$ | $R_{10}$ | $R_{11}$ | physikal. Konstante |
|---|---|---|---|---|---|---|
| 4.60 | $C_6H_5$ | $CH_3$ | cyclo-$C_3H_5$ | H | n-$C_3H_7$ | |
| 4.62 | $C_6H_5$ | $CH_3$ | cyclo-$C_3H_5$ | $CF_3CH_2$ | H | |
| 4.63 | 2-F-$C_6H_4$ | $CH_3$ | cyclo-$C_3H_5$ | $Br_3CCH_2$ | H | |
| 4.64 | 2-F-$C_6H_4$ | $C_2H_5$ | $CH_3$ | $Cl_2CHCH_2$ | H | |
| 4.65 | 4-F-$C_6H_4$ | $CH_3$ | cyclo-$C_3H_5$ | 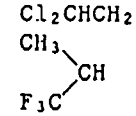 | H | |
| 4.67 | 4-F-$C_6H_4$ | $CH_3$ | cyclo-$C_3H_5$ | H | n-$C_3H_7$ | |
| 4.68 | 4-F-$C_6H_4$ | $CH_3$ | cyclo-$C_3H_5$ | H | $(CH_3)_2CHCH_2$ | |
| 4.69 | $C_6H_5$ | $CH_3$ | $CH_2OCH_3$ | $CH_3$ | $CH_3$ | $n_D^{24}$ 1,5595 |
| 4.70 | $C_6H_5$ | $CH_3$ | $CH_2OCH_3$ | n-$C_3H_7$ | n-$C_3H_7$ | $n_D^{23}$ 1,5483 |
| 4.71 | $C_6H_5$ | $CH_3$ | $CH_2OCH_3$ | H | $CH_3(CH_2)_7$ | |
| 4.72 | $C_6H_5$ | $CH_3$ | $CH_2OCH_3$ | $BrCH_2CHO$ | H | |
| 4.73 | $C_6H_5$ | $CH_3$ | $CH_2OCH_3$ | 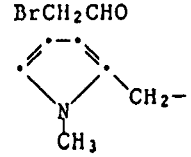 | H | Smp. 67-68°C |
| 4.74 | $C_6H_5$ | $CH_3$ | $CH_3$ | H | $C_2H_5(CH_3)CH-$ | $n_D^{23}$ 1,5638 |
| 4.75 | $C_6H_5$ | $CH_3$ | $CH_3$ | $C_6H_5(CF_3)CH-$ | H | |

Tabelle 4: (Fortsetzung)

| Verb. | $R_1$ | $R_2$ | $R_3$ | $R_{10}$ | $R_{11}$ | physikal. Konstante |
|---|---|---|---|---|---|---|
| 4.77 | $C_6H_5$ | $CH_3$ | $CH_3$ | $(CH_3)_2CHCH_2$ | H | $n_D^{24}$ 1,5623 |
| 4.79 | $C_6H_5$ | $CH_3$ | $CH_3$ | 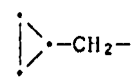 | H | |
| 4.80 | $C_6H_5$ | $CH_3$ | $CH_3$ | $CF_3CH_2$- | H | |
| 4.81 | $C_6H_5$ | $CH_3$ | cyclo-$C_3H_5$ | $CH_3$ | $C_2H_5$ | $n_D^{24}$ 1,5803 |
| 4.83 | $C_6H_5$ | $CH_3$ | cyclo-$C_3H_5$ | 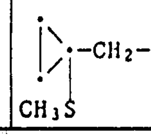 | H | |

<u>Tabelle 4:</u> (Fortsetzung)

| Verb. | $R_1$ | $R_2$ | $R_3$ | $R_{10}$ | $R_{11}$ | physikal. Konstante |
|---|---|---|---|---|---|---|
| 4.86 | $C_6H_5$ | $CH_3$ | cyclo-$C_3H_5$ | 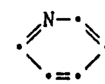 $-CH_2-$ | H | |
| 4.87 | $C_6H_5$ | $CH_3$ | cyclo-$C_3H_5$ | H | $(CH_3)_2CHCH_2-$ | $n_D^{25}$ 1,5733 |
| 4.88 | $C_6H_5$ | $CH_3$ | cyclo-$C_3H_5$ | H | $CH_3CH{=}CHCH_2-$ | |
| 4.89 | $C_6H_5$ | $CH_3$ | cyclo-$C_3H_5$ | $C_6H_5CH_2$ | H | |
| 4.90 | 4-F-$C_6H_4$ | $CH_3$ | cyclo-$C_3H_5$ | n-$C_3H_7$ | n-$C_3H_7$ | $n_D^{23}$ 1,5483 |
| 4.91 | 3-F-$C_6H_4$ | $CH_3OCH_2$ | $CH_3$ | H | $CH_3$ | |
| 4.92 | 3-F-$C_6H_4$ | $CH_3OCH_2$ | $CH_3$ | H | $C_2H_5$ | |
| 4.93 | 3-F-$C_6H_4$ | $CH_3OCH_2$ | $CH_3$ | H | n-$C_4H_9$ | |
| 4.94 | 4-$CF_3O$-$C_6H_4$ | $CH_3OCH_2$ | $CH_3$ | H | $C_2H_5$ | |
| 4.95 | 4-$CF_3O$-$C_6H_4$ | $CH_3OCH_2$ | $CH_3$ | $CH_3$ | $CH_3$ | |
| 4.96 | 4-J-$C_6H_4$ | $CH_3$ | cyclo-$C_3H_5$ | H | n-$C_3H_7$ | |
| 4.97 | 4-J-$C_6H_4$ | $CH_3$ | cyclo-$C_3H_5$ | H | $(CH_3)_2CHCH_2$ | |
| 4.98 | $C_6H_5$ | $CH_3$ | cyclo-$C_3H_5$ | $CH_3$ | $CH_3$ | $n_D^{31}$ 1,5813 |
| 4.99 | $C_6H_5$ | $CH_3$ | cyclo-$C_3H_5$ | $C_2H_5$ | $C_2H_5$ | $n_D^{24}$ 1,5762 |
| 4.100 | $C_6H_5$ | $CH_3$ | cyclo-$C_3H_5$ | H | $CH_2{=}CHCH_2-$ | |
| 4.101 | $C_6H_5$ | $CH_3$ | cyclo-$C_3H_5$ | $CH_3SCH_2CH_2CH_2$ | H | |
| 4.102 | $C_6H_5$ | $CH_3$ | cyclo-$C_3H_5$ | $CH_3$ | $CH_2CH(CH_3)_2$ | $n_D^{30}$ 1,5613 |

Tabelle 4: (Fortsetzung)

| Verb. | $R_1$ | $R_2$ | $R_3$ | $R_{10}$ | $R_{11}$ | physikal. Konstante |
|---|---|---|---|---|---|---|
| 4.103 | $C_6H_5$ | $CH_3$ | cyclo-$C_3H_5$ | 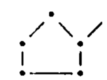 | H | |
| 4.104 | $C_6H_5$ | $C_2H_5$ | $CH_2OCH_3$ | H | $CH_3$ | $n_D^{24}$ 1,5753 |
| 4.105 | $C_6H_5$ | $CH_3$ | $CH_3$ | $CH_2=C(CH_3)-CH_2-$ | H | |
| 4.106 | $C_6H_5$ | cyclo-$C_3H_5$ | $CH_3$ | $CH_3OCH_2(CH_3)CH-$ | $CH_3$ | |
| 4.107 | $C_6H_5$ | cyclo-$C_3H_5$ | $CH_3$ | $CF_3(CH_3)CH-$ | $CH_3$ | |
| 4.108 | $C_6H_5$ | cyclo-$C_3H_5$ | $CH_3$ | $(CF_3)_2CH-$ | H | |
| 4.109 | 3-F-$C_6H_4$ | n-$C_3H_7$ | n-$C_3H_7$ | H | n-$C_3H_7$ | |
| 4.110 | 3-F-$C_6H_4$ | $CH_3$ | 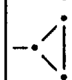 | $CH_3$ | $CH_3$ | $n_D^{23}$ 1,5765 |
| 4.111 | $C_6H_5$ | $CH_3$ | 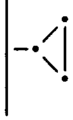 | $CH_2C(CH_3)_3$ | H | Smp. 69-70°C |

2. Formulierungsbeispiele für flüssige Wirkstoffe der Formel I % = Gewichtsprozent)

| 2.1. Emulsionskonzentrate | | | |
|---|---|---|---|
| | a) | b) | c) |
| Wirkstoff aus der Tabelle 1 | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenoyl-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2. Lösungen | | | | |
|---|---|---|---|---|
| | a) | b) | c) | d) |
| Wirkstoff aus der Tabelle 1 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) (MG = Molekulargewicht) | - | - | 94 % | - |

Die Lösungen sind zur Anwendung in Form kleinster Tropfen geeignet.

| 2.3. Granulate | | |
|---|---|---|
| | a) | b) |
| Wirkstoff aus der Tabelle 1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4. Stäubemittel | | |
|---|---|---|
| | a) | b) |
| Wirkstoff aus der Tabelle 1 | 2 % | 5 % |
| Hochdisperse Kieselsäure | 1 % | 5 % |
| Talkum | 97 % | - |
| Kaolin | - | 90 % |

Durch inniges Vermischen auf Trägerstoffe mit dem Wirkstoff erhält man gebrauchsfertige Stäubemittel.

Formulierungsbeispiele für feste Wirkstoffe der Formel I (% = Gewichtsprozent)

| 2.5. Spritzpulver | | | |
|---|---|---|---|
| | a) | b) | c) |
| Wirkstoff aus der Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.6. Emulsions-Konzentrat | |
|---|---|
| Wirkstoff aus der Tabelle 1 | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 34 % |
| Xylolgemisch | 50 % |

Aus diesem Konzentrat können durch Verdünnen mit Wasser Emulsionen jeder gewünschten Konzentration hergestellt werden.

| 2.7. Stäubemittel | | |
|---|---|---|
| | a) | b) |
| Wirkstoff aus der Tabelle 1 | 5 % | 8 % |
| Talkum | 95 % | - |
| Kaolin | - | 92 % |

Man erhält anwendungsfertige Stäubemittel, indem der Wirkstoff mit dem Träger vermischt auf einer geeigneten Mühle vermahlen wird.

| 2.8. Extruder Granulat | |
|---|---|
| Wirkstoff aus der Tabelle 1 | 10 % |
| N-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.9. Umhüllungs-Granulat | |
|---|---|
| Wirkstoff aus der Tabelle 1 | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 2.10. Suspensions-Konzentrat | |
|---|---|
| Wirkstoff aus der Tabelle 1 | 40 % |
| Ethylenglykol | 10 % |
| Nonylphenolpolyethylenglykolether (15 Mol Ethylenoxid) | 6 % |
| N-Ligninsulfonat | 10 % |
| Carboxymethylcellulose | 1 % |
| 37%ige wässrige Formaldehyd-Lösung | 0,2% |
| Silkonöl in Form einer 75%igen wässrigen Emulsion | 0,8% |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

3. Biologische Beispiele

Beispiel 3.1: Wirkung gegen Venturia inaequalis auf Apfeltrieben

Residual-protektive Wirkung

Apfelstecklinge mit 10-20 cm langen Frischtrieben werden mit einer aus Spritzpulver des Wirkstoffes hergestellten Spritzbrühe (0,006 % Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24°C aufgestellt. Der Schorfbefall wird 15 Tage nach der Infektion beurteilt.

Verbindungen aus den Tabellen zeigen gegen Venturia gute Wirksamkeit (Befall: weniger als 20 %). So reduzieren z.B. die Verbindungen Nr. 1.79, 1.108, 1.122 und 4.45 den Venturia-Befall auf 0 bis 10 %. Unbehandelte aber infizierte Kontrollpflanzen weisen dagegen einen Venturia-Befall von 100 % auf.

Beispiel 3.2: Wirkung gegen Botrytis cinerea an Apfelfrüchten

Residual-protektive Wirkung

Künstlich verletzte Aepfel werden behandelt, indem eine aus Spritzpulver oder Emulsionskonzentrat der Wirksubstanz hergestellte Spritzbrühe (0,002 % oder 0,02 % Aktivsubstanz) auf die Verletzungsstellen aufgetropft wird. Die behandelten Früchte werden anschliessend mit einer Sporensuspension des Pilzes inokuliert und während einer Woche bei hoher Luftfeuchtigkeit und ca. 20°C inkubiert. Bei der Auswertung werden die angefaulten Verletzungsstellen gezählt und daraus die fungizide Wirkung der Testsubstanz abgeleitet.

Verbindungen aus den Tabellen zeigen gegen Botrytis gute Wirksamkeit (Befall: weniger als 20 %). So reduzieren z.B. die Verbindungen Nr. 1.4, 1.6, 1.7, 1.12, 1.17, 1.48, 1.55, 1.56, 1.65, 1.68, 1.79, 1.104, 1.108, 1.118, 1.119, 1.122, 1.124, 1.129, 1.137, 1.138, 3.5, 3.6, 3.7, 3.8, 3.10. 3.11, 3.25, 3.26, 3.35, 3.40, 3.50, 3.52, 3.78, 3.79, 4.1, 4.4, 4.15, 4.17, 4.20, 4.45, 4.56, 4.73, 4.74, 4.87 und 4.98 den Botrytis-Befall auf 0 bis 10%. Unbehandelte aber infizierte Kontrollpflanzen weisen dagegen einen Botrytis-Befall von 100 % auf.

Beispiel 3.3: Wirkung gegen Erysiphae graminis auf Gerste

a) Residual-protektive Wirkung

Ca. 8 cm hohe Gerstenpflanzen werden mit einer aus Spritzpulver des Wirkstoffes hergestellten Spritzbrühe (0,02 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

Verbindungen aus den Tabellen zeigen gegen Erysiphae gute Wirksamkeit (Befall: weniger als 20 %). So reduzieren z.B. die Verbindungen Nr. 1.32, 1.56, 1.104, 1.122, 1.124, 3.5, 3.8 und 3.52 den Erysiphae-Befall auf 0 bis 10 %. Unbehandelte aber infizierte Kontrollpflanzen weisen dagegen einen Erysiphae-Befall von 100 % auf.

Beispiel 3.4: Wirkung gegen Helminthosporium gramineum

Weizenkörner werden mit einer Sporensuspension des Pilzes kontaminiert und wieder angetrocknet. Die kontaminierten Körner werden mit einer aus Spritzpulver hergestellten Suspension der Testsubstanz gebeizt (600 ppm Wirkstoff bezogen auf das Gewicht der Samen). Nach zwei Tagen werden die Körner auf geeignete Agarschalen ausgelegt und nach weiteren vier Tagen wird die Entwicklung der Pilzkolonien um die Körner herum beurteilt. Anzahl und Grösse der Pilzkolonien werden zur Beurteilung der Testsubstanz herangezogen. Verbindungen aus den Tabellen verhindern den Pilzbefall weitgehend (weniger als 20 % Pilzbefall).

Beispiel 3.5 Wirkung gegen Colletotrichum lagenarium auf Gurken

Gurkenpflanzen werden nach 2-wöchiger Anzucht mit einer aus Spritzpulver des Wirkstoffs hergestellten Spritzbrühe (Konzentration 0,002 %) besprüht. Nach 2 Tagen werden die Pflanzen mit einer Sporensuspension (1,5 x $10^5$ Sporen/ml) des Pilzes infiziert und für 36 Stunden bei 23°C und hoher Luftfeuchtigkeit inkubiert. Die Inkubation wird dann bei normaler Luftfeuchtigkeit und ca. 22-23°C weitergeführt. Der eingetretene Pilzbefall wird 8 Tage nach der Infektion beurteilt. Unbehandelte aber infizierte Kontrollpflanzen weisen einen Pilzbefall von 100 % auf.

Verbindungen aus den Tabellen zeigen gute Wirksamkeit und verhindern die Ausbreitung des Krankheitsbefalls. Der Pilzbefall wird auf 20 % oder weniger zurückgedrängt.

Beispiel 3.6: Wirkung gegen Puccinia graminis auf Weizen

Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

Verbindungen aus den Tabellen zeigen gegen Puccinia gute Wirksamkeit (Befall: weniger als 20 %). So reduziert z.B. Verbindung Nr. 3.10 den Puccinia-Befall auf 0 bis 10 %. Unbehandelte aber infizierte Kontrollpflanzen weisen dagegen einen Puccinia-Befall von 100 % auf.

Beispiel 3.7: Wirkung gegen Phytophthora auf Tomatenpflanzen

a) Residual-protektive Wirkung

Tomatenpflanzen werden nach dreiwöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.

b) Systemische Wirkung

Zu Tomatenpflanzen wird nach dreiwöchiger Anzucht eine aus Spritzpulver des Wirkstoffes hergestellte Spritzbrühe (0,002 % Aktivsubstanz bezogen auf das Erdvolumen) gegossen. Es wird dabei darauf geachtet, dass die Spritzbrühe nicht mit den oberirdischen Pflanzenteilen in Berührung kommt. Nach 48 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.

Verbindungen aus den Tabellen zeigen gegen Phytophthora gute Wirksamkeit (Befall: weniger als 20 %). So reduzieren z.B. die Verbindungen 1.104, 1.122, 4.31 und 4.98 den Phytophthora-Befall auf 0 bis 10 %. Unbehandelte aber infizierte Kontrollpflanzen weisen dagegen einen Phytophthora-Befall von 100 % auf.

## Patentansprüche

**Patentansprüche für folgende Vertragsstaaten : AT, BE, CH, LI, DE, FR, GB, GR, IT, LU, NL, SE**

1. Verbindungen der Formel I

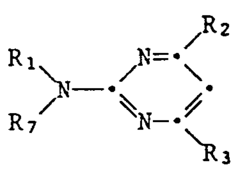

(I)

in welcher bedeuten:
$R_1$ Phenyl oder bis zu 3-fach durch $R_4$ substituiertes Phenyl;
$R_2$ Wasserstoff, $C_1$-$C_5$-Alkyl, mit den Resten $OR_5$ oder $SR_5$ substituiertes $C_1$-$C_5$-Alkyl, $C_3$-$C_6$-Cycloalkyl, mit $C_1$-$C_4$-Alkyl oder Halogen bis zu 3-fach substituiertes $C_3$-$C_6$-Cycloalkyl, $C_2$-$C_5$-Alkenyl, $C_2$-$C_5$-Alkinyl oder den Formylrest;
$R_3$ Wasserstoff, $C_1$-$C_4$-Alkyl, mit Halogen, Cyano, oder den Resten $OR_5$ oder $SR_5$ substituiertes $C_1$-$C_4$-Alkyl, $C_3$-$C_6$-Cycloalkyl oder mit $C_1$-$C_4$-Alkyl oder Halogen bis zu 3-fach substituiertes $C_3$-$C_6$-Cycloalkyl;
$R_4$ Halogen, $C_1$-$C_3$-Alkyl, $C_1$-$C_2$-Halogenalkyl, $C_1$-$C_3$-Alkoxy oder $C_1$-$C_3$-Halogenalkoxy;
$R_5$ Wasserstoff, $C_1$-$C_5$-Alkyl, $C_3$-$C_5$-Alkenyl, $C_3$-$C_5$-Alkinyl oder den Rest $(CH_2)_n$-X-$C_1$-$C_3$-Alkyl; $R_7$ die Gruppe -$NH_2$,

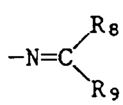 oder 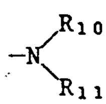 ;

$R_8$ Wasserstoff, $C_1$-$C_3$-Alkyl oder $C_1$-$C_3$-Halogenalkyl;
$R_9$ Wasserstoff, $C_1$-$C_8$-Alkyl, durch Hydroxy, $OR_{12}$, $SR_{12}$ oder $N(R_{12})_2$ substituiertes $C_1$-$C_3$-Alkyl, $C_3$-$C_6$-Cycloalkyl, durch $SR_{12}$ substituiertes Cyclopropyl, $C_3$-$C_{10}$-Alkenyl, $C_1$-$C_3$-Halogenalkyl, 1-, 2- oder 3-Pyridyl,

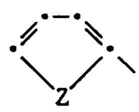 oder 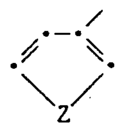 ;

$R_8$ und $R_9$ zusammen mit dem Kohlenstoffatom im Rest $R_7$ einen gesättigten oder ungesättigten Ring, bestehend aus 4 bis 7 Kohlenstoffatomen;
$R_{10}$ $CH(R_8)R_9$, Phenyl, $C_3$-$C_5$-Alkenyl, $C_3$-$C_5$-Alkinyl oder Cyanoalkyl mit 2 oder 3 Kohlenstoffatomen im Alkylrest;
$R_{11}$ Wasserstoff, $C_1$-$C_5$-Alkyl, $C_3$-$C_5$-Alkenyl, $C_3$-$C_5$-Alkinyl oder Cyanoalkyl mit 2 oder 3 Kohlenstoffatomen im Alkylrest;
$R_{12}$ $CH_3$ oder $C_2H_5$;
X Sauerstoff oder Schwefel;
Z O, S, NH oder $NCH_3$; und
n 1 bis 3;
unter Einschluss ihrer Säureadditionssalze und Metallsalzkomplexe.

**2.** Verbindungen der Formel I gemäss Anspruch 1, in welcher bedeuten:
$R_1$ Phenyl oder bis zu 3-fach durch $R_4$ substituiertes Phenyl;
$R_2$ Wasserstoff, $C_1$-$C_5$-Alkyl, mit den Resten $OR_5$ oder $SR_5$ substituiertes $C_1$-$C_5$-Alkyl, $C_3$-$C_6$-Cycloalkyl mit $C_1$-$C_4$-Alkyl oder Halogen bis zu 3-fach substituiertes $C_3$-$C_6$-Cycloalkyl, $C_2$-$C_5$-Alkenyl, $C_2$-$C_5$-Alkinyl oder den Formylrest;
$R_3$ $C_1$-$C_4$-Alkyl, mit Halogen, Cyano, oder den Resten $OR_5$ oder $SR_5$ substituiertes $C_1$-$C_4$-Alkyl, $C_3$-$C_6$-Cycloalkyl oder mit $C_1$-$C_4$-Alkyl oder Halogen bis zu 3-fach substituiertes $C_3$-$C_6$-Cycloalkyl;
$R_4$ Halogen, $C_1$-$C_3$-Alkyl;
$R_5$ Wasserstoff, $C_1$-$C_5$-Alkyl, $C_3$-$C_5$-Alkenyl, $C_3$-$C_5$-Alkinyl oder den Rest $(CH_2)_n$-X-$C_1$-$C_3$-Alkyl;
$R_9$ Wasserstoff, $C_1$-$C_5$-Alkyl, $C_3$-$C_6$-Cycloalkyl, $C_3$-$C_5$-Alkenyl, $C_1$-$C_3$-Halogenalkyl;
$R_8$ und $R_9$ zusammen mit dem Kohlenstoffatom in Rest $R_7$ einen gesättigten oder ungesättigten Ring bestehend aus 5 oder 6 Kohlenstoffatomen;
$R_7$, $R_{10}$, $R_{11}$, $R_{12}$ und Z besitzen die unter Formel I angegebenen Bedeutungen;
X Sauerstoff oder Schwefel;
n 1 bis 3.

**3.** Verbindungen der Formel I gemäss Anspruch 1, in welcher bedeuten:
$R_1$ Phenyl oder einfach durch Halogen substituiertes Phenyl;
$R_2$ Wasserstoff, $C_1$-$C_5$-Alkyl, mit $OR_5$ substituiertes $C_1$-$C_5$-Alkyl, $C_3$-$C_6$-Cycloalkyl, mit $C_1$-$C_4$-Alkyl oder Halogen bis zu 3-fach substituiertes $C_3$-$C_6$-Cycloalkyl, $C_2$-$C_5$-Alkenyl, $C_2$-$C_5$-Alkinyl oder den Formylrest;
$R_3$ $C_1$-$C_4$-Alkyl, mit Halogen, Cyano oder $OR_5$ substituiertes $C_1$-$C_4$-Alkyl, $C_3$-$C_6$-Cycloalkyl oder mit Methyl substituiertes $C_3$-$C_6$-Cycloalkyl;
$R_5$ Wasserstoff oder $C_1$-$C_2$-Alkyl;
$R_9$ Wasserstoff, $C_1$-$C_5$-Alkyl, $C_3$-$C_6$-Cycloalkyl, $C_3$-$C_5$-Alkenyl, $C_1$-$C_3$-Halogenalkyl;
$R_8$ und $R_9$ zusammen mit dem Kohlenstoffatom in Rest $R_7$ einen gesättigten oder ungesättigten Ring bestehend aus 5 oder 6 Kohlenstoffatomen;
$R_7$, $R_{10}$, $R_{11}$ und $R_{12}$ besitzen die unter Formel I angegebenen Bedeutungen.

**4.** Verbindungen der Formel I in Anspruch 1, in welcher bedeuten:
$R_1$ Phenyl oder bis zu 3-fach durch $R_4$ substituiertes Phenyl;
$R_2$ Wasserstoff, $C_1$-$C_5$-Alkyl, mit den Resten $OR_5$ oder $SR_5$ substituiertes $C_1$-$C_5$-Alkyl, $C_3$-$C_6$-Cycloalkyl, mit $C_1$-$C_4$-Alkyl oder Halogen bis zu 3-fach substituiertes $C_3$-$C_6$-Cycloalkyl, $C_2$-$C_5$-Alkenyl, $C_2$-$C_5$-Alkinyl oder den Formylrest;
$R_3$ $C_1$-$C_4$-Alkyl, mit Halogen, Cyano, oder den Resten $OR_5$ oder $SR_5$ substituiertes $C_1$-$C_4$-Alkyl, $C_3$-$C_6$-Cycloalkyl oder mit $C_1$-$C_4$-Alkyl oder Halogen bis zu 3-fach substituiertes $C_3$-$C_6$-Cycloalkyl;
$R_4$ Halogen, $C_1$-$C_3$-Alkyl, $C_1$-$C_2$-Halogenalkyl, $C_1$-$C_3$-Alkoxy oder $C_1$-$C_3$-Halogenalkoxy;
$R_5$ Wasserstoff, $C_1$-$C_5$-Alkyl, $C_3$-$C_5$-Alkenyl, $C_3$-$C_5$-Alkinyl oder den Rest $(CH_2)_n$-X-$C_1$-$C_3$-Alkyl;
$R_7$ $-NH_2$;
X Sauerstoff oder Schwefel;
n 1-3; unter Einschluss ihrer Säureadditionssalze und Metallsalzkomplexe.

**5.** Verbindungen der Formel I gemäss Anspruch 4, in welcher bedeuten:
$R_1$ Phenyl oder bis zu 3-fach durch $R_4$ substituiertes Phenyl;
$R_2$ Wasserstoff, $C_1$-$C_5$-Alkyl, mit den Resten $OR_5$ oder $SR_5$ substituiertes $C_1$-$C_5$-Alkyl, $C_3$-$C_6$-Cycloalkyl, mit $C_1$-$C_4$-Alkyl oder Halogen bis zu 3-fach substituiertes $C_3$-$C_6$-Cycloalkyl, $C_2$-$C_5$-Alkenyl, $C_2$-$C_5$-Alkinyl oder den Formylrest;
$R_3$ $C_1$-$C_4$-Alkyl, mit Halogen, Cyano, oder den Resten $OR_5$ oder $SR_5$ substituiertes $C_1$-$C_4$-Alkyl, $C_3$-$C_6$-Cycloalkyl oder mit $C_1$-$C_4$-Alkyl oder Halogen bis zu 3-fach substituiertes $C_3$-$C_6$-Cycloalkyl;
$R_4$ Halogen;
$R_5$ Wasserstoff, $C_1$-$C_5$-Alkyl, $C_3$-$C_5$-Alkenyl, $C_3$-$C_5$-Alkinyl oder den Rest $(CH_2)_n$-X-$C_1$-$C_3$-Alkyl;
X Sauerstoff oder Schwefel;
n 1-3.

**6.** Verbindungen der Formel I gemäss Anspruch 1, in welcher bedeuten:
$R_1$ Phenyl oder bis zu 3-fach durch Halogen substituiertes Phenyl;
$R_2$ Wasserstoff, $C_1$-$C_5$-Alkyl, mit den Resten $OR_5$ oder $SR_5$ substituiertes $C_1$-$C_5$-Alkyl, $C_3$-$C_6$-Cycloalkyl, mit $C_1$-$C_4$-Alkyl oder Halogen bis zu 3-fach substituiertes $C_3$-$C_6$-Cycloalkyl, $C_2$-$C_5$-Alkenyl, $C_2$-$C_5$-Alkinyl oder den Formylrest;
$R_3$ $C_1$-$C_4$-Alkyl, mit Halogen, Cyano, oder den Resten $OR_5$ oder $SR_5$ substituiertes $C_1$-$C_4$-Alkyl, $C_3$-$C_6$-Cycloalkyl oder mit $C_1$-$C_4$-Alkyl oder Halogen bis zu 3-fach substituiertes $C_3$-$C_6$-Cycloalkyl;
$R_5$ Wasserstoff, $C_1$-$C_5$-Alkyl, $C_3$-$C_5$-Alkenyl, $C_3$-$C_5$-Alkinyl oder den Rest $(CH_2)_n$-X-$C_1$-$C_3$-Alkyl;
X Sauerstoff oder Schwefel;
n 1-3.

**7.** Verbindungen der Formel I gemäss Anspruch 1, in welcher bedeuten:
$R_1$ Phenyl oder einfach durch Chlor oder Fluor substituiertes Phenyl;
$R_2$ $C_1$-$C_5$-Alkyl, mit $OR_5$ substituiertes $C_1$-$C_2$-Alkyl, $C_3$-$C_6$-Cycloalkyl mit $C_1$-$C_4$-Alkyl oder Halogen bis zu 3-fach substituiertes $C_3$-$C_6$-Cycloalkyl, $C_2$-$C_5$-Alkenyl, $C_2$-$C_5$-Alkinyl oder den Formylrest;
$R_3$ $C_1$-$C_4$-Alkyl, $C_1$-$C_4$-Halogenalkyl, $C_3$-$C_6$-Cycloalkyl oder mit Methyl substituiertes $C_3$-$C_6$-Cycloalkyl;
$R_5$ Wasserstoff oder $C_1$-$C_2$-Alkyl.

**8.** Verbindungen der Formel I gemäss den Ansprüchen 6 und 7, worin $R_3$ bedeutet: Methyl, Fluormethyl, Chlormethyl, Brommethyl, $C_3$-$C_6$-Cycloalkyl oder Methoxymethyl.

**9.** Eine Verbindung der Formel I gemäss Anspruch 1 aus der Gruppe:
N-(4-Fluormethyl-6-cyclopropylpyrimidyl-2)-N-phenyl-hydrazin;
N-(4-Methyl-6-cyclopropylpyrimidyl-2)-N-m-fluorphenyl-hydrazin;
N-(4-Methyl-6-cyclopropylpyrimidyl-2)-N-p-fluorphenyl-hydrazin;

**10.** Eine Verbindung der Formel I gemäss Anspruch 5 aus der Gruppe:
N-(4-Methyl-6-cyclopropylpyrimidyl-2)-N-phenyl-hydrazin;
N-(4,6-Dimethylpyrimidyl-2)-N-phenyl-hydrazin;
N-(4-Methyl-6-methoxymethylpyrimidyl-2)-N-phenyl-hydrazin;

**11.** Eine Verbindung der Formel I gemäss Anspruch 1 aus der Gruppe:
N-(4,6-Dimethylpyrimidyl-2)-N-phenyl-propionaldehydhydrazon;
N-(4,6-Dimethylpyrimidyl-2)-N-phenyl-isobutyraldehydhydrazon;
N-(4-Methyl-6-methoxymethylpyrimidyl-2)-N-phenyl-isobutyraldehydhydrazon;
N-(4-Methyl-6-methoxymethylpyrimidyl-2)-N-phenyl-propionaldehydhydrazon;
N-(4-Methyl-6-cyclopropylpyrimidyl-2)-N-phenyl-propionaldehydhydrazon;
N-(4-Methyl-6-cyclopropylpyrimidyl-2)-N-phenyl-n-butyraldehydhydrazon;
N-(4-Methyl-6-cyclopropylpyrimidyl-2)-N-phenyl-isobutyraldehydhydrazon;
N-(4-Methyl-6-cyclopropylpyrimidyl-2)-N-phenyl-trichloracetaldehydhydrazon;
N-(4-Methyl-6-cyclopropylpyrimidyl-2)-N-p-fluorphenyl-acetaldehydhydrazon;
N-(4-Methyl-6-cyclopropylpyrimidyl-2)-N-p-fluorphenyl-isobutyraldehydhydrazon;
N-(4-Methyl-6-cyclopropylpyrimidyl-2)-N-m-fluorphenyl-isobutyraldehydhydrazon;
N-(4,6-Dimethylpyrimidyl-2)-N-phenyl-N′-methylhydrazin;
N-(4,6-Dimethylpyrimidyl-2)-N-phenyl-N′-dimethylhydrazin;
N-(4,6-Dimethylpyrimidyl-2)-N-phenyl-N′-n-propylhydrazin;
N-(4,6-Dimethylpyrimidyl-2)-N-phenyl-N′-isobutylhydrazin;

N-(4-Methyl-6-methoxymethylpyrimidyl-2)-N-phenyl-N′-methylhydrazin;
N-(4-Methyl-6-methoxymethylpyrimidyl-2)-N-phenyl-N′-n-propylhydrazin;
N-(4-Methyl-6-methoxymethylpyrimidyl-2)-N-phenyl-N′-dimethylhydrazin;
N-(4-Methyl-6-cyclopropylpyrimidyl-2)-N-phenyl-N′-methylhydrazin;
N-(4-Methyl-6-cyclopropylpyrimidyl-2)-N-phenyl-N′-isobutylhydrazin;
N-(4-Methyl-6-cyclopropylpyrimidyl-2)-N-phenyl-N′-dimethylhydrazin;
N-(4-Methyl-6-cyclopropylpyrimidyl-2)-N-phenyl-N′-diäthylhydrazin;
N-(4-Methyl-6-cyclopropylpyrimidyl-2)-N-phenyl-N′-methyl-N′-äthylhydrazin;
N-(4-Methyl-6-cyclopropylpyrimidyl-2)-N-p-fluorphenyl-N′-äthylhydrazin;
N-(4-Methyl-6-methoxymethylpyrimidyl-2)-N-m-fluorphenyl-N′-isopropylhydrazin;

**12.** Verfahren zur Herstellung einer Verbindung der Formel I gemäss Anspruch 1, dadurch gekennzeichnet, dass man

a) ein Pyrimidinderivat der Formel II

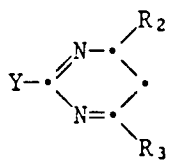

(II)

mit einem Phenylhydrazinderivat der Formel III

$$R_1\text{-NH-NH-R} \qquad (III)$$

in Gegenwart einer Base in aprotischen Lösungsmitteln bei Temperaturen von -50° bis 150°C umsetzt, wobei Y Halogen, den Rest $SO_2R_6$ oder $N^{\oplus}(CH_3)_3$ darstellt, $R_6$ $C_1$-$C_4$-Alkyl, Phenyl oder mit Methyl oder Chlor substituiertes Phenyl bedeutet und R die Bedeutungen von $R_{10}$ und $R_{11}$ hat und diese sowie $R_1$-$R_3$ den unter der Formel I angegebenen Definitionen entsprechen,
oder
b) ein Pyrimidinhydrazinderivat der Formel IV mit einem Aldehyd oder Keton der Formel V zu Verbindungen der Formel VII unter Abspaltung von Wasser

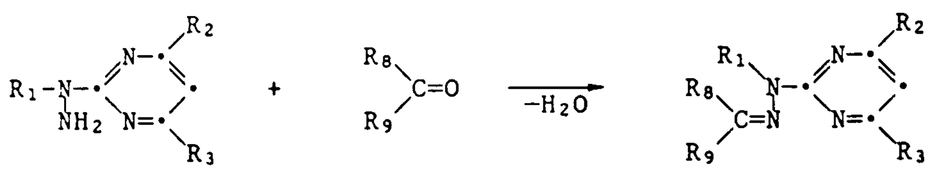


(IV) (V) (VII)

in einem beliebigen Lösungsmittel in Gegenwart einer Säure bei Temperaturen von -20° bis 120° umsetzt, wobei $R_1$-$R_3$ und $R_8$ und $R_9$ die unter Formel I angegebenen Bedeutungen besitzen,
oder

c) ein Hydrazonderivat der Formel VII

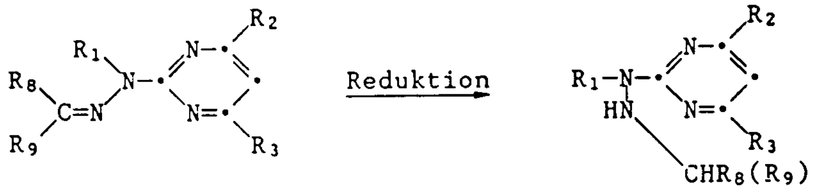


(VII) (VIII)

mittels eines Reduktionsmittels in einem inerten Lösungsmittel bei Temperaturen von 0° bis 50°C oder durch katalytische Hydrierung an Katalysatoren reduziert
oder
d) ein Pyrimidinhydrazinderivat der Formel IV

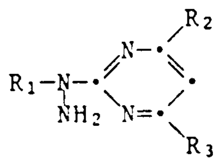

(IV)

mit einem Aldehyd oder Keton der Formel V

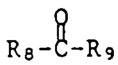

(V)

in Gegenwart eines Reduktionsmittels in einem inerten Lösungsmittel bei Temperaturen von 0° bis 50°C reduktiv alkyliert
oder
e) ein Pyrimidinhydrazin der Formel IV oder VIII mit einem Alkylhalogenid $R_0Hal$

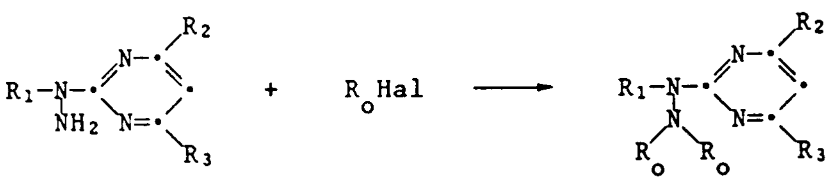


(IV) (IX)

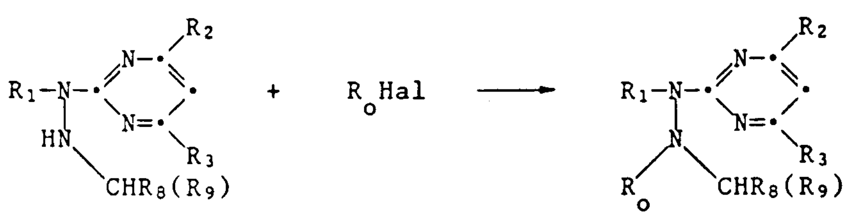


(VIII) (X)

in einem inerten Lösungsmittel in Gegenwart einer Base bei Temperaturen von 0°-60°C alkyliert, wobei $R_o$ $C_1$-$C_4$-Alkyl darstellt und $R_1$-$R_9$ in den vorstehend beschriebenen Verfahren (a-e) die unter Formel I angegebenen Bedeutungen hat.

13. Mittel zur Bekämpfung oder Verhütung eines Befalls durch schädliche Insekten oder Mikroorganismen, dadurch gekennzeichnet, dass es als aktive Komponente mindestens eine Verbindung der Formel I gemäss Anspruch 1 zusammen mit einem geeigneten Trägermaterial enthält.

14. Mittel gemäss Anspruch 13, dadurch gekennzeichnet, dass es als aktive Komponente mindestens eine Verbindung der Formel I gemäss Anspruch 4 enthält.

15. Mittel gemäss Anspruch 13, dadurch gekennzeichnet, dass es als aktive Komponente mindestens eine Verbindung der Formel I gemäss Anspruch 9 enthält.

16. Mittel gemäss Anspruch 13, dadurch gekennzeichnet, dass es als aktive Komponente mindestens eine Verbindung der Formel I gemäss Anspruch 10 enthält.

17. Mittel gemäss Anspruch 13, dadurch gekennzeichnet, dass es als aktive Komponente mindestens eine Verbindung der Formel I gemäss Anspruch 11 enthält.

18. Verfahren zur Bekämpfung oder Verhütung eines Befalls von Kulturpflanzen durch Schadinsekten oder phytopathogene Mikroorganismen, dadurch gekennzeichnet, dass man als Wirkstoff eine Verbindung der Formel I gemäss Anspruch 1 auf die Pflanze, auf Pflanzenteile oder ihren Standort appliziert.

19. Verfahren gemäss Anspruch 18, dadurch gekennzeichnet, dass man als Wirkstoff eine Verbindung gemäss einem der Ansprüche 2 bis 11 appliziert.

20. Verfahren gemäss Anspruch 18, dadurch gekennzeichnet, dass phytopathogene Pilze bekämpft werden.

21. Verfahren zur Herstellung eines agrochemischen Mittels gemäss Anspruch 13, dadurch gekennzeichnet, dass man mindestens eine Verbindung der Formel I gemäss Anspruch 1 mit geeigneten festen oder flüssigen Zusatzstoffen und/oder Tensiden innig vermischt.

**Patentansprüche für folgenden Vertragsstaat : ES**

1. Verfahren zur Herstellung einer Verbindung der Formel I

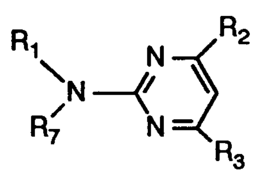

(I)

in welcher bedeuten: $R_1$ Phenyl oder bis zu 3-fach durch $R_4$ substituiertes Phenyl;
$R_2$ Wasserstoff, $C_1$-$C_5$-Alkyl, mit den Resten $OR_5$ oder $SR_5$ substituiertes $C_1$-$C_5$-Alkyl, $C_3$-$C_6$-Cycloalkyl, mit $C_1$-$C_4$-Alkyl oder Halogen bis zu 3-fach substituiertes $C_3$-$C_6$-Cycloalkyl, $C_2$-$C_5$-Alkenyl, $C_2$-$C_5$-Alkinyl oder den Formylrest;
$R_3$ Wasserstoff, $C_1$-$C_4$-Alkyl, mit Halogen, Cyano, oder den Resten $OR_5$ oder $SR_5$ substituiertes $C_1$-$C_4$-Alkyl, $C_3$-$C_6$-Cycloalkyl oder mit $C_1$-$C_4$-Alkyl oder Halogen bis zu 3-fach substituiertes $C_3$-$C_6$-Cycloalkyl;
$R_4$ Halogen, $C_1$-$C_3$-Alkyl, $C_1$-$C_2$-Halogenalkyl, $C_1$-$C_3$-Alkoxy oder $C_1$-$C_3$-Halogenalkoxy;
$R_5$ Wasserstoff, $C_1$-$C_5$-Alkyl, $C_3$-$C_5$-Alkenyl, $C_3$-$C_5$-Alkinyl oder den Rest $(CH_2)_n$-X-$C_1$-$C_3$-Alkyl;
$R_7$ die Gruppe $-NH_2$,

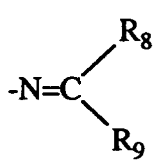

oder

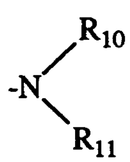

;

$R_8$ Wasserstoff, $C_1$-$C_3$-Alkyl oder $C_1$-$C_3$-Halogenalkyl;

$R_9$ Wasserstoff, $C_1$-$C_8$-Alkyl, durch Hydroxy, $OR_{12}$, $SR_{12}$ oder $N(R_{12})_2$ substituiertes $C_1$-$C_3$-Alkyl, $C_3$-$C_6$-Cycloalkyl, durch $SR_{12}$ substituiertes Cyclopropyl, $C_3$-$C_{10}$-Alkenyl, $C_1$-$C_3$-Halogenalkyl, 1-, 2- oder 3-Pyridyl,

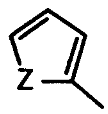 oder 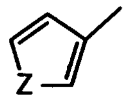 ;

$R_8$ und $R_9$ zusammen mit dem Kohlenstoffatom im Rest $R_7$ einen gesättigten oder ungesättigten Ring, bestehend aus 4 bis 7 Kohlenstoffatomen;
$R_{10}$ $CH(R_8)R_9$, Phenyl, $C_3$-$C_5$-Alkenyl, $C_3$-$C_5$-Alkinyl oder Cyanoalkyl mit 2 oder 3 Kohlenstoffatomen im Alkylrest;
$R_{11}$ Wasserstoff, $C_1$-$C_5$-Alkyl, $C_3$-$C_5$-Alkenyl, $C_3$-$C_5$-Alkinyl oder Cyanoalkyl mit 2 oder 3 Kohlenstoffatomen im Alkylrest;
$R_{12}$ $CH_3$ oder $C_2H_5$;
X Sauerstoff oder Schwefel;
Z O, S, NH oder $NCH_3$; und
n 1 bis 3;
unter Einschluss ihrer Säureadditionssalze und Metallsalzkomplexe, dadurch gekennzeichnet, dass man

a) ein Pyrimidinderivat der Formel II

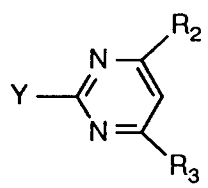 (II)

mit einem Phenylhydrazinderivat der Formel III

$$R_1\text{-NH-NH-R} \qquad \text{(III)}$$

in Gegenwart einer Base in aprotischen Lösungsmitteln bei Temperaturen von -50° bis 150°C umsetzt, wobei Y Halogen, den Rest $SO_2R_6$ oder $N^{\oplus}(CH_3)_3$ darstellt, $R_6$ $C_1$-$C_4$-Alkyl, Phenyl oder mit Methyl oder Chlor substituiertes Phenyl bedeutet und R die Bedeutungen von $R_{10}$ und $R_{11}$ hat und diese sowie $R_1$-$R_3$ den unter der Formel I angegebenen Definitionen entsprechen,
oder

b) ein Pyrimidinhydrazinderivat der Formel IV mit einem Aldehyd oder Keton der Formel V zu Verbindungen der Formel VII unter Abspaltung von Wasser

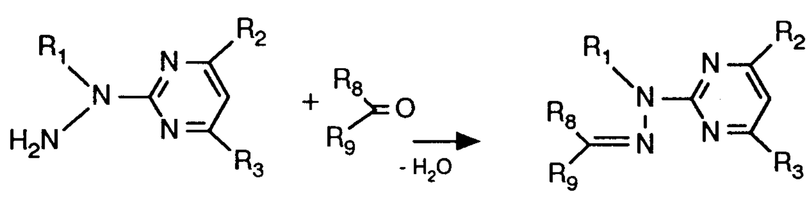


(IV) (V) (VII)

in einem beliebigen Lösungsmittel in Gegenwart einer Säure bei Temperaturen von -20° bis 120°C umsetzt, wobei $R_1$-$R_3$ und $R_8$ und $R_9$ die unter Formel I angegebenen Bedeutungen besitzen,
oder

c) ein Hydrazonderivat der Formel VII

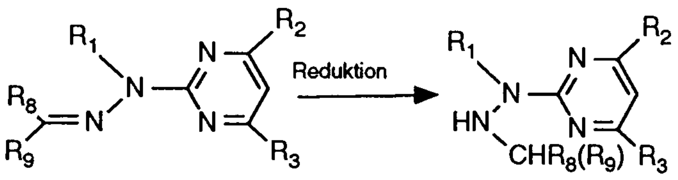


(VII) (VIII)

mittels eines Reduktionsmittels in einem inerten Lösungsmittel bei Temperaturen von 0° bis 50°C oder durch katalytische Hydrierung an Katalysatoren reduziert
oder

d) ein Pyrimidinhydrazinderivat der Formel IV

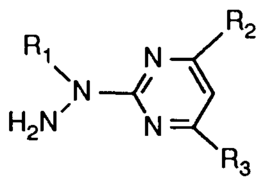

(IV)

mit einem Aldehyd oder Keton der Formel V

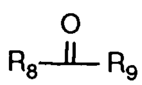

(V)

in Gegenwart eines Reduktionsmittels in einem inerten Lösungsmittel bei Temperaturen von 0° bis 50°C reduktiv alkyliert
oder

e) ein Pyrimidinhydrazin der Formel IV oder VIII mit einem Alkylhalogenid $R_oHal$

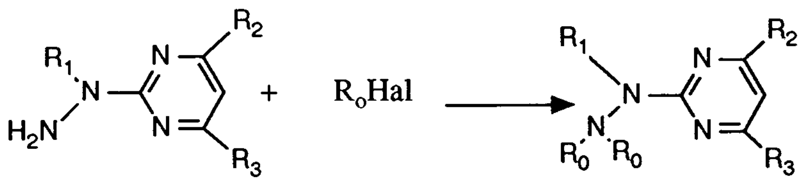


(IV) (IX)

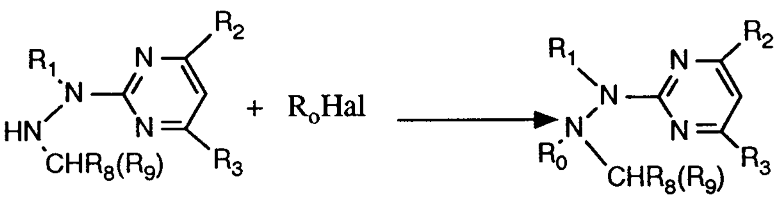


(VIII) (X)

in einem inerten Lösungsmittel in Gegenwart einer Base bei Temperaturen von 0°-60°C alkyliert, wobei Ro $C_1$-$C_4$-Alkyl darstellt und $R_1$-$R_9$ in den vorstehend beschriebenen Verfahren (a-e) die unter Formel I angegebenen Bedeutungen hat.

**2.** Verfahren gemäss Anspruch 1, dadurch gekennzeichnet, dass Verbindungen hergestellt werden worin bedeuten:
$R_1$ Phenyl oder bis zu 3-fach durch $R_4$ substituiertes Phenyl;
$R_2$ Wasserstoff, $C_1$-$C_5$-Alkyl, mit den Resten $OR_5$ oder $SR_5$ substituiertes $C_1$-$C_5$-Alkyl, $C_3$-$C_6$-Cycloalkyl mit $C_1$-$C_4$-Alkyl oder Halogen bis zu 3-fach substituiertes $C_3$-$C_6$-Cycloalkyl, $C_2$-$C_5$-Alkenyl, $C_2$-$C_5$-Alkinyl oder den Formylrest;
$R_3$ $C_1$-$C_4$-Alkyl, mit Halogen, Cyano, oder den Resten $OR_5$ oder $SR_5$ substituiertes $C_1$-$C_4$-Alkyl, $C_3$-$C_6$-Cycloalkyl oder mit $C_1$-$C_4$-Alkyl oder Halogen bis zu 3-fach substituiertes $C_3$-$C_6$-Cycloalkyl;
$R_4$ Halogen, $C_1$-$C_3$-Alkyl;
$R_5$ Wasserstoff, $C_1$-$C_5$-Alkyl, $C_3$-$C_5$-Alkenyl, $C_3$-$C_5$-Alkinyl oder den Rest $(CH_2)_n$-X-$C_1$-$C_3$-Alkyl;
$R_9$ Wasserstoff, $C_1$-$C_5$-Alkyl, $C_3$-$C_6$-Cycloalkyl, $C_3$-$C_5$-Alkenyl, $C_1$-$C_3$-Halogenalkyl;
$R_8$ und $R_9$ zusammen mit dem Kohlenstoffatom in Rest $R_7$ einen gesättigten oder ungesättigten Ring bestehend aus 5 oder 6 Kohlenstoffatomen; $R_7$, $R_{10}$, $R_{11}$, $R_{12}$ und Z besitzen die unter Formel I angegebenen Bedeutungen;
X Sauerstoff oder Schwefel;
n 1 bis 3.

**3.** Verfahren gemäss Anspruch 1, dadurch gekennzeichnet, dass Verbindungen hergestellt werden worin bedeuten:
$R_1$ Phenyl oder einfach durch Halogen substituiertes Phenyl;
$R_2$ Wasserstoff, $C_1$-$C_5$-Alkyl, mit $OR_5$ substituiertes $C_1$-$C_5$-Alkyl, $C_3$-$C_6$-Cycloalkyl, mit $C_1$-$C_4$-Alkyl oder Halogen bis zu 3-fach substituiertes $C_3$-$C_6$-Cycloalkyl, $C_2$-$C_5$-Alkenyl, $C_2$-$C_5$-Alkinyl oder den Formylrest;
$R_3$ $C_1$-$C_4$-Alkyl, mit Halogen, Cyano oder $OR_5$ substituiertes $C_1$-$C_4$-Alkyl, $C_3$-$C_6$-Cycloalkyl oder mit Methyl substituiertes $C_3$-$C_6$-Cycloalkyl;
$R_5$ Wasserstoff oder $C_1$-$C_2$-Alkyl;
$R_9$ Wasserstoff, $C_1$-$C_5$-Alkyl, $C_3$-$C_6$-Cycloalkyl, $C_3$-$C_5$-Alkenyl, $C_1$-$C_3$-Halogenalkyl;
$R_8$ und $R_9$ zusammen mit dem Kohlenstoffatom in Rest $R_7$ einen gesättigten oder ungesättigten Ring bestehend aus 5 oder 6 Kohlenstoffatomen;
$R_7$, $R_{10}$, $R_{11}$ und $R_{12}$ besitzen die unter Formel I angegebenen Bedeutungen.

**4.** Verfahren gemäss Anspruch 1, dadurch gekennzeichnet, dass Verbindungen hergestellt werden worin bedeuten:
$R_1$ Phenyl oder bis zu 3-fach durch $R_4$ substituiertes Phenyl;
$R_2$ Wasserstoff, $C_1$-$C_5$-Alkyl, mit den Resten $OR_5$ oder $SR_5$ substituiertes $C_1$-$C_5$-Alkyl, $C_3$-$C_6$-Cycloalkyl, mit $C_1$-$C_4$-Alkyl oder Halogen bis zu 3-fach substituiertes $C_3$-$C_6$-Cycloalkyl, $C_2$-$C_5$-Alkenyl, $C_2$-$C_5$-Alkinyl oder den Formylrest;

$R_3$ $C_1$-$C_4$-Alkyl, mit Halogen, Cyano, oder den Resten $OR_5$ oder $SR_5$ substituiertes $C_1$-$C_4$-Alkyl, $C_3$-$C_6$-Cycloalkyl oder mit $C_1$-$C_4$-Alkyl oder Halogen bis zu 3-fach substituiertes $C_3$-$C_6$-Cycloalkyl;
$R_4$ Halogen, $C_1$-$C_3$-Alkyl, $C_1$-$C_2$-Halogenalkyl, $C_1$-$C_3$-Alkoxy oder $C_1$-$C_3$-Halogenalkoxy;
$R_5$ Wasserstoff, $C_1$-$C_5$-Alkyl, $C_3$-$C_5$-Alkenyl, $C_3$-$C_5$-Alkinyl oder den Rest $(CH_2)_n$-X-$C_1$-$C_3$-Alkyl;
$R_7$-$NH_2$;
X Sauerstoff oder Schwefel;
n 1-3; unter Einschluss ihrer Säureadditionssalze und Metallsalzkomplexe.

**5.** Verfahren gemäss Anspruch 4, dadurch gekennzeichnet, dass Verbindungen hergestellt werden worin bedeuten:
$R_1$ Phenyl oder bis zu 3-fach durch $R_4$ substituiertes Phenyl;
$R_2$ Wasserstoff, $C_1$-$C_5$-Alkyl, mit den Resten $OR_5$ oder $SR_5$ substituiertes $C_1$-$C_5$-Alkyl, $C_3$-$C_6$-Cycloalkyl, mit $C_1$-$C_4$-Alkyl oder Halogen bis zu 3-fach substituiertes $C_3$-$C_6$-Cycloalkyl, $C_2$-$C_5$-Alkenyl, $C_2$-$C_5$-Alkinyl oder den Formylrest;
$R_3$ $C_1$-$C_4$-Alkyl, mit Halogen, Cyano, oder den Resten $OR_5$ oder $SR_5$ substituiertes $C_1$-$C_4$-Alkyl, $C_3$-$C_6$-Cycloalkyl oder mit $C_1$-$C_4$-Alkyl oder Halogen bis zu 3-fach substituiertes $C_3$-$C_6$-Cycloalkyl;
$R_4$ Halogen;
$R_5$ Wasserstoff, $C_1$-$C_5$-Alkyl, $C_3$-$C_5$-Alkenyl, $C_3$-$C_5$-Alkinyl oder den Rest $(CH_2)_n$-X-$C_1$-$C_3$-Alkyl;
X Sauerstoff oder Schwefel;
n 1-3.

**6.** Verfahren gemäss Anspruch 1, dadurch gekennzeichnet, dass Verbindungen hergestellt werden worin bedeuten:
$R_1$ Phenyl oder bis zu 3-fach durch Halogen substituiertes Phenyl;
$R_2$ Wasserstoff, $C_1$-$C_5$-Alkyl, mit den Resten $OR_5$ oder $SR_5$ substituiertes $C_1$-$C_5$-Alkyl, $C_3$-$C_6$-Cycloalkyl mit $C_1$-$C_4$-Alkyl oder Halogen bis zu 3-fach substituiertes $C_3$-$C_6$-Cycloalkyl, $C_2$-$C_5$-Alkenyl, $C_2$-$C_5$-Alkinyl oder den Formylrest;
$R_3$ $C_1$-$C_4$-Alkyl, mit Halogen, Cyano, oder den Resten $OR_5$ oder $SR_5$ substituiertes $C_1$-$C_4$-Alkyl, $C_3$-$C_6$-Cycloalkyl oder mit $C_1$-$C_4$-Alkyl oder Halogen bis zu 3-fach substituiertes $C_3$-$C_6$-Cycloalkyl;
$R_5$ Wasserstoff, $C_1$-$C_5$-Alkyl, $C_3$-$C_5$-Alkenyl, $C_3$-$C_5$-Alkinyl oder den Rest $(CH_2)_n$-X-$C_1$-$C_3$-Alkyl;
X Sauerstoff oder Schwefel;
n 1-3.

**7.** Verfahren gemäss Anspruch 1, dadurch gekennzeichnet, dass Verbindungen hergestellt werden worin bedeuten:
$R_1$ Phenyl oder einfach durch Chlor oder Fluor substituiertes Phenyl;
$R_2$ $C_1$-$C_5$-Alkyl, mit $OR_5$ substituiertes $C_1$-$C_2$-Alkyl, $C_3$-$C_6$-Cycloalkyl mit $C_1$-$C_4$-Alkyl oder Halogen bis zu 3-fach substituiertes $C_3$-$C_6$-Cycloalkyl, $C_2$-$C_5$-Alkenyl,
$C_2$-$C_5$-Alkinyl oder den Formylrest;
$R_3$ $C_1$-$C_4$-Alkyl, $C_1$-$C_4$-Halogenalkyl, $C_3$-$C_6$-Cycloalkyl oder mit Methyl substituiertes $C_3$-$C_6$-Cycloalkyl;
$R_5$ Wasserstoff oder $C_1$-$C_2$-Alkyl.

**8.** Verfahren gemäss den Ansprüchen 6 und 7, dadurch gekennzeichnet, dass Verbindungen hergestellt werden worin $R_3$ bedeutet: Methyl, Fluormethyl, Chlormethyl, Brommethyl, $C_3$-$C_6$-Cycloalkyl oder Methoxymethyl.

**9.** Verfahren gemäss Anspruch 1, dadurch gekennzeichnet, dass eine Verbindung hergestellt wird, ausgewählt aus :
N-(4-Fluormethyl-6-cyclopropylpyrimidyl-2)-N-phenyl-hydrazin;
N-(4-Methyl-6-cyclopropylpyrimidyl-2)-N-m-fluorphenyl-hydrazin;
N-(4-Methyl-6-cyclopropylpyrimidyl-2)-N-p-fluorphenyl-hydrazin.

**10.** Verfahren gemäss Anspruch 5, dadurch gekennzeichnet, dass eine Verbindung hergestellt wird, ausgewählt aus :
N-(4-Methyl-6-cyclopropylpyrimidy-2)-N-phenyl-hydrazin;
N-(4,6-Dimethylpyrimidyl-2)-N-phenyl-hydrazin;
N-(4-Methyl-6-methoxymethylpyrimidyl-2)-N-phenyl-hydrazin.

**11.** Verfahren gemäss Anspruch 1, dadurch gekennzeichnet, dass eine Verbindung hergestellt wird, ausgewählt aus :
N-(4,6-Dimethylpyrimidy-2)-N-phenyl-propionaldehydhydrazon;
N-(4,6-Dimethylpyrimidyl-2)-N-phenyl-isobutyraldehydhydrazon;
N-(4-Methyl-6-methoxymethylpyrimidyl-2)-N-phenyl-isobutyraldehydhydrazon;
N-(4-Methyl-6-methoxymethylpyrimidyl-2)-N-phenyl-propionaldehydhydrazon;
N-(4-Methyl-6-cyclopropylpyrimidyl-2)-N-phenyl-propionaldehydhydrazon;
N-(4-Methyl-6-cyclopropylpyrimidyl-2)-N-phenyl-n-butyraldehydhydrazon;

N-(4-Methyl-6-cyclopropylpyrimidyl-2)-N-phenyl-isobutyraldehydhydrazon;
N-(4-Methyl-6-cyclopropylpyrimidyl-2)-N-phenyl-trichloracetaldehydhydrazon;
N-(4-Methyl-6-cyclopropylpyrimidyl-2)-N-p-fluorphenyl-acetaldehydhydrazon;
N-(4-Methyl-6-cyclopropylpyrimidyl-2)-N-p-fluorphenyl-isobutyraldehydhydrazon;
N-(4-Methyl-6-cyclopropylpyrimidyl-2)-N-m-fluorphenyl-isobutyraldehydhydrazon;
N-(4,6-Dimethylpyrimidyl-2)-N-phenyl-N'-methylhydrazin;
N-(4,6-Dimethylpyrimidyl-2)-N-phenyl-N'-dimethylhydrazin;
N-(4,6-Dimethylpyrimidyl-2)-N-phenyl-N'-n-propylhydrazin;
N-(4,6-Dimethylpyrimidyl-2)-N-phenyl-N'-isobutylhydrazin;
N-(4-Methyl-6-methoxymethylpyrimidyl-2)-N-phenyl-N'-methylhydrazin;
N-(4-Methyl-6-methoxymethylpyrimidyl-2)-N-phenyl-N'-n-propylhydrazin;
N-(4-Methyl-6-methoxymethylpyrimidyl-2)-N-phenyl-N'-dimethylhydrazin;
N-(4-Methyl-6-cyclopropylpyrimidyl-2)-N-phenyl-N'-methylhydrazin;
N-(4-Methyl-6-cyclopropylpyrimidyl-2)-N-phenyl-N'-isobutylhydrazin;
N-(4-Methyl-6-cyclopropylpyrimidyl-2)-N-phenyl-N'-dimethylhydrazin;
N-(4-Methyl-6-cyclopropylpyrimidyl-2)-N-phenyl-N'-diäthylhydrazin;
N-(4-Methyl-6-cyclopropylpyrimidyl-2)-N-phenyl-N'-methyl-N'-äthylhydrazin;
N-(4-Methyl-6-cyclopropylpyrimidyl-2)-N-p-fluorpheny-N'-äthylhydrazin;
N-(4-Methyl-6-methoxymethylpyrimidyl-2)-N-m-fluorphenyl-N'-isopropylhydrazin.

**12.** Mittel zur Bekämpfung oder Verhütung eines Befalls durch schädliche Insekten oder Mikroorganismen, dadurch gekennzeichnet, dass es, zusammen mit einem geeigneten Trägermaterialal, als aktive Komponente mindestens eine Verbindung der Formel I enthält

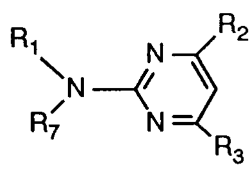
(I)

in welcher bedeuten: $R_1$ Phenyl oder bis zu 3-fach durch $R_4$ substituiertes Phenyl;
$R_2$ Wasserstoff, $C_1$-$C_5$-Alkyl, mit den Resten $OR_5$ oder $SR_5$ substituiertes $C_1$-$C_5$-Alkyl, $C_3$-$C_6$-Cycloalkyl, mit $C_1$-$C_4$-Alkyl oder Halogen bis zu 3-fach substituiertes $C_3$-$C_6$-Cycloalkyl, $C_2$-$C_5$-Alkenyl, $C_2$-$C_5$-Alkinyl oder den Formylrest;
$R_3$ Wasserstoff, $C_1$-$C_4$-Alkyl, mit Halogen, Cyano, oder den Resten $OR_5$ oder $SR_5$ substituiertes $C_1$-$C_4$-Alkyl, $C_3$-$C_6$-Cycloalkyl oder mit $C_1$-$C_4$-Alkyl oder Halogen bis zu 3-fach substituiertes $C_3$-$C_6$-Cycloalkyl;
$R_4$ Halogen, $C_1$-$C_3$-Alkyl, $C_1$-$C_2$-Halogenalkyl, $C_1$-$C_3$-Alkoxy oder $C_1$-$C_3$-Halogenalkoxy;
$R_5$ Wasserstoff, $C_1$-$C_5$-Alkyl, $C_3$-$C_5$-Alkenyl, $C_3$-$C_5$-Alkinyl oder den Rest $(CH_2)_n$-X-$C_1$-$C_3$-Alkyl;
$R_7$ die Gruppe $-NH_2$,

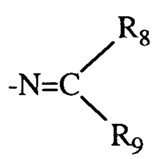 oder 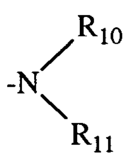 ;

$R_8$ Wasserstoff, $C_1$-$C_3$-Alkyl oder $C_1$-$C_3$-Halogenalkyl;
$R_9$ Wasserstoff, $C_1$-$C_8$-Alkyl, durch Hydroxy, $OR_{12}$, $SR_{12}$ oder $N(R_{12})_2$ substituiertes $C_1$-$C_3$-Alkyl, $C_3$-$C_6$-Cycloalkyl, durch $SR_{12}$ substituiertes Cyclopropyl, $C_3$-$C_{10}$-Alkenyl, $C_1$-$C_3$-Halogenalkyl, 1-, 2- oder 3-Pyridyl,

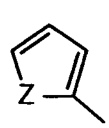 oder 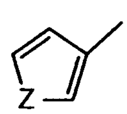 ;

$R_8$ und $R_9$ zusammen mit dem Kohlenstoffatom im Rest $R_7$ einen gesättigten oder ungesättigten Ring, bestehend aus 4 bis 7 Kohlenstoffatomen;
$R_{10}$ $CH(R_8)R_9$, Phenyl, $C_3$-$C_5$-Alkenyl, $C_3$-$C_5$-Alkinyl oder Cyanoalkyl mit 2 oder 3 Kohlenstoffatomen im Alkylrest;
$R_{11}$ Wasserstoff, $C_1$-$C_5$-Alkyl, $C_3$-$C_5$-Alkenyl, $C_3$-$C_5$-Alkinyl oder Cyanoalkyl mit 2 oder 3 Kohlenstoffatomen im Alkylrest;
$R_{12}$ $CH_3$ oder $C_2H_5$;
X Sauerstoff oder Schwefel;
Z O, S, NH oder $NCH_3$; und
n 1 bis 3.

**13.** Mittel gemäss Anspruch 12, dadurch gekennzeichnet, dass es als aktive Komponente mindestens eine Verbindung der Formel I enthält, in welcher bedeuten
$R_1$ Phenyl oder bis zu 3-fach durch $R_4$ substituiertes Phenyl;
$R_2$ Wasserstoff, $C_1$-$C_5$-Alkyl, mit den Resten $OR_5$ oder $SR_5$ substituiertes $C_1$-$C_5$-Alkyl, $C_3$-$C_6$-Cycloalkyl, mit $C_1$-$C_4$-Alkyl oder Halogen bis zu 3-fach substituiertes $C_3$-$C_6$-Cycloalkyl, $C_2$-$C_5$-Alkenyl, $C_2$-$C_5$-Alkinyl oder den Formylrest;
$R_3$ $C_1$-$C_4$-Alkyl, mit Halogen, Cyano, oder den Resten $OR_5$ oder $SR_5$ substituiertes $C_1$-$C_4$-Alkyl, $C_3$-$C_6$-Cycloalkyl oder mit $C_1$-$C_4$-Alkyl oder Halogen bis zu 3-fach substituiertes $C_3$-$C_6$-Cycloalkyl;
$R_4$ Halogen, $C_1$-$C_3$-Alkyl, $C_1$-$C_2$-Halogenalkyl, $C_1$-$C_3$-Alkoxy oder $C_1$-$C_3$-Halogenalkoxy;
$R_5$ Wasserstoff, $C_1$-$C_5$-Alkyl, $C_3$-$C_5$-Alkenyl, $C_3$-$C_5$-Alkinyl oder den Rest $(CH_2)_n$-X-$C_1$-$C_3$-Alkyl;
$R_7$ $-NH_2$;
X Sauerstoff oder Schwefel;
n 1-3.

**14.** Mittel gemäss Anspruch 12, dadurch gekennzeichnet, dass es als aktive Komponente mindestens eine Verbindung der Formel I enthält, ausgewählt aus
N-(4-Fluormethyl-6-cyclopropylpyrimidyl-2)-N-phenyl-hydrazin;
N-(4-Methyl-6-cyclopropylpyrimidyl-2)-N-m-fluorphenyl-hydrazin;
N-(4-Methyl-6-cyclopropylpyrimidyl-2)-N-p-fluorphenyl-hydrazin.

**15.** Mittel gemäss Anspruch 12, dadurch gekennzeichnet, dass es als aktive Komponente mindestens eine Verbindung der Formel I enthält, ausgewählt aus
N-(4-Methyl-6-cyclopropylpyrimidyl-2)-N-phenyl-hydrazin;
N-(4,6-Dimethylpyrimidyl-2)-N-phenyl-hydrazin;
N-(4-Methyl-6-methoxymethylpyrimidyl-2)-N-phenyl-hydrazin.

**16.** Mittel gemäss Anspruch 12, dadurch gekennzeichnet, dass es als aktive Komponente mindestens eine Verbindung der Formel I enthält, ausgewählt aus
N-(4,6-Dimethylpyrimidyl-2)-N-phenyl-propionaldehydhydrazon;
N-(4,6-Dimethylpyrimidyl-2)-N-phenyl-isobutyraldehydhydrazon;
N-(4-Methyl-6-methoxymethylpyrimidyl-2)-N-phenyl-isobutyraldehydhydrazon;
N-(4-Methyl-6-methoxymethylpyrimidyl-2)-N-phenyl-propionaldehydhydrazon;
N-(4-Methyl-6-cyclopropylpyrimidyl-2)-N-phenyl-propionaldehydhydrazon;
N-(4-Methyl-6-cyclopropylpyrimidyl-2)-N-phenyl-n-butyraldehydhydrazon;
N-(4-Methyl-6-cyclopropylpyrimidyl-2)-N-phenyl-isobutyraldehydhydrazon;
N-(4-Methyl-6-cyclopropylpyrimidyl-2)-N-phenyl-trichloracetaldehydhydrazon;
N-(4-Methyl-6-cyclopropylpyrimidyl-2)-N-p-fluorphenyl-acetaldehydhydrazon;
N-(4-Methyl-6-cyclopropylpyrimidyl-2)-N-p-fluorphenyl-isobutyraldehydhydrazon;
N-(4-Methyl-6-cyclopropylpyrimidyl-2)-N-m-fluorphenyl-isobutyraldehydhydrazon;
N-(4,6-Dimethylpyrimidyl-2)-N-phenyl-N'-methylhydrazin;
N-(4,6-Dimethylpyrimidyl-2)-N-phenyl-N'-dimethylhydrazin;
N-(4,6-Dimethylpyrimidyl-2)-N-phenyl-N'-n-propylhydrazin;
N-(4,6-Dimethylpyrimidyl-2)-N-phenyl-N'-isobutylhydrazin;
N-(4-Methyl-6-methoxymethylpyrimidyl-2)-N-phenyl-N'-methylhydrazin;
N-(4-Methyl-6-methoxymethylpyrimidyl-2)-N-phenyl-N'-n-propylhydrazin;
N-(4-Methyl-6-methoxymethylpyrimidyl-2)-N-phenyl-N'-dimethylhydrazin;
N-(4-Methyl-6-cyclopropylpyrimidyl-2)-N-phenyl-N'-methylhydrazin;
N-(4-Methyl-6-cyclopropylpyrimidyl-2)-N-phenyl-N'-isobutylhydrazin;
N-(4-Methyl-6-cyclopropylpyrimidyl-2)-N-phenyl-N'-dimethylhydrazin;

N-(4-Methyl-6-cyclopropylpyrimidyl-2)-N-phenyl-N'-diäthylhydrazin;
N-(4-Methyl-6-cyclopropylpyrimidyl-2)-N-phenyl-N'-methyl-N'-äthylhydrazin;
N-(4-Methyl-6-cyclopropylpyrimidyl-2)-N-p-fluorphenyl-N'-äthylhydrazin;
N-(4-Methyl-6-methoxymethylpyrimidyl-2)-N-m-fluorphenyl-N'-isopropylhydrazin.

**17.** Verfahren zur Bekämpfung oder Verhütung eines Befalls von Kulturpflanzen durch Schadinsekten oder phytopathogene Mikroorganismen, dadurch gekennzeichnet, dass man als Wirkstoff eine Verbindung der Formel I hergestellt gemäss Anspruch 1 auf die Pflanze, auf Pflanzenteile oder ihren Standort appliziert.

**18.** Verfahren gemäss Anspruch 17, dadurch gekennzeichnet, dass man als Wirkstoff eine Verbindung hergestellt gemäss einem der Ansprüche 2 bis 11 appliziert.

**19.** Verfahren gemäss Anspruch 17, dadurch gekennzeichnet, dass phytopathogene Pilze bekämpft werden.

**20.** Verfahren zur Herstellung eines agrochemischen Mittels gemäss Anspruch 12, dadurch gekennzeichnet, dass man mindestens eine Verbindung der Formel I hergestellt gemäss Anspruch 1 mit geeigneten festen oder flüssigen Zusatzstoffen und/oder Tensiden innig vermischt.

## Claims

**Claims for the following Contracting States : AT, BE, CH, LI, DE, FR, GB, GR, IT, LU, NL, SE**

**1.** A compound of the formula I

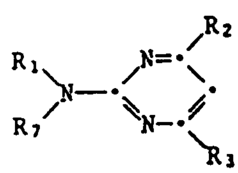

(I)

wherein: $R_1$ is phenyl or phenyl mono- to tri-substituted by $R_4$; $R_2$ is hydrogen, $C_1$-$C_5$alkyl, $C_1$-$C_5$alkyl substituted by the radical $OR_5$ or by the radical $SR_5$, $C_3$-$C_6$cycloalkyl, $C_3$-$C_6$cycloalkyl mono- to tri-substituted by $C_1$-$C_4$alkyl or by halogen, $C_2$-$C_5$alkenyl, $C_2$-$C_5$alkynyl or the formyl radical; $R_3$ is hydrogen, $C_1$-$C_4$alkyl, $C_1$-$C_4$alkyl substituted by halogen, cyano or by the radical $OR_5$ or by the radical $SR_5$, $C_3$-$C_6$cycloalkyl or $C_3$-$C_6$cycloalkyl mono- to tri-substituted by $C_1$-$C_4$alkyl or by halogen; $R_4$ is halogen, $C_1$-$C_3$alkyl, $C_1$-$C_2$haloalkyl, $C_1$-$C_3$alkoxy or $C_1$-$C_3$haloalkoxy; $R_5$ is hydrogen, $C_1$-$C_5$alkyl, $C_1$-$C_5$alkenyl, $C_1$-$C_5$alkynyl or the radical $(CH_2)_n$-X-$C_1$-$C_3$alkyl;
$R_7$ is the group $-NH_2$,

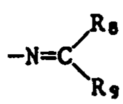 or 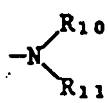 ; ;

$R_8$ is hydrogen, $C_1$-$C_3$alkyl or $C_1$-$C_3$haloalkyl; $R_9$ is hydrogen, $C_1$-$C_8$alkyl, $C_1$-$C_3$alkyl substituted by hydroxy, $OR_{12}$, $SR_{12}$ or by $N(R_{12})_2$, $C_3$-$C_6$cycloalkyl, cyclopropyl substituted by $SR_{12}$, $C_3$-$C_{10}$alkenyl, $C_1$-$C_3$haloalkyl, 1-, 2- or 3-pyridyl,

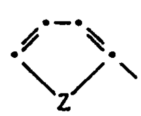 or 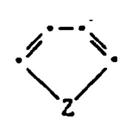 ;

$R_8$ and $R_9$, together with the carbon atom in the radical $R_7$, are a saturated or unsaturated ring comprising 4 to 7 carbon atoms; $R_{10}$ is $CH(R_8)R_9$, phenyl, $C_3$-$C_5$alkenyl, $C_3$-$C_5$alkynyl or cyanoalkyl having 2 or 3 carbon atoms in the alkyl radical; $R_{11}$ is hydrogen, $C_1$-$C_5$alkyl, $C_3$-$C_5$alkenyl, $C_3$-$C_5$alkynyl or cyanoalkyl having 2 or 3 carbon atoms in the alkyl radical; $R_{12}$ is $CH_3$ or $C_2H_5$; X is oxygen or sulfur; Z is O, S, NH or $NCH_3$; and n is 1 to 3; including the acid addition salts and metal salt complexes thereof.

2. A compound of the formula I according to claim 1, wherein: $R_1$ is phenyl or phenyl mono- to tri-substituted by $R_4$; $R_2$ is hydrogen, $C_1$-$C_5$alkyl, $C_1$-$C_5$alkyl substituted by the radical $OR_5$ or by the radical $SR_5$, $C_3$-$C_6$cycloalkyl, $C_3$-$C_6$cycloalkyl mono- to tri-substituted by $C_1$-$C_4$alkyl or by halogen, $C_2$-$C_5$alkenyl, $C_2$-$C_5$alkynyl or the formyl radical; $R_3$ is $C_1$-$C_4$alkyl, $C_1$-$C_4$alkyl substituted by halogen, cyano or by the radical $OR_5$ or by the radical $SR_5$, $C_3$-$C_6$cycloalkyl or $C_3$-$C_6$cycloalkyl mono- to tri-substituted by $C_1$-$C_4$alkyl or by halogen; $R_4$ is halogen or $C_1$-$C_3$alkyl; $R_5$ is hydrogen, $C_1$-$C_5$alkyl, $C_3$-$C_5$alkenyl, $C_3$-$C_5$alkynyl or the radical $(CH_2)_n$-X-$C_1$-$C_3$alkyl; $R_9$ is hydrogen, $C_1$-$C_5$alkyl, $C_3$-$C_6$cycloalkyl, $C_5$-$C_5$alkenyl, $C_1$-$C_3$haloalkyl; $R_8$ and $R_9$, together with the carbon atom in the radical $R_7$ are a saturated or unsaturated ring comprising 5 or 6 carbon atoms; $R_7$, $R_{10}$, $R_{11}$, $R_{12}$ and Z are as defined under formula I; X is oxygen or sulfur; and n is 1 to 3.

3. A compound of the formula I according to claim 1, wherein: $R_1$ is phenyl or phenyl mono-substituted by halogen; $R_2$ is hydrogen, $C_1$-$C_5$alkyl, $C_1$-$C_5$alkyl substituted by $OR_5$, $C_3$-$C_6$cycloalkyl, $C_3$-$C_6$cycloalkyl mono- to tri-substituted by $C_1$-$C_4$alkyl or by halogen, $C_2$-$C_5$alkenyl, $C_2$-$C_5$alkynyl or the formyl radical; $R_3$ is $C_1$-$C_4$alkyl, $C_1$-$C_4$alkyl substituted by halogen, cyano or by $OR_5$, $C_3$-$C_6$cycloalkyl or $C_3$-$C_6$cycloalkyl substituted by methyl; $R_5$ is hydrogen or $C_1$-$C_2$alkyl; $R_9$ is hydrogen, $C_1$-$C_5$alkyl, $C_3$-$C_6$cycloalkyl, $C_3$-$C_5$alkenyl, $C_1$-$C_3$haloalkyl, $R_8$ and $R_9$, together with the carbon atom in the radical $R_7$, are a saturated or unsaturated ring comprising 5 or 6 carbon atoms; and $R_7$, $R_{10}$, $R_{11}$ and $R_{12}$ are as defined under formula I.

4. A compound of formula I in claim 1, wherein: $R_1$ is phenyl or phenyl mono- to tri-substituted by $R_4$; $R_2$ is hydrogen, $C_1$-$C_5$alkyl, $C_1$-$C_5$alkyl substituted by the radical $OR_5$ or by the radical $SR_5$, $C_3$-$C_6$cycloalkyl, $C_3$-$C_6$cycloalkyl mono- to tri-substituted by $C_1$-$C_4$alkyl or by halogen, $C_2$-$C_5$alkenyl, $C_2$-$C_5$alkynyl or the formyl radical; $R_3$ is $C_1$-$C_4$alkyl, $C_1$-$C_4$alkyl substituted by halogen, cyano or by the radical $OR_5$ or by the radical $SR_5$, $C_3$-$C_6$cycloalkyl or $C_3$-$C_6$cycloalkyl mono- to tri-substituted by $C_1$-$C_4$alkyl or by halogen; $R_4$ is halogen, $C_1$-$C_3$alkyl, $C_1$-$C_2$haloalkyl, $C_1$-$C_3$alkoxy or $C_1$-$C_3$haloalkoxy; $R_5$ is hydrogen, $C_1$-$C_5$alkyl, $C_3$-$C_5$alkenyl, $C_3$-$C_5$alkynyl or the radical $(CH_2)_n$-X-$C_1$-$C_3$alkyl; $R_7$ is $-NH_2$; X is oxygen or sulfur; and n is 1 to 3; including the acid addition salts and metal salt complexes thereof.

5. A compound of the formula I according to claim 4, wherein: $R_1$ is phenyl or phenyl mono- to tri-substituted by $R_4$; $R_2$ is hydrogen, $C_1$-$C_5$alkyl, $C_1$-$C_5$alkyl substituted by the radical $OR_5$ or by the radical $SR_5$, $C_3$-$C_6$cycloalkyl, $C_3$-$C_6$cycloalkyl mono- to tri-substituted by $C_1$-$C_4$alkyl or by halogen, $C_2$-$C_5$alkenyl, $C_2$-$C_5$alkynyl or the formyl radical; $R_3$ is $C_1$-$C_4$alkyl, $C_1$-$C_4$alkyl substituted by halogen, cyano or by the radical $OR_5$ or by the radical $SR_5$, $C_3$-$C_6$cycloalkyl or $C_3$-$C_6$cycloalkyl mono- to tri-substituted by $C_1$-$C_4$alkyl or by halogen; $R_4$ is halogen; $R_5$ is hydrogen, $C_1$-$C_5$alkyl, $C_3$-$C_5$alkenyl, $C_3$-$C_5$alkynyl or the radical $(CH_2)_n$-X-$C_1$-$C_3$alkyl; X is oxygen or sulfur; and n is 1 to 3.

6. A compound of the formula I according to claim 1, wherein: $R_1$ is phenyl or phenyl mono- to tri-substituted by halogen; $R_2$ is hydrogen, $C_1$-$C_5$alkyl, $C_1$-$C_5$alkyl substituted by the radical $OR_5$ or by the radical $SR_5$, $C_3$-$C_6$cycloalkyl, $C_3$-$C_6$cycloalkyl mono- to tri-substituted by $C_1$-$C_4$alkyl or by halogen, $C_2$-$C_5$alkenyl, $C_2$-$C_5$alkynyl or the formyl radical; $R_3$ is $C_1$-$C_4$alkyl, $C_1$-$C_4$alkyl substituted by halogen, cyano or by the radical $OR_5$ or by the radical $SR_5$, $C_3$-$C_6$cycloalkyl or $C_3$-$C_6$cycloalkyl mono- to tri-substituted by $C_1$-$C_4$alkyl or by halogen; $R_5$ is hydrogen, $C_1$-$C_5$alkyl, $C_3$-$C_5$alkenyl, $C_3$-$C_5$alkynyl or the radical $(CH_2)_n$-X-$C_1$-$C_3$alkyl; X is oxygen or sulfur; and n is 1 to 3.

7. A compound of the formula I according to claim 1, wherein: $R_1$ is phenyl or phenyl mono-substituted by chlorine or by fluorine; $R_2$ is $C_1$-$C_5$alkyl, or is $C_1$-$C_2$alkyl substituted by $OR_5$, $C_3$-$C_6$cycloalkyl, $C_3$-$C_6$cycloalkyl mono- to tri-substituted by $C_1$-$C_4$alkyl or by halogen, $C_2$-$C_5$alkenyl, $C_2$-$C_5$alkynyl or the formyl radical; $R_3$ is $C_1$-$C_4$alkyl, $C_1$-$C_4$haloalkyl, $C_3$-$C_6$cycloalkyl or $C_3$-$C_6$cycloalkyl substituted by methyl; and $R_5$ is hydrogen or $C_1$-$C_2$alkyl.

8. A compound of the formula I according to claims 6 and 7, wherein $R_3$ is: methyl, fluoromethyl, chloromethyl, bromomethyl, $C_3$-$C_6$cycloalkyl or methoxymethyl.

9. A compound of formula I according to claim 1, from the group:
N-(4-fluoromethyl-6-cyclopropylpyrimid-2-yl)-N-phenylhydrazine;
N-(4-methyl-6-cyclopropylpyrimid-2-yl)-N-m-fluorophenylhydrazine;
N-(4-methyl-6-cyclopropylpyrimid-2-yl)-N-p-fluorophenylhydrazine.

10. A compound of formula I according to claim 5 from the group:
N-(4-methyl-6-cyclopropylpyrimid-2-yl)-N-phenylhydrazine; N-(4,6-dimethylpyrimid-2-yl)-N-phenylhydrazine; N-(4-methyl-6-methoxymethylpyrimid-2-yl)-N-phenylhydrazine.

11. A compound of formula I according to claim 1 from the group:
N-(4,6-dimethylpyrimid-2-yl)-N-phenylpropionaldehyde hydrazone;
N-(4,6-dimethylpyrimid-2-yl)-N-phenylisobutyraldehyde hydrazone;
N-(4-methyl-6-methoxymethylpyrimid-2-yl)-N-phenylisobutyraldehyde hydrazone;
N-(4-methyl-6-methoxymethylpyrimid-2-yl)-N-phenylpropionaldehyde hydrazone;
N-(4-methyl-6-cyclopropylpyrimid-2-yl)-N-phenylpropionaldehyde hydrazone;
N-(4-methyl-6-cyclopropylpyrimid-2-yl)-N-phenyl-n-butyraldehyde hydrazone;
N-(4-methyl-6-cyclopropylpyrimid-2-yl)-N-phenylisobutyraldehyde hydrazone;
N-(4-methyl-6-cyclopropylpyrimid-2-yl)-N-phenyltrichloroacetaldehyde hydrazone;
N-(4-methyl-6-cyclopropylpyrimid-2-yl)-N-p-fluorophenylacetaldehyde hydrazone;
N-(4-methyl-6-cyclopropylpyrimid-2-yl)-N-p-fluorophenylisobutyraldehyde hydrazone;
N-(4-methyl-6-cyclopropylpyrimid-2-yl)-N-m-fluorophenylisobutyraldehyde hydrazone;
N-(4,6-dimethylpyrimid-2-yl)-N-phenyl-N'-methylhydrazine;
N-(4,6-dimethylpyrimid-2-yl)-N-phenyl-N'-dimethylhydrazine;
N-(4,6-dimethylpyrimid-2-yl)-N-phenyl-N'-n-propylhydrazine;
N-(4,6-dimethylpyrimid-2-yl)-N-phenyl-N'-isobutylhydrazine;
N-(4-methyl-6-methoxymethylpyrimid-2-yl)-N-phenyl-N'-methylhydrazine;
N-(4-methyl-6-methoxymethylpyrimid-2-yl)-N-phenyl-N'-n-propylhydrazine;
N-(4-methyl-6-methoxymethylpyrimid-2-yl)-N-phenyl-N'-dimethylhydrazine;
N-(4-methyl-6-cyclopropylpyrimid-2-yl)-N-phenyl-N'-methylhydrazine;
N-(4-methyl-6-cyclopropylpyrimid-2-yl)-N-phenyl-N'-isobutylhydrazine;
N-(4-methyl-6-cyclopropylpyrimid-2-yl)-N-phenyl-N'-dimethylhydrazine;
N-(4-methyl-6-cyclopropylpyrimid-2-yl)-N-phenyl-N'-diethylhydrazine;
N-(4-methyl-6-cyclopropylpyrimid-2-yl)-N-phenyl-N'-methyl-N'-ethylhydrazine;
N-(4-methyl-6-cyclopropylpyrimid-2-yl)-N-p-fluorophenyl-N'-ethylhydrazine;
N-(4-methyl-6-methoxymethylpyrimid-2-yl)-N-m-fluorophenyl-N'-isopropylhydrazine.

12. A process for the preparation of a compound of the formula I according to claim 1, which comprises

a) reacting a pyrimidine derivative of the formula II

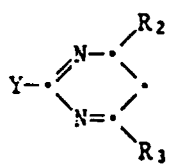

(II)

with a phenylhydrazine derivative of the formula III

$$R_1\text{-NH-NH-R} \quad \text{(III)}$$

in the presence of a base, in an aprotic solvent and at temperatures of -50°C to 150°C, wherein Y is halogen, the radical $SO_2R_6$ or $N^{\oplus}(CH_3)_3$, $R_6$ is $C_1$-$C_4$alkyl, phenyl or phenyl substituted by methyl or by chlorine and R is as defined for $R_{10}$ and $R_{11}$, and the latter and also $R_1$-$R_3$ are as defined under formula I,
or

b) reacting a pyrimidine hydrazine derivative of formula IV with an aldehyde or ketone of formula V to form a compound of formula VII with the removal of water

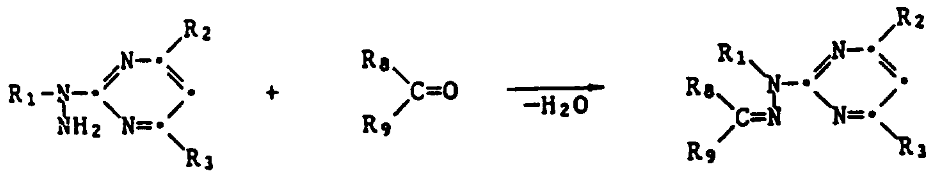


(IV) (V) (VII)

in any desired solvent, in the presence of an acid and at temperatures of -20° to 120°C, $R_1$-$R_3$ and $R_8$ and $R_9$ being as defined under formula I,
or

c) reducing a hydrazone derivative of formula VII

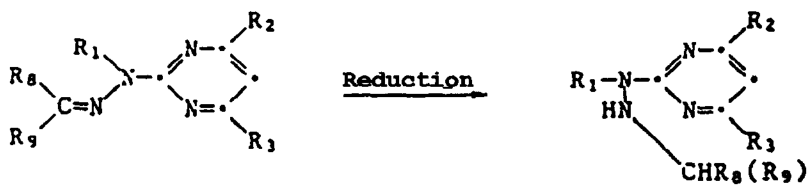


(VII) (VIII)

using a reducing agent, in an inert solvent and at temperatures of 0° to 50°C or by catalytic hydrogenation using catalysts,
or

d) subjecting a pyrimidine hydrazine derivative of formula IV

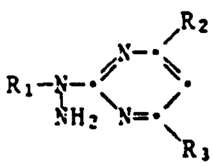

(IV)

to reductive alkylation with an aldehyde or ketone of formula V

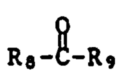

(V)

in the presence of a reducing agent, in an inert solvent and at temperatures of 0° to 50°C,
or

e) alkylating a pyrimidine hydrazine of formula IV or VIII with an alkyl halide $R_oHal$

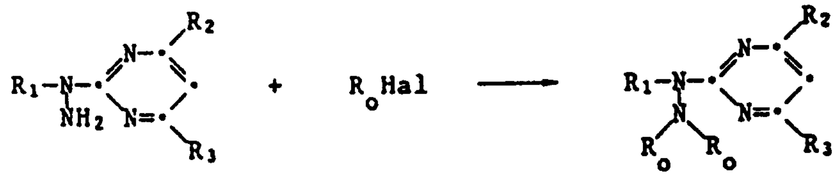

(IV) (IX)

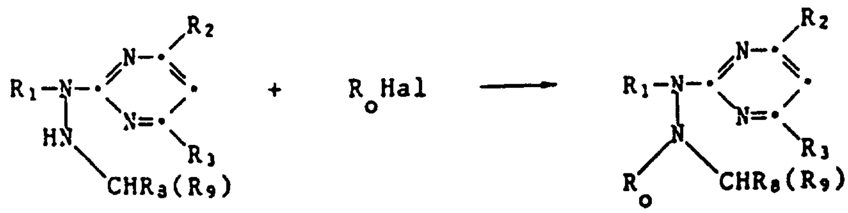

(VIII) (X)

in an inert solvent, in the presence of a base and at temperatures of 0°-60°C, $R_0$ being $C_1$-$C_4$alkyl and $R_1$-$R_9$ in processes (a-e) described above being as defined under formula I.

**13.** A composition for controlling or preventing attack by insect pests or destructive microorganisms, which composition contains as active ingredient at least one compound of formula I according to claim 1 together with a suitable carrier.

**14.** A composition according to claim 13, which contains as active ingredient at least one compound of formula I according to claim 4.

**15.** A composition according to claim 13, which contains as active ingredient at least one compound of formula I according to claim 9.

**16.** A composition according to claim 13, which contains as active ingredient at least one compound of formula I according to claim 10.

**17.** A composition according to claim 13, which contains as active ingredient at least one compound of formula I according to claim 11.

**18.** A method of controlling or preventing attacks on cultivated plants by insect pests or phytopathogenic microorganisms, which comprises applying to the plant, parts of the plant or the locus thereof, as active ingredient, a compound of formula I according to claim 1.

**19.** A method according to claim 18, which comprises applying as active ingredient a compound according to any one of claims 2 to 11.

**20.** A method according to claim 18, which comprises controlling phytopathogenic fungi.

**21.** A process for the preparation of an agrochemical composition according to claim 13, which process comprises intimately mixing at least one compound of formula I according to claim 1 with suitable solid or liquid adjuvants and/or surfactants.

**Claims for the following Contracting State : ES**

1. A process for preparing a compound of the formula I

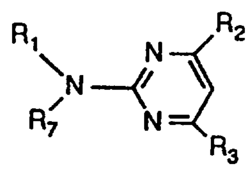

(I)

wherein: $R_1$ is phenyl or phenyl mono- to tri-substituted by $R_4$: $R_2$ is hydrogen, $C_1$-$C_5$alkyl, $C_1$-$C_5$alkyl substituted by the radical $OR_5$ or by the radical $SR_5$, $C_3$-$C_6$cycloalkyl, $C_3$-$C_6$cycloalkyl mono- to tri-substituted by $C_1$-$C_4$alkyl or by halogen, $C_2$-$C_5$alkenyl, $C_2$-$C_5$alkynyl or the formyl radical; $R_3$ is hydrogen, $C_1$-$C_4$alkyl, $C_1$-$C_4$alkyl substituted by halogen, cyano or by the radical $OR_5$ or by the radical $SR_5$, $C_3$-$C_6$cycloalkyl or $C_3$-$C_6$cycloalkyl mono- to tri-substituted by $C_1$-$C_4$alkyl or by halogen; $R_4$ is halogen, $C_1$-$C_3$alkyl, $C_1$-$C_2$haloalkyl, $C_1$-$C_3$alkoxy or $C_1$-$C_3$haloalkoxy; $R_5$ is hydrogen, $C_1$-$C_5$alkyl, $C_3$-$C_5$alkenyl, $C_3$-$C_5$alkynyl or the radical $(CH_2)_n$-X-$C_1$-$C_3$alkyl;
$R_7$ is the group $-NH_2$,

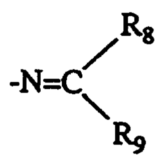 or 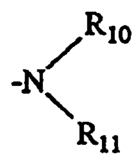 ;

$R_8$ is hydrogen, $C_1$-$C_3$alkyl or $C_1$-$C_3$haloalkyl; $R_9$ is hydrogen, $C_1$-$C_8$alkyl, $C_1$-$C_3$alkyl substituted by hydroxy, $OR_{12}$, $SR_{12}$ or by $N(R_{12})_2$, $C_3$-$C_6$cycloalkyl, cyclopropyl substituted by $SR_{12}$, $C_3$-$C_{10}$alkenyl, $C_1$-$C_3$haloalkyl, 1-, 2- or 3-pyridyl,

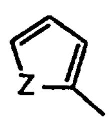 or 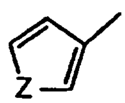 ;

$R_8$ and $R_9$, together with the carbon atom in the radical $R_7$, are a saturated or unsaturated ring comprising 4 to 7 carbon atoms; $R_{10}$ is $CH(R_8)R_9$, phenyl, $C_3$-$C_5$alkenyl, $C_3$-$C_5$alkynyl or cyanoalkyl having 2 or 3 carbon atoms in the alkyl radical; $R_{11}$ is hydrogen, $C_1$-$C_5$alkyl, $C_3$-$C_5$alkenyl, $C_3$-$C_5$alkynyl or cyanoalkyl having 2 or 3 carbon atoms in the alkyl radical; $R_{12}$ is $CH_3$ or $C_2H_5$; X is oxygen or sulfur; Z is O, S, NH or $NCH_3$; and n is 1 to 3; including the acid addition salts and metal salt complexes thereof, which comprises

a) reacting a pyrimidine derivative of the formula II

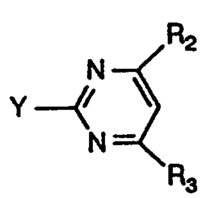

(II)

with a phenylhydrazine derivative of the formula III

$$R_1\text{-NH-NH-R} \qquad (III)$$

in the presence of a base, in an aprotic solvent and at temperatures of -50°C to 150°C, wherein Y is halogen, the radical $SO_2R_6$ or $N^{\oplus}(CH_3)_3$, $R_6$ is $C_1$-$C_4$alkyl, phenyl or phenyl substituted by methyl or by chlorine and R

is as defined for $R_{10}$ and $R_{11}$, and the latter and also $R_1$-$R_3$ are as defined under formula I,
or

b) reacting a pyrimidine hydrazine derivative of formula IV with an aldehyde or ketone of formula V to form a compound of formula VII with the removal of water

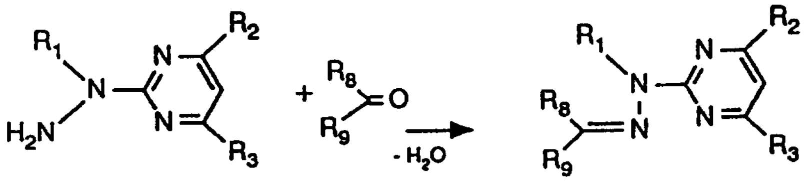


(IV) (V) (VII)

in any desired solvent, in the presence of an acid and at temperatures of -20° to 120°C, $R_1$-$R_3$ and $R_8$ and $R_9$ being as defined under formula I,
or

c) reducing a hydrazone derivative of formula VII

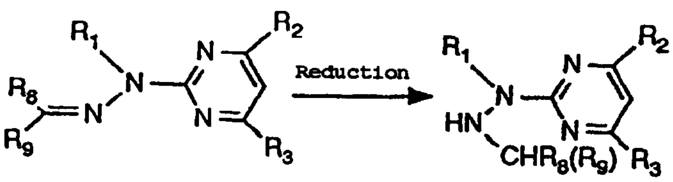


(VII) (VIII)

using a reducing agent, in an inert solvent and at temperatures of 0° to 50°C or by catalytic hydrogenation using catalysts,
or

d) subjecting a pyrimidine hydrazine derivative of formula IV

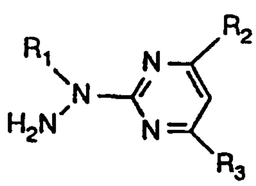

(IV)

to reductive alkylation with an aldehyde or ketone of formula V

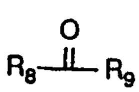

(V)

in the presence of a reducing agent, in an inert solvent and at temperatures of 0° to 50°C,
or

e) alkylating a pyrimidine hydrazine of formula IV or VIII with an alkyl halide $R_oHal$

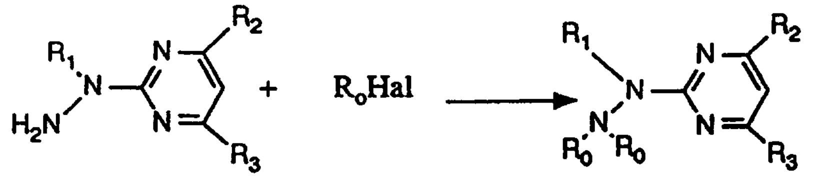


(IV) (IX)

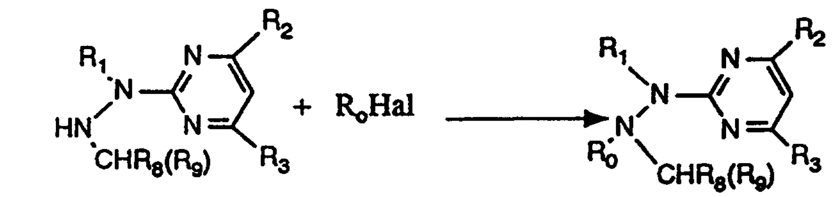


(VIII) (X)

in an inert solvent, in the presence of a base and at temperatures of 0°-60°C, $R_o$ being $C_1$-$C_4$alkyl and $R_1$-$R_9$ in processes (a-e) described above being as defined under formula I.

**2.** A process according to claim 1, which comprises preparing compounds wherein: $R_1$ is phenyl or phenyl mono- to tri-substituted by $R_4$; $R_2$ is hydrogen, $C_1$-$C_5$alkyl, $C_1$-$C_5$alkyl substituted by the radical $OR_5$ or by the radical $SR_5$, $C_3$-$C_6$cycloalkyl, $C_3$-$C_6$cycloalkyl mono- to tri-substituted by $C_1$-$C_4$alkyl or by halogen, $C_2$-$C_5$alkenyl, $C_2$-$C_5$alkynyl or the formyl radical; $R_3$ is $C_1$-$C_4$alkyl, $C_1$-$C_4$alkyl substituted by halogen, cyano or by the radical $OR_5$ or by the radical $SR_5$, $C_3$-$C_6$cycloalkyl or $C_3$-$C_6$cycloalkyl mono- to tri-substituted by $C_1$-$C_4$alkyl or by halogen; $R_4$ is halogen or $C_1$-$C_3$alkyl; $R_5$ is hydrogen, $C_1$-$C_5$alkyl, $C_3$-$C_5$alkenyl, $C_3$-$C_5$alkynyl or the radical $(CH_2)_n$-X-$C_1$-$C_3$alkyl; $R_9$ is hydrogen, $C_1$-$C_5$alkyl, $C_3$-$C_6$cycloalkyl, $C_5$-$C_5$alkenyl, $C_1$-$C_3$haloalkyl; $R_8$ and $R_9$, together with the carbon atom in the radical $R_7$ are a saturated or unsaturated ring comprising 5 or 6 carbon atoms; $R_7$, $R_{10}$, $R_{11}$, $R_{12}$ and Z are as defined under formula I; X is oxygen or sulfur; and n is 1 to 3.

**3.** A process according to claim 1, which comprises preparing compounds wherein: $R_1$ is phenyl or phenyl mono-substituted by halogen; $R_2$ is hydrogen, $C_1$-$C_5$alkyl, $C_1$-$C_5$alkyl substituted by $OR_5$, $C_3$-$C_6$cycloalkyl, $C_3$-$C_6$cycloalkyl mono- to tri-substituted by $C_1$-$C_4$alkyl or by halogen, $C_2$-$C_5$alkenyl, $C_2$-$C_5$alkynyl or the formyl radical; $R_3$ is $C_1$-$C_4$alkyl, $C_1$-$C_4$alkyl substituted by halogen, cyano or by $OR_5$, $C_3$-$C_6$cycloalkyl or $C_3$-$C_6$cycloalkyl substituted by methyl; $R_5$ is hydrogen or $C_1$-$C_2$alkyl; $R_9$ is hydrogen, $C_1$-$C_5$alkyl, $C_3$-$C_6$cycloalkyl, $C_3$-$C_5$alkenyl, $C_1$-$C_3$haloalkyl, $R_8$ and $R_9$, together with the carbon atom in the radical $R_7$, are a saturated or unsaturated ring comprising 5 or 6 carbon atoms; and $R_7$ $R_{10}$, $R_{11}$ and $R_{12}$ are as defined under formula I.

**4.** A process according to claim 1, which comprises preparing compounds wherein: $R_1$ is phenyl or phenyl mono- to tri-substituted by $R_4$; $R_2$ is hydrogen, $C_1$-$C_5$alkyl, $C_1$-$C_5$alkyl substituted by the radical $OR_5$ or by the radical $SR_5$, $C_3$-$C_6$cycloalkyl, $C_3$-$C_6$cycloalkyl mono- to tri-substituted by $C_1$-$C_4$alkyl or by halogen, $C_2$-$C_5$alkenyl, $C_2$-$C_5$alkynyl or the formyl radical; $R_3$ is $C_1$-$C_4$alkyl, $C_1$-$C_4$alkyl substituted by halogen, cyano or by the radical $OR_5$ or by the radical $SR_5$, $C_3$-$C_6$cycloalkyl or $C_3$-$C_6$cycloalkyl mono- to tri-substituted by $C_1$-$C_4$alkyl or by halogen; $R_4$ is halogen, $C_1$-$C_3$alkyl, $C_1$-$C_2$haloalkyl, $C_1$-$C_3$alkoxy or $C_1$-$C_3$haloalkoxy; $R_5$ is hydrogen, $C_1$-$C_5$alkyl, $C_3$-$C_5$alkenyl, $C_3$-$C_5$alkynyl or the radical $(CH_2)_n$-X-$C_1$-$C_3$alkyl; $R_7$ is $-NH_2$; X is oxygen or sulfur; and n is 1 to 3; including the acid addition salts and metal salt complexes thereof.

**5.** A process according to claim 4, which comprises preparing compounds wherein: $R_1$ is phenyl or phenyl mono- to tri-substituted by $R_4$; $R_2$ is hydrogen, $C_1$-$C_5$alkyl, $C_1$-$C_5$alkyl substituted by the radical $OR_5$ or by the radical $SR_5$, $C_3$-$C_6$cycloalkyl, $C_3$-$C_6$cycloalkyl mono- to tri-substituted by $C_1$-$C_4$alkyl or by halogen, $C_2$-$C_5$alkenyl, $C_2$-$C_5$alkynyl or the formyl radical; $R_3$ is $C_1$-$C_4$alkyl, $C_1$-$C_4$alkyl substituted by halogen, cyano or by the radical $OR_5$ or by the radical $SR_5$, $C_3$-$C_6$cycloalkyl or $C_3$-$C_6$cycloalkyl mono- to tri-substituted by $C_1$-$C_4$alkyl or by halogen; $R_4$ is halogen;

$R_5$ is hydrogen, $C_1$-$C_5$alkyl, $C_3$-$C_5$alkenyl, $C_3$-$C_5$alkynyl or the radical $(CH_2)_n$-X-$C_1$-$C_3$alkyl; X is oxygen or sulfur; and n is 1 to 3.

6. A process according to claim 1, which comprises preparing compounds wherein: $R_1$ is phenyl or phenyl mono- to tri-substituted by halogen; $R_2$ is hydrogen, $C_1$-$C_5$alkyl, $C_1$-$C_5$alkyl substituted by the radical $OR_5$ or by the radical $SR_5$, $C_3$-$C_6$cycloalkyl, $C_3$-$C_6$cycloalkyl mono- to tri-substituted by $C_1$-$C_4$alkyl or by halogen, $C_2$-$C_5$alkenyl, $C_2$-$C_5$alkynyl or the formyl radical; $R_3$ is $C_1$-$C_4$alkyl, $C_1$-$C_4$alkyl substituted by halogen, cyano or by the radical $OR_5$ or by the radical $SR_5$, $C_3$-$C_6$cycloalkyl or $C_3$-$C_6$cycloalkyl mono- to tri-substituted by $C_1$-$C_4$alkyl or by halogen; $R_5$ is hydrogen, $C_1$-$C_5$alkyl, $C_3$-$C_5$alkenyl, $C_3$-$C_5$alkynyl or the radical $(CH_2)_n$-X-$C_1$-$C_3$alkyl; X is oxygen or sulfur; and n is 1 to 3.

7. A process according to claim 1, which comprises preparing compounds wherein: $R_1$ is phenyl or phenyl mono-substituted by chlorine or by fluorine; $R_2$ is $C_1$-$C_5$alkyl, or is $C_1$-$C_2$alkyl substituted by $OR_5$, $C_3$-$C_6$cycloalkyl, $C_3$-$C_6$cycloalkyl mono- to tri-substituted by $C_1$-$C_4$alkyl or by halogen, $C_2$-$C_5$alkenyl, $C_2$-$C_5$alkynyl or the formyl radical; $R_3$ is $C_1$-$C_4$alkyl, $C_1$-$C_4$haloalkyl, $C_3$-$C_6$cycloalkyl or $C_3$-$C_6$cycloalkyl substituted by methyl; and $R_5$ is hydrogen or $C_1$-$C_2$alkyl.

8. A process according to claims 6 and 7, which comprises preparing compounds wherein $R_3$ is: methyl, fluoromethyl, chloromethyl, bromomethyl, $C_3$-$C_6$cycloalkyl or methoxymethyl.

9. A process according to claim 1, which comprises preparing a compound selected from:
N-(4-fluoromethyl-6-cyclopropylpyrimid-2-yl)-N-phenylhydrazine;
N-(4-methyl-6-cyclopropylpyrimid-2-yl)-N-m-fluorophenylhydrazine;
N-(4-methyl-6-cyclopropylpyrimid-2-yl)-N-p-fluorophenylhydrazine.

10. A process according to claim 5 which comprises preparing a compound selected from:
N-(4-methyl-6-cyclopropylpyrimid-2-yl)-N-phenylhydrazine; N-(4,6-dimethylpyrimid-2-yl)-N-phenylhydrazine; N-(4-methyl-6-methoxymethylpyrimid-2-yl)-N-phenylhydrazine.

11. A process according to claim 1 which comprises preparing a compound selected from:
N-(4,6-dimethylpyrimid-2-yl)-N-phenylpropionaldehyde hydrazone;
N-(4,6-dimethylpyrimid-2-yl)-N-phenylisobutyraldehyde hydrazone;
N-(4-methyl-6-methoxymethylpyrimid-2-yl)-N-phenylisobutyraldehyde hydrazone;
N-(4-methyl-6-methoxymethylpyrimid-2-yl)-N-phenylpropionaldehyde hydrazone;
N-(4-methyl-6-cyclopropylpyrimid-2-yl)-N-phenylpropionaldehyde hydrazone;
N-(4-methyl-6-cyclopropylpyrimid-2-yl)-N-phenyl-n-butyraldehyde hydrazone;
N-(4-methyl-6-cyclopropylpyrimid-2-yl)-N-phenylisobutyraldehyde hydrazone;
N-(4-methyl-6-cyclopropylpyrimid-2-yl)-N-phenyltrichloroacetaldehyde hydrazone;
N-(4-methyl-6-cyclopropylpyrimid-2-yl)-N-p-fluorophenylacetaldehyde hydrazone;
N-(4-methyl-6-cyclopropylpyrimid-2-yl)-N-p-fluorophenylisobutyraldehyde hydrazone;
N-(4-methyl-6-cyclopropylpyrimid-2-yl)-N-m-fluorophenylisobutyraldehyde hydrazone;
N-(4,6-dimethylpyrimid-2-yl)-N-phenyl-N'-methylhydrazine;
N-(4,6-dimethylpyrimid-2-yl)-N-phenyl-N'-dimethylhydrazine;
N-(4,6-dimethylpyrimid-2-yl)-N-phenyl-N'-n-propylhydrazine;
N-(4,6-dimethylpyrimid-2-yl)-N-phenyl-N'-isobutylhydrazine;
N-(4-methyl-6-methoxymethylpyrimid-2-yl)-N-phenyl-N'-methylhydrazine;
N-(4-methyl-6-methoxymethylpyrimid-2-yl)-N-phenyl-N'-n-propylhydrazine;
N-(4-methyl-6-methoxymethylpyrimid-2-yl)-N-phenyl-N'-dimethylhydrazine;
N-(4-methyl-6-cyclopropylpyrimid-2-yl)-N-phenyl-N'-methylhydrazine;
N-(4-methyl-6-cyclopropylpyrimid-2-yl)-N-phenyl-N'-isobutylhydrazine;
N-(4-methyl-6-cyclopropylpyrimid-2-yl)-N-phenyl-N'-dimethylhydrazine;
N-(4-methyl-6-cyclopropylpyrimid-2-yl)-N-phenyl-N'-diethylhydrazine;
N-(4-methyl-6-cyclopropylpyrimid-2-yl)-N-phenyl-N'-methyl-N'-ethylhydrazine;
N-(4-methyl-6-cyclopropylpyrimid-2-yl)-N-p-fluorophenyl-N'-ethylhydrazine;
N-(4-methyl-6-methoxymethylpyrimid-2-yl)-N-m-fluorophenyl-N'-isopropylhydrazine.

12. A composition for controlling or preventing attack by insect pests or destructive microorganisms, which composition contains as active ingredient, together with a suitable carrier, at least one compound of formula I

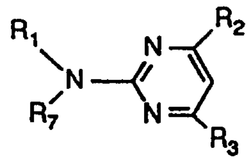 (I)

wherein: $R_1$ is phenyl or phenyl mono- to tri-substituted by $R_4$; $R_2$ is hydrogen, $C_1$-$C_5$alkyl, $C_1$-$C_5$alkyl substituted by the radical $OR_5$ or by the radical $SR_5$, $C_3$-$C_6$cycloalkyl, $C_3$-$C_6$cycloalkyl mono- to tri-substituted by $C_1$-$C_4$alkyl or by halogen, $C_2$-$C_5$alkenyl, $C_2$-$C_5$alkynyl or the formyl radical; $R_3$ is hydrogen, $C_1$-$C_4$alkyl, $C_1$-$C_4$alkyl substituted by halogen, cyano or by the radical $OR_5$ or by the radical $SR_5$, $C_3$-$C_6$cycloalkyl or $C_3$-$C_6$cycloalkyl mono- to tri-substituted by $C_1$-$C_4$alkyl or by halogen; $R_4$ is halogen, $C_1$-$C_3$alkyl, $C_1$-$C_2$haloalkyl, $C_1$-$C_3$alkoxy or $C_1$-$C_3$haloalkoxy; $R_5$ is hydrogen, $C_1$-$C_5$alkyl, $C_1$-$C_5$alkenyl, $C_1$-$C_5$alkynyl or the radical $(CH_2)_n$-X-$C_1$-$C_3$alkyl;
$R_7$ is the group $-NH_2$,

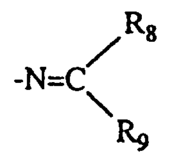 or 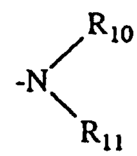 ;

$R_8$ is hydrogen, $C_1$-$C_3$alkyl or $C_1$-$C_3$haloalkyl; $R_9$ is hydrogen, $C_1$-$C_8$alkyl, $C_1$-$C_3$alkyl substituted by hydroxy, $OR_{12}$, $SR_{12}$ or by $N(R_{12})_2$, $C_3$-$C_6$cycloalkyl, cyclopropyl substituted by $SR_{12}$, $C_3$-$C_{10}$alkenyl, $C_1$-$C_3$haloalkyl, 1-, 2- or 3-pyridyl,

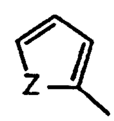 or 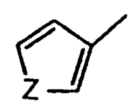 ;

$R_8$ and $R_9$, together with the carbon atom in the radical $R_7$, are a saturated or unsaturated ring comprising 4 to 7 carbon atoms; $R_{10}$ is $CH(R_8)R_9$, phenyl, $C_3$-$C_5$alkenyl, $C_3$-$C_5$alkynyl or cyanoalkyl having 2 or 3 carbon atoms in the alkyl radical; $R_{11}$ is hydrogen, $C_1$-$C_5$alkyl, $C_3$-$C_5$alkenyl, $C_3$-$C_5$alkynyl or cyanoalkyl having 2 or 3 carbon atoms in the alkyl radical; $R_{12}$ is $CH_3$ or $C_2H_5$; X is oxygen or sulfur; Z is O, S, NH or $NCH_3$; and n is 1 to 3.

**13.** A composition according to claim 12, which composition contains as active ingredient at least one compound of the formula I wherein $R_1$ is phenyl or phenyl mono- to tri-substituted by $R_4$; $R_2$ is hydrogen, $C_1$-$C_5$alkyl, $C_1$-$C_5$alkyl substituted by the radical $OR_5$ or by the radical $SR_5$, $C_3$-$C_6$cycloalkyl, $C_3$-$C_6$cycloalkyl mono- to tri-substituted by $C_1$-$C_4$alkyl or by halogen, $C_2$-$C_5$alkenyl, $C_2$-$C_5$alkynyl or the formyl radical; $R_3$ is $C_1$-$C_4$alkyl, $C_1$-$C_4$alkyl substituted by halogen, cyano or by the radical $OR_5$ or by the radical $SR_5$, $C_3$-$C_6$cycloalkyl or $C_3$-$C_6$cycloalkyl mono- to tri-substituted by $C_1$-$C_4$alkyl or by halogen; $R_4$ is halogen, $C_1$-$C_3$alkyl, $C_1$-$C_2$haloalkyl, $C_1$-$C_3$alkoxy or $C_1$-$C_3$haloalkoxy; $R_5$ is hydrogen, $C_1$-$C_5$alkyl, $C_3$-$C_5$alkenyl, $C_3$-$C_5$alkynyl or the radical $(CH_2)_n$-X-$C_1$-$C_3$alkyl; $R_7$ is $-NH_2$; X is oxygen or sulfur; and n is 1 to 3.

**14.** A composition according to claim 12, which contains as active ingredient at least one compound of formula I selected from
N-(4-fluoromethyl-6-cyclopropylpyrimid-2-yl)-N-phenylhydrazine;
N-(4-methyl-6-cyclopropylpyrimid-2-yl)-N-m-fluorophenylhydrazine;
N-(4-methyl-6-cyclopropylpyrimid-2-yl)-N-p-fluorophenylhydrazine.

**15.** A composition according to claim 12, which contains as active ingredient at least one compound of formula I selected from
N-(4-methyl-6-cyclopropylpyrimid-2-yl)-N-phenylhydrazine; N-(4,6-dimethylpyrimid-2-yl)-N-phenylhydrazine; N-(4-methyl-6-methoxymethylpyrimid-2-yl)-N-phenylhydrazine.

**16.** A composition according to claim 12, which contains as active ingredient at least one compound of formula I selected from
N-(4,6-dimethylpyrimid-2-yl)-N-phenylpropionaldehyde hydrazone;
N-(4,6-dimethylpyrimid-2-yl)-N-phenylisobutyraldehyde hydrazone;
N-(4-methyl-6-methoxymethylpyrimid-2-yl)-N-phenylisobutyraldehyde hydrazone;
N-(4-methyl-6-methoxymethylpyrimid-2-yl)-N-phenylpropionaldehyde hydrazone;
N-(4-methyl-6-cyclopropylpyrimid-2-yl)-N-phenylpropionaldehyde hydrazone;
N-(4-methyl-6-cyclopropylpyrimid-2-yl)-N-phenyl-n-butyraldehyde hydrazone;
N-(4-methyl-6-cyclopropylpyrimid-2-yl)-N-phenylisobutyraldehyde hydrazone;
N-(4-methyl-6-cyclopropylpyrimid-2-yl)-N-phenyltrichloroacetaldehyde hydrazone;
N-(4-methyl-6-cyclopropylpyrimid-2-yl)-N-p-fluorophenylacetaldehyde hydrazone;
N-(4-methyl-6-cyclopropylpyrimid-2-yl)-N-p-fluorophenylisobutyraldehyde hydrazone;
N-(4-methyl-6-cyclopropylpyrimid-2-yl)-N-m-fluorophenylisobutyraldehyde hydrazone;
N-(4,6-dimethylpyrimid-2-yl)-N-phenyl-N'-methylhydrazine;
N-(4,6-dimethylpyrimid-2-yl)-N-phenyl-N'-dimethylhydrazine;
N-(4,6-dimethylpyrimid-2-yl)-N-phenyl-N'-n-propylhydrazine;
N-(4,6-dimethylpyrimid-2-yl)-N-phenyl-N'-isobutylhydrazine;
N-(4-methyl-6-methoxymethylpyrimid-2-yl)-N-phenyl-N'-methylhydrazine;
N-(4-methyl-6-methoxymethylpyrimid-2-yl)-N-phenyl-N'-n-propylhydrazine;
N-(4-methyl-6-methoxymethylpyrimid-2-yl)-N-phenyl-N'-dimethylhydrazine;
N-(4-methyl-6-cyclopropylpyrimid-2-yl)-N-phenyl-N'-methylhydrazine;
N-(4-methyl-6-cyclopropylpyrimid-2-yl)-N-phenyl-N'-isobutylhydrazine;
N-(4-methyl-6-cyclopropylpyrimid-2-yl)-N-phenyl-N'-dimethylhydrazine;
N-(4-methyl-6-cyclopropylpyrimid-2-yl)-N-phenyl-N'-diethylhydrazine;
N-(4-methyl-6-cyclopropylpyrimid-2-yl)-N-phenyl-N'-methyl-N'-ethylhydrazine;
N-(4-methyl-6-cyclopropylpyrimid-2-yl)-N-p-fluorophenyl-N'-ethylhydrazine;
N-(4-methyl-6-methoxymethylpyrimid-2-yl)-N-m-fluorophenyl-N'-isopropylhydrazine.

**17.** A method of controlling or preventing attacks on cultivated plants by insect pests or phytopathogenic microorganisms, which method comprises applying to the plant, parts of the plant or the locus thereof, as active ingredient, a compound of formula I prepared according to claim 1.

**18.** A method according to claim 17, which comprises applying as active ingredient a compound prepared according to any one of claims 2 to 11.

**19.** A method according to claim 17, which comprises controlling phytopathogenic fungi.

**20.** A process for the preparation of an agrochemical composition according to claim 12, which process comprises intimately mixing at least one compound of formula I prepared according to claim 1 with suitable solid or liquid adjuvants and/or surfactants.

**Revendications**

**Revendications pour les Etats contractants suivants : AT, BE, CH, LI, DE, FR, GB, GR, IT, LU, NL, SE**

**1.** Composés de formule I

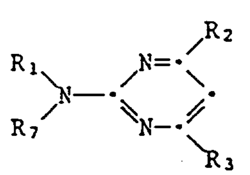

(I)

dans laquelle
$R_1$ représente un groupe phényle ou un groupe phényle portant jusqu'à trois substituants $R_4$ ;
$R_2$ représente l'hydrogène, un groupe alkyle en C1-C5, un groupe alkyle en C1-C5 substitué par $OR_5$ ou $SR_5$, un groupe cycloalkyle en C3-C6, un groupe cycloalkyle en C3-C6 portant jusqu'à trois substituants alkyle en C1-C4

ou halogéno, un groupe alcényle en C2-C5, alcynyle en C2-C5 ou formyle ;

$R_3$ représente l'hydrogène, un groupe alkyle en C1-C4, un groupe alkyle en C1-C4 substitué par des halogènes, des groupes cyano, ou les groupes $OR_5$ ou $SR_5$, un groupe cycloalkyle en C3-C6 ou un groupe cycloalkyle en C3-C6 portant jusqu'à trois substituants alkyle en C1-C4 ou halogéno ;

$R_4$ représente un halogène, un groupe alkyle en C1-C3, halogénoalkyle en C1-C2, alcoxy en C1-C3 ou halogénoalcoxy en C1-C3 ;

$R_5$ représente l'hydrogène, un groupe alkyle en C1-C5, alcényle en C3-C5, alcynyle en C3-C5 ou $(CH_2)_n$-X-alkyle en C1-C3 ;

$R_7$ représente un groupe $-NH_2$,

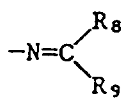 ou 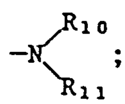 ;

$R_8$ représente l'hydrogène, un groupe alkyle en C1-C3 ou halogénoalkyle en C1-C3 ;

$R_9$ représente l'hydrogène, un groupe alkyle en C1-C8, un groupe alkyle en C1-C3 substitué par des groupes hydroxy, $OR_{12}$, $SR_{12}$ ou $N(R_{12})_2$, un groupe cycloalkyle en C3-C6, un groupe cyclopropyle substitué par $SR_{12}$, un groupe alcényle en C3-C10, halogénoalkyle en C1-C3, 1-, 2- ou 3-pyridyle,

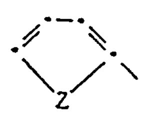 ou 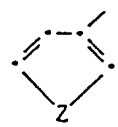 ;

$R_8$ et $R_9$ forment ensemble et avec l'atome de carbone de $R_7$ un cycle saturé ou insaturé consistant en 4 à 7 atomes de carbone ;

$R_{10}$ représente un groupe $CH(R_8)R_9$, un groupe phényle, alcényle en C3-C5, alcynyle en C3-C5 ou cyanalkyle contenant deux ou trois atomes de carbone dans la partie alkyle ;

$R_{11}$ représente l'hydrogène, un groupe alkyle en C1-C5, alcényle en C3-C5, alcynyle en C3-C5 ou cyanalkyle contenant deux ou trois atomes de carbone dans la partie alkyle ;

$R_{12}$ représente $CH_3$ ou $C_2H_5$ ;

X représente l'oxygène ou le soufre ;

Z représente O, S, NH ou $NH_3$ ; et

n est un nombre allant de 1 à 3 ;

y compris leurs sels formés par addition avec des acides et complexes de sels métalliques.

**2.** Composés de formule I selon revendication 1, dans laquelle :

$R_1$ représente un groupe phényle ou un groupe phényle portant jusqu'à trois substituants $R_4$ ;

$R_2$ représente l'hydrogène, un groupe alkyle en C1-C5, un groupe alkyle en C1-C5 substitué par $OR_5$ ou $SR_5$, un groupe cycloalkyle en C3-C6 ou un groupe cycloalkyle en C3-C6 portant jusqu'à trois substituants alkyle en C1-C5 ou halogéno, un groupe alcényle en C2-C5, alcynyle en C2-C5 ou formyle ;

$R_3$ représente un groupe alkyle en C1-C4, un groupe alkyle en C1-C4 substitué par des halogènes, des groupes cyano, ou les groupes $OR_5$ ou $SR_5$, un groupe cycloalkyle en C3-C6 ou cycloalkyle en C3-C6 portant jusqu'à trois substituants alkyle en C1-C4 ou halogéno ;

$R_4$ représente un halogène, un groupe alkyle en C1-C3 ;

$R_5$ représente l'hydrogène, un groupe alkyle en C1-C5, alcényle en C3-C5, alcynyle en C3-C5 ou $(CH_2)_n$-X-alkyle en C1-C3 ;

$R_9$ représente l'hydrogène, un groupe alkyle en C1-C5, cycloalkyle en C3-C6, alcényle en C3-C5, halogénoalkyle en C1-C3 ;

$R_8$ et $R_9$ forment ensemble et avec l'atome de carbone de $R_7$ un cycle saturé ou insaturé consistant en 5 ou 6 atomes de carbone ;

$R_7$, $R_{10}$, $R_{11}$, $R_{12}$ et Z ont les significations indiquées en référence à la formule I :

X représente l'oxygène ou le soufre :

n est un nombre allant de 1 à 3.

3. Composés de formule I selon revendication 1, dans laquelle :
$R_1$ représente un groupe phényle ou un groupe phényle monosubstitué par un halogène ;
$R_2$ représente l'hydrogène, un groupe alkyle en C1-C5, un groupe alkyle en C1-C5 substitué par $OR_5$, un groupe cycloalkyle en C3-C6, un groupe cycloalkyle en C3-C6 portant jusqu'à trois substituants alkyle en C1-C4 ou halogéno, un groupe alcényle en C2-C5, alcynyle en C2-C5 ou formyle ;
$R_3$ représente un groupe alkyle en C1-C4, un groupe alkyle en C1-C4 substitué par des halogènes, des groupes cyano ou $OR_5$, un groupe cycloalkyle en C3-C6 ou cycloalkyle en C3-C6 substitué par un groupe méthyle ;
$R_5$ représente l'hydrogène ou un groupe alkyle en C1-C2 ;
$R_9$ représente l'hydrogène, un groupe alkyle en C1-C5, cycloalkyle en C3-C6, alcényle en C3-C5, halogénoalkyle en C1-C3 ;
$R_8$ et $R_9$ forment ensemble et avec l'atome de carbone de $R_7$ un cycle saturé ou insaturé consistant en 5 ou 6 atomes de carbone ;
$R_7$, $R_{10}$, $R_{11}$ et $R_{12}$ ont les significations indiquées en référence à la formule I.

4. Composés de formule I de la revendication 1, dans laquelle :
$R_1$ représente un groupe phényle ou un groupe phényle portant jusqu'à trois substituants $R_4$ ;
$R_2$ représente l'hydrogène, un groupe alkyle en C1-C5, un groupe alkyle en C1-C5 substitué par $OR_5$ ou $SR_5$, un groupe cycloalkyle en C3-C6, un groupe cycloalkyle en C3-C6 portant jusqu'à trois substituants alkyle en C1-C4 ou halogéno, un groupe alcényle en C2-C5, alcynyle en C2-C5 ou formyle ;
$R_3$ représente un groupe alkyle en C1-C4, alkyle en C1-C4 substitué par des halogènes, des groupes cyano ou les groupes $OR_5$ ou $SR_5$, un groupe cycloalkyle en C3-C6 ou un groupe cycloalkyle en C3-C6 portant jusqu'à trois substituants alkyle en C1-C4 ou halogéno ;
$R_4$ représente un halogène, un groupe alkyle en C1-C3, halogénoalkyle en C1-C2, alcoxy en C1-C3 ou halogénoalcoxy en C1-C3 ;
$R_5$ représente l'hydrogène, un groupe alkyle en C1-C5, alcényle en C3-C5, alcynyle en C3-C5 ou $(CH_2)_n$-X-alkyle en C1-C3 ;
$R_7$ représente $-NH_2$ ;
X représente l'oxygène ou le soufre ;
n est un nombre allant de 1 à 3 ;
y compris leurs sels formés par addition avec des acides et complexes de sels métalliques.

5. Composés de formule I selon revendication 4, dans laquelle :
$R_1$ représente un groupe phényle ou un groupe phényle portant jusqu'à trois substituants $R_4$ ;
$R_2$ représente l'hydrogène, un groupe alkyle en C1-C5, alkyle en C1-C5 substitué par $OR_5$ ou $SR_5$, cycloalkyle en C3-C6, cycloalkyle en C3-C6 portant jusqu'à trois substituants alkyle en C1-C4 ou halogéno, alcényle en C2-C5, alcynyle en C2-C5 ou formyle ;
$R_3$ représente un groupe alkyle en C1-C4, alkyle en C1-C4 substitué par des halogènes, des groupes cyano ou les groupes $OR_5$ ou $SR_5$, cycloalkyle en C3-C6 ou cycloalkyle en C3-C6 portant jusqu'à trois substituants alkyle en C1-C4 ou halogéno ;
$R_4$ représente un halogène ;
$R_5$ représente l'hydrogène, un groupe alkyle en C1-C5, alcényle en C3-C5, alcynyle en C3-C5 ou $(CH_2)_n$-X-alkyle en C1-C3 ;
X représente l'oxygène ou le soufre ;
n est un nombre allant de 1 à 3.

6. Composés de formule I selon revendication 1, dans laquelle :
$R_1$ représente un groupe phényle ou phényle portant jusqu'à trois substituants halogéno ;
$R_2$ représente l'hydrogène, un groupe alkyle en C1-C5, un groupe alkyle en C1-C5 substitué par $OR_5$ ou $SR_5$, un groupe cycloalkyle en C3-C6 ou cycloalkyle en C3-C6 portant jusqu'à trois substituants alkyle en C1-C4 ou halogéno, alcényle en C2-C5, alcynyle en C2-C5 ou formyle; $R_3$ représente un groupe alkyle en C1-C4, alkyle en C1-C4 substitué par des halogènes, des groupes cyano ou les groupes $OR_5$ ou $SR_5$, cycloalkyle en C3-C6 ou cycloalkyle en C3-C6 portant jusqu'à trois substituants alkyle en C1-C4 ou halogéno ;
$R_5$ représente l'hydrogène, un groupe alkyle en C1-C5, alcényle en C3-C5, alcynyle en C3-C5 ou $(CH_2)_n$-X-alkyle en C1-C3 ;
X représente l'oxygène ou le soufre ;
n est un nombre allant de 1 à 3.

7. Composés de formule I selon revendication 1, dans laquelle :
$R_1$ représente un groupe phényle ou phényle portant un substituant chloro ou fluoro ;

$R_2$ représente un groupe alkyle en C1-C5, un groupe alkyle en C1-C2 substitué par $OR_5$, un groupe cycloalkyle en C3-C6 ou cycloalkyle en C3-C6 portant jusqu'à trois substituants alkyle en C1-C4 ou halogéno, un groupe alcényle en C2-C5, alcynyle en C2-C5 ou formyle ;
$R_3$ représente un groupe alkyle en C1-C4, halogénoalkyle en C1-C4, cycloalkyle en C3-C6 ou cycloalkyle en C3-C6 portant un substituant méthyle ;
$R_5$ représente l'hydrogène ou un groupe alkyle en C1-C2.

**8.** Composés de formule I selon les revendications 6 et 7 dans laquelle $R_3$ représente un groupe méthyle, fluorométhyle, chlorométhyle, bromométhyle, cycloalkyle en C3-C6 ou méthoxyméthyle.

**9.** Un composé de formule I selon revendication 1, pris dans le groupe des composés suivants :
N-(4-fluorométhyl-6-cyclopropylpyrimidyl-2)-N-phénylhydrazine ;
N-(4-méthyl-6-cyclopropylpyrimidyl-2)-N-m-fluorophénylhydrazine ;
N-(4-méthyl-6-cyclopropylpyrimidyl-2)-N-p-fluorophénylhydrazine.

**10.** Un composé de formule I selon revendication 5, pris dans le groupe suivant :
N-(4-méthyl-6-cyclopropylpyrimidyl-2)-N-phénylhydrazine;
N-(4,6-diméthylpyrimidyl-2)-N-phénylhydrazine;
N-(4-méthyl-6-méthoxyméthylpyrimidyl-2)-N-phénylhydrazine.

**11.** Un composé de formule I selon revendication 1, pris dans le groupe suivant :
N-(4,6-diméthylpyrimidyl-2)-N-phényl-propionaldéhydhydrazone ;
N-(4,6-diméthylpyrimidyl-2)-N-phényl-isobutyraldéhydhydrazone ;
N-(4-méthyl-6-méthoxyméthylpyrimidyl-2)-N-phényl-isobutyraldéhydhydrazone ;
N-(4-méthyl-6-méthoxyméthylpyrimidyl-2)-N-phényl-propionaldéhydhydrazone ;
N-(4-méthyl-6-cyclopropylpyrimidyl-2)-N-phényl-propionaldéhydhydrazone ;
N-(4-méthyl-6-cyclopropylpyrimidyl-2)-N-phényl-n-butyraldéhydhydrazone ;
N-(4-méthyl-6-cyclopropylpyrimidyl-2)-N-phényl-isobutyraldéhydhydrazone ;
N-(4-méthyl-6-cyclopropylpyrimidyl-2)-N-phényl-trichloracétaldéhydhydrazone ;
N-(4-méthyl-6-cyclopropylpyrimidyl-2)-N-p-fluorophényl-acétaldéhydhydrazone ;
N-(4-méthyl-6-cyclopropylpyrimidyl-2)-N-p-fluorophényl-isobutyraldéhydhydrazone ;
N-(4-méthyl-6-cyclopropylpyrimidyl-2)-N-m-fluorophényl-isobutyraldéhydhydrazone ;
N-(4,6-diméthylpyrimidyl-2)-N-phényl-N'-méthylhydrazine;
N-(4,6-diméthylpyrimidyl-2)-N-phényl-N'-diméthylhydrazine;
N-(4,6-diméthylpyrimidyl-2)-N-phényl-N'-n-propylhydrazine;
N-(4,6-diméthylpyrimidyl-2)-N-phényl-N'-isobutylhydrazine;
N-(4-méthyl-6-méthoxyméthylpyrimidyl-2)-N-phényl-N'-méthylhydrazine ;
N-(4-méthyl-6-méthoxyméthylpyrimidyl-2)-N-phényl-N'-n-propylhydrazine ;
N-(4-méthyl-6-méthoxyméthylpyrimidyl-2)-N-phényl-N'-diméthylhydrazine ;
N-(4-méthyl-6-cyclopropylpyrimidyl-2)-N-phényl-N'-méthylhydrazine ;
N-(4-méthyl-6-cyclopropylpyrimidyl-2)-N-phényl-N'-isobutylhydrazine ;
N-(4-méthyl-6-cyclopropylpyrimidyl-2)-N-phényl-N'-diméthylhydrazine ;
N-(4-méthyl-6-cyclopropylpyrimidyl-2)-N-phényl-N'-diéthylhydrazine ;
N-(4-méthyl-6-cyclopropylpyrimidyl-2)-N-phényl-N'-méthyl-N'-éthylhydrazine ;
N-(4-méthyl-6-cyclopropylpyrimidyl-2)-N-p-fluorophényl-N'-éthylhydrazine ;
N-(4-méthyl-6-méthoxyméthylpyrimidyl-2)-N-m-fluorophényl-N'-isopropylhydrazine.

**12.** Procédé de préparation d'un composé de formule I selon revendication 1, caractérisé en ce que

**a)** On fait réagir un dérivé de la pyrimidine répondant à la formule II

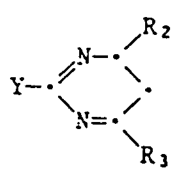

(II)

avec un dérivé de la phénylhydrazine répondant à la formule III

$R_1$-NH-NH-R (III)

en présence d'une base, dans un solvant aprotonique, à des températures de -50 à 150°C ; Y représentant un halogène, le groupe $SO_2R_6$ ou le groupe $N^{\oplus}(CH_3)_3$, $R_6$ un groupe alkyle en C1-C4, phényle ou phényle substitué par un groupe méthyle ou le chlore, et R ayant les significations de $R_{10}$ et $R_{11}$, ces derniers de même que $R_1$ à $R_3$ ayant les significations indiquées en référence à la formule I,
ou bien
**b)** on fait réagir un dérivé de pyrimidinhydrazine de formule IV avec un aldéhyde ou une cétone de formule V, ce qui donne, avec scission d'eau, des composés de formule VII

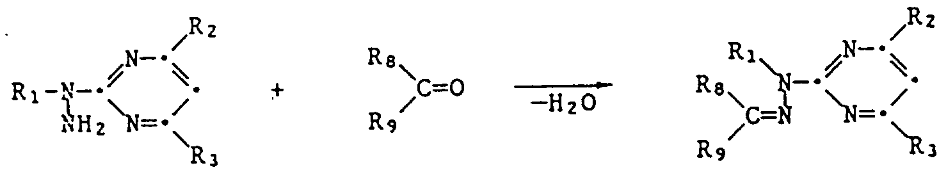


(IV) (V) (VII)

dans un solvant quelconque, en présence d'un acide, à des températures de -20 à 120°C, $R_1$ à $R_3$ et $R_8$ et $R_9$ ayant les significations indiquées en référence à la formule I, ou bien
**c)** on réduit un dérivé d'hydrazone de formule VII

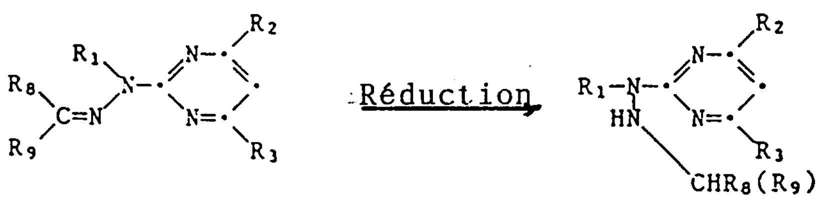


(VII) (VIII)

à l'aide d'un agent réducteur dans un solvant inerte, à des températures de 0 à 50°C ou bien par hydrogénation catalytique sur des catalyseurs,
ou bien
**d)** on soumet un dérivé de pyrimidinhydrazine de formule IV

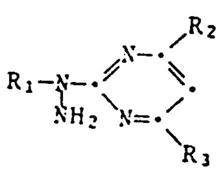

(IV)

à alkylation réductive à l'aide d'un aldéhyde ou une cétone de formule V

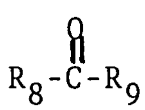

(V)

en présence d'un agent réducteur, dans un solvant inerte, à des températures de 0 à 50°C,
ou bien

**e)** on alkyle une pyrimidinhydrazine de formule IV ou VIII à l'aide d'un halogénure d'alkyle $R_oHal$

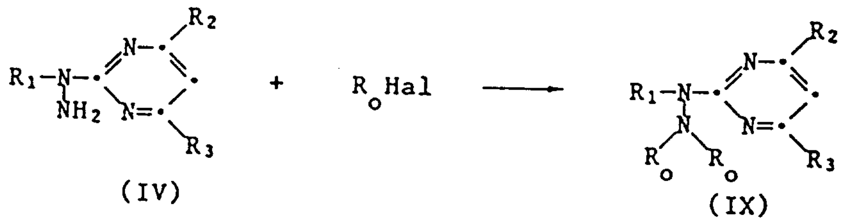


(IV) (IX)

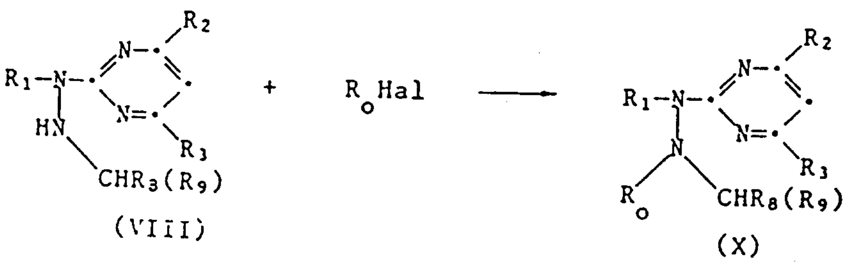


(VIII) (X)

dans un solvant inerte, en présence d'une base, à des températures de 0 à 60°C, $R_o$ représentant un groupe alkyle en C1-C4 et $R_1$ à $R_9$ ayant dans les procédés a) à e) décrits ci-dessus les significations indiquées en référence à la formule I.

**13.** Produit pour combattre ou prévenir une attaque par des insectes ou des microorganismes nuisibles, caractérisé en ce qu'il contient en tant que composant actif au moins un composé de formule I selon revendication 1, avec un véhicule approprié.

**14.** Produit selon revendication 13, caractérisé en ce qu'il contient en tant que composant actif, au moins un composé de formule I selon revendication 4.

**15.** Produit selon revendication 13, caractérisé en ce qu'il contient en tant que composant actif, au moins un composé de formule I selon revendication 9.

**16.** Produit selon revendication 13, caractérisé en ce qu'il contient, en tant que composant actif, au moins un composé de formule I selon revendication 10.

**17.** Produit selon revendication 13, caractérisé en ce qu'il contient, en tant que composant actif, au moins un composé de formule I selon revendication 11.

**18.** Procédé pour combattre ou prévenir une attaque de végétaux cultivés par des insectes nuisibles ou des microorganismes phytopathogènes, caractérisé en ce que l'on applique sur les plantes, des parties des plantes ou leur lieu de plantation, en tant que substance active, un composé de formule I selon revendication 1.

**19.** Procédé selon revendication 18, caractérisé en ce que l'on applique, en tant que substance active, un composé selon une des revendications 2 à 11.

**20.** Procédé selon revendication 18, caractérisé en ce que l'on combat des mycètes phytopathogènes.

**21.** Procédé pour préparer un produit agrochimique selon revendication 13, caractérisé en ce que l'on mélange intimement au moins un composé de formule I selon revendication 1, avec des additifs et/ou agents tensio-actifs solides ou liquides appropriés.

**Revendications pour l'Etat contractant suivant : ES**

1. Procédé de préparation d'un composé de formule I

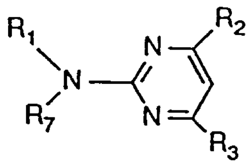

(I)

dans laquelle
$R_1$ représente un groupe phényle ou un groupe phényle portant jusqu'à trois substituants $R_4$ ;
$R_2$ représente l'hydrogène, un groupe alkyle en C1-C5, un groupe alkyle en C1-C5 substitué par $OR_5$ ou $SR_5$, un groupe cycloalkyle en C3-C6, un groupe cycloalkyle en C3-C6 portant jusqu'à trois substituants alkyle en C1-C4 ou halogéno, un groupe alcényle en C2-C5, alcynyle en C2-C5 ou formyle ;
$R_3$ représente l'hydrogène, un groupe alkyle en 1-C4, un groupe alkyle en C1-C4 substitué par des halogènes, des groupes cyano, ou les groupes $OR_5$ ou $SR_5$, un groupe cycloalkyle en C3-C6 ou un groupe cycloalkyle en C3-C6 portant jusqu'à trois substituants alkyle en C1-C4 ou halogéno ;
$R_4$ représente un halogène, un groupe alkyle en C1-C3, halogénoalkyle en C1-C2, alcoxy en C1-C3 ou halogénoalcoxy en C1-C3 ;
$R_5$ représente l'hydrogène, un groupe alkyle en C1-C5, alcényle en C3-C5, alcynyle en C3-C5 ou $(CH_2)_n$-X-alkyle en C1-C3 ;
$R_7$ représente un groupe $-NH_2$,

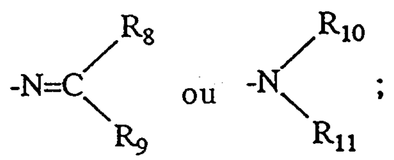

$R_8$ représente l'hydrogène, un groupe alkyle en C1-C3 ou halogénoalkyle en C1-C3 ;
$R_9$ représente l'hydrogène, un groupe alkyle en C1-C8, un groupe alkyle en C1-C3 substitué par des groupes hydroxy, $OR_{12}$, $SR_{12}$ ou $N(R_{12})_2$, un groupe cycloalkyle en C3-C6, un groupe cyclopropyle substitué par $SR_{12}$, un groupe alcényle en C3-C10, halogénoalkyle en C1-C3, 1-, 2- ou 3-pyridyle,

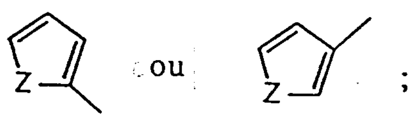

$R_8$ et $R_9$ forment ensemble et avec l'atome de carbone de $R_7$ un cycle saturé ou insaturé consistant en 4 à 7 atomes de carbone ;
$R_{10}$ représente un groupe $CH(R_8)R_9$, un groupe phényle, alcényle en C3-C5, alcynyle en C3-C5 ou cyanalkyle contenant deux ou trois atomes de carbone dans la partie alkyle ;
$R_{11}$ représente l'hydrogène, un groupe alkyle en C1-C5, alcényle en C3-C5, alcynyle en C3-C5 ou cyanalkyle contenant deux ou trois atomes de carbone dans la partie alkyle ;
$R_{12}$ représente $CH_3$ ou $C_2H_5$ ;
X représente l'oxygène ou le soufre ;
Z représente O, S, NH ou $NH_3$ ; et
n est un nombre allant de 1 à 3 ;
y compris leurs sels formés par addition avec des acides et complexes de sels métalliques, caractérisé en ce que

**a)** on fait réagir un dérivé de la pyrimidine répondant à la formule II

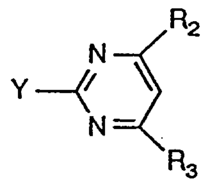

(II)

avec un dérivé de la phénylhydrazine répondant à la formule III

$$R_1\text{-NH-NH-R} \qquad \text{(III)}$$

en présence d'une base, dans un solvant aprotonique, à des températures de -50 à 150°C ; Y représentant un halogène, le groupe $SO_2R_6$ ou le groupe $N^{\oplus}(CH_3)_3$, $R_6$ un groupe alkyle en C1-C4, phényle ou phényle substitué par un groupe méthyle ou le chlore, et R ayant les significations de $R_{10}$ et $R_{11}$, ces derniers de même que $R_1$ à $R_3$ ayant les significations indiquées en référence à la formule I,
ou bien
**b)** on fait réagir un dérivé de pyrimidinhydrazine de formule IV avec un aldéhyde ou une cétone de formule V, ce qui donne, avec scission d'eau, des composés de formule VI

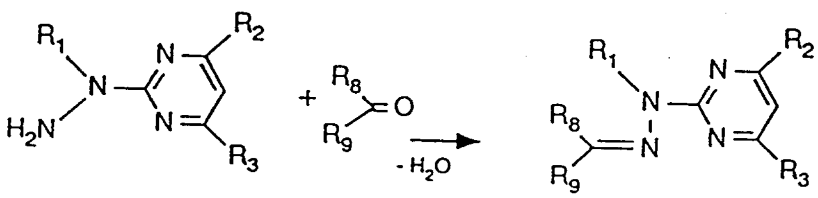


(IV) (V) (VII)

dans un solvant quelconque, en présence d'un acide, à des températures de -20 à 120°C, $R_1$ à $R_3$ et $R_8$ et $R_9$ ayant les significations indiquées en référence à la formule I, ou bien
**c)** on réduit un dérivé d'hydrazone de formule VII

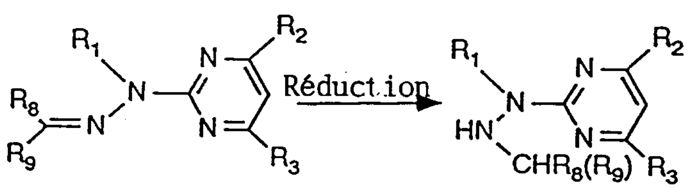


(VII) (VIII)

à l'aide d'un agent réducteur dans un solvant inerte, à des températures de 0 à 50°C ou bien par hydrogénation catalytique sur des catalyseurs,
ou bien

**d)** on soumet un dérivé de pyrimidinhydrazine de formule IV

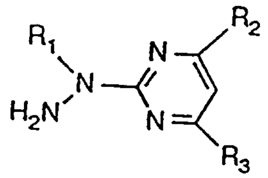

(IV)

à alkylation réductive à l'aide d'un aldéhyde ou une cétone de formule V

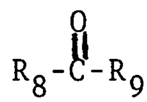

(V)

en présence d'un agent réducteur, dans un solvant inerte, à des températures de 0 à 50°C, ou bien

**e)** on alkyle une pyrimidinhydrazine de formule IV ou VIII à l'aide d'un halogénure d'alkyle $R_oHal$

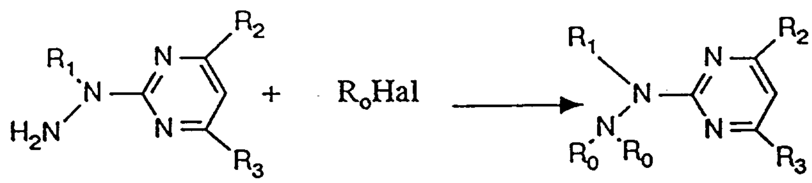


(IV) (IX)

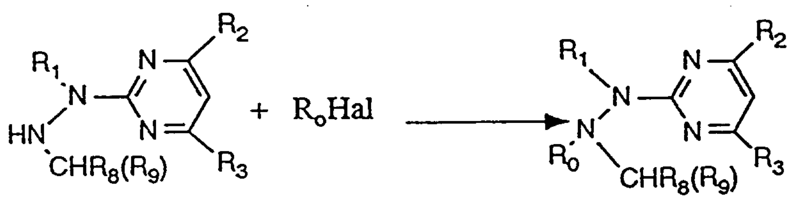


(VIII) (X)

dans un solvant inerte, en présence d'une base, à des températures de 0 à 60°C, $R_o$ représentant un groupe alkyle en C1-C4 et $R_1$ à $R_9$ ayant dans les procédés a) à

e) décrits ci-dessus les significations indiquées en référence à la formule I.

**2.** Procédé selon revendication 2, caractérisé en ce que l'on prépare des composés pour lesquels $R_1$ représente un groupe phényle ou un groupe phényle portant jusqu'à trois substituants $R_4$ ;

$R_2$ représente l'hydrogène, un groupe alkyle en C1-C5, un groupe alkyle en C1-C5 substitué par $OR_5$ ou $SR_5$, un groupe cycloalkyle en C3-C6 ou un groupe cycloalkyle en C3-C6 portant jusqu'à trois substituants alkyle en C1-C5 ou halogéno, un groupe alcényle en C2-C5, alcynyle en C2-C5 ou formyle ;

$R_3$ représente un groupe alkyle en C1-C4, un groupe alkyle en C1-C4 substitué par des halogènes, des groupes cyano, ou les groupes $OR_5$ ou $SR_5$, un groupe cycloalkyle en C3-C6 ou cycloalkyle en C3-C6 portant jusqu'à trois substituants alkyle en C1-C4 ou halogéno ;

$R_4$ représente un halogène, un groupe alkyle en C1-C3 ;

$R_5$ représente l'hydrogène, un groupe alkyle en C1-C5, alcényle en C3-C5, alcynyle en C3-C5 ou $(CH_2)_n$-X-alkyle en C1-C3 ;

$R_9$ représente l'hydrogène, un groupe alkyle en C1-C5, cycloalkyle en C3-C6, alcényle en C3-C5, halogénoalkyle en C1-C3 ;
$R_8$ et $R_9$ forment ensemble et avec l'atome de carbone de $R_7$ un cycle saturé ou insaturé consistant en 5 ou 6 atomes de carbone ;
$R_7$, $R_{10}$, $R_{11}$, $R_{12}$ et Z ont les significations indiquées en référence à la formule I ;
X représente l'oxygène ou le soufre ;
n est un nombre allant de 1 à 3.

**3.** Procédé selon revendication 1, caractérisé en ce que l'on prépare des composés pour lesquels :
$R_1$ représente un groupe phényle ou un groupe phényle monosubstitué par un halogène ;
$R_2$ représente l'hydrogène, un groupe alkyle en C1-C5, un groupe alkyle en C1-C5 substitué par $OR_5$, un groupe cycloalkyle en C3-C6, un groupe cycloalkyle en C3-C6 portant jusqu'à trois substituants alkyle en C1-C4 ou halogéno, un groupe alcényle en C2-C5, alcynyle en C2-C5 ou formyle ;
$R_3$ représente un groupe alkyle en C1-C4, un groupe alkyle en C1-C4 substitué par des halogènes, des groupes cyano ou $OR_5$, un groupe cycloalkyle en C3-C6 ou cycloalkyle en C3-C6 substitué par un groupe méthyle ;
$R_5$ représente l'hydrogène ou un groupe alkyle en C1-C2 ;
$R_9$ représente l'hydrogène, un groupe alkyle en C1-C5, cycloalkyle en C3-C6, alcényle en C3-C5, halogénoalkyle en C1-C3 ;
$R_8$ et $R_9$ forment ensemble et avec l'atome de carbone de $R_7$ un cycle saturé ou insaturé consistant en 5 ou 6 atomes de carbone ;
$R_7$, $R_{10}$, $R_{11}$ et $R_{12}$ ont les significations indiquées en référence à la formule I.

**4.** Procédé selon revendication 1, caractérisé en ce que l'on prépare des composés pour lesquels :
$R_1$ représente un groupe phényle ou un groupe phényle portant jusqu'à trois substituants $R_4$ ;
$R_2$ représente l'hydrogène, un groupe alkyle en C1-C5, un groupe alkyle en C1-C5 substitué par $OR_5$ ou $SR_5$, un groupe cycloalkyle en C3-C6, un groupe cycloalkyle en C3-C6 portant jusqu'à trois substituants alkyle en C1-C4 ou halogéno, un groupe alcényle en C2-C5, alcynyle en C2-C5 ou formyle ;
$R_3$ représente un groupe alkyle en 1-C4, alkyle en C1-C4 substitué par des halogènes, des groupes cyano ou les groupes $OR_5$ ou $SR_5$, un groupe cycloalkyle en C3-C6 ou un groupe cycloalkyle en C3-C6 portant jusqu'à trois substituants alkyle en C1-C4 ou halogéno ;
$R_4$ représente un halogène, un groupe alkyle en C1-C3, halogénoalkyle en C1-C2, alcoxy en C1-C3 ou halogénoalcoxy en C1-C3 ;
$R_5$ représente l'hydrogène, un groupe alkyle en C1-C5, alcényle en C3-C5, alcynyle en C3-C5 ou $(CH_2)_n$-X-alkyle en C1-C3 ;
$R_7$ représente $-NH_2$ ;
X représente l'oxygène ou le soufre ;
n est un nombre allant de 1 à 3 ;
y compris leurs sels formés par addition avec des acides et complexes de sels métalliques.

**5.** Procédé selon revendication 4, caractérisé en ce que l'on prépare des composés pour lesquels
$R_1$ représente un groupe phényle ou un groupe phényle portant jusqu'à trois substituants $R_4$ ;
$R_2$ représente l'hydrogène, un groupe alkyle en C1-C5, alkyle en C1-C5 substitué par $OR_5$ ou $SR_5$, cycloalkyle en C3-C6, cycloalkyle en C3-C6 portant jusqu'à trois substituants alkyle en C1-C4 ou halogéno, alcényle en C2-C5, alcynyle en C2-C5 ou formyle ;
$R_3$ représente un groupe alkyle en C1-C4, alkyle en C1-C4 substitué par des halogènes, des groupes cyano ou les groupes $OR_5$ ou $SR_5$, cycloalkyle en C3-C6 ou cycloalkyle en C3-C6 portant jusqu'à trois substituants alkyle en C1-C4 ou halogéno ;
$R_4$ représente un halogène ;
$R_5$ représente l'hydrogène, un groupe alkyle en C1-C5, alcényle en C3-C5, alcynyle en C3-C5 ou $(CH_2)_n$-X-alkyle en C1-C3 ;
X représente l'oxygène ou le soufre ;
n est un nombre allant de 1 à 3.

**6.** Procédé selon revendication 1, caractérisé en ce que l'on prépare des composés pour lesquels
$R_1$ représente un groupe phényle ou phényle portant jusqu'à trois substituants halogéno ;
$R_2$ représente l'hydrogène, un groupe alkyle en C1-C5, un groupe alkyle en C1-C5 substitué par $OR_5$ ou $SR_5$, un groupe cycloalkyle en C3-C6 ou cycloalkyle en C3-C6 portant jusqu'à trois substituants alkyle en C1-C4 ou halogéno, alcényle en C2-C5, alcynyle en C2-C5 ou formyle;
$R_3$ représente un groupe alkyle en C1-C4, alkyle en C1-C4 substitué par des halogènes, des groupes cyano ou les

groupes $OR_5$ ou $SR_5$, cycloalkyle en C3-C6 ou cycloalkyle en C3-C6 portant jusqu'à trois substituants alkyle en C1-C4 ou halogéno ;
$R_5$ représente l'hydrogène, un groupe alkyle en C1-C5, alcényle en C3-C5, alcynyle en C3-C5 ou $(CH_2)_n$-X-alkyle en C1-C3 ;
X représente l'oxygène ou le soufre ;
n est un nombre allant de 1 à 3.

**7.** Procédé selon revendication 1, caractérisé en ce que l'on prépare des composés pour lesquels
$R_1$ représente un groupe phényle ou phényle portant un substituant chloro ou fluoro ;
$R_2$ représente un groupe alkyle en C1-C5, un groupe alkyle en C1-C2 substitué par $OR_5$, un groupe cycloalkyle en C3-C6 ou cycloalkyle en C3-C6 portant jusqu'à trois substituants alkyle en C1-C4 ou halogéno, un groupe alcényle en C2-C5, alcynyle en C2-C5 ou formyle ;
$R_3$ représente un groupe alkyle en C1-C4, halogénoalkyle en C1-C4, cycloalkyle en C3-C6 ou cycloalkyle en C3-C6 portant un substituant méthyle ;
$R_5$ représente l'hydrogène ou un groupe alkyle en C1-C2.

**8.** Procédé selon les revendications 6 et 7, caractérisé en ce que l'on prépare des composés pour lesquels $R_3$ représente un groupe méthyle, fluorométhyle, chlorométhyle, bromométhyle, cycloalkyle en C3-C6 ou méthoxyméthyle.

**9.** Procédé selon revendication 1, caractérisé en ce que l'on prépare un composé choisi parmi les suivants :
N-(4-fluorométhyl-6-cyclopropylpyrimidyl-2)-N-phénylhydrazine :
N-(4-méthyl-6-cyclopropylpyrimidyl-2)-N-m-fluorophénylhydrazine ;
N-(4-méthyl-6-cyclopropylpyrimidyl-2)-N-p-fluorophénylhydrazine.

**10.** Procédé selon revendication 5, caractérisé en ce que l'on prépare un composé choisi parmi les suivants :
N-(4-méthyl-6-cyclopropylpyrimidyl-2)-N-phénylhydrazine;
N-(4,6-diméthylpyrimidyl-2)-N-phénylhydrazine;
N-(4-méthyl-6-méthoxyméthylpyrimidyl-2)-N-phénylhydrazine.

**11.** Procédé selon revendication 1, caractérisé en ce que l'on prépare un composé choisi parmi les suivants :
N-(4,6-diméthylpyrimidyl-2)-N-phényl-propionaldéhydhydrazone ;
N-(4,6-diméthylpyrimidyl-2)-N-phényl-isobutyraldéhydhydrazone ;
N-(4-méthyl-6-méthoxyméthylpyrimidyl-2)-N-phényl-isobutyraldéhydhydrazone ;
N-(4-méthyl-6-méthoxyméthylpyrimidyl-2)-N-phényl-propionaldéhydhydrazone ;
N-(4-méthyl-6-cyclopropylpyrimidyl-2)-N-phényl-propionaldéhydhydrazone ;
N-(4-méthyl-6-cyclopropylpyrimidyl-2)-N-phényl-n-butyraldéhydhydrazone ;
N-(4-méthyl-6-cyclopropylpyrimidyl-2)-N-phényl-isobutyraldéhydhydrazone ;
N-(4-méthyl-6-cyclopropylpyrimidyl-2)-N-phényl-trichloracétaldéhydhydrazone ;
N-(4-méthyl-6-cyclopropylpyrimidyl-2)-N-p-fluorophényl-acétaldéhydhydrazone ;
N-(4-méthyl-6-cyclopropylpyrimidyl-2)-N-p-fluorophényl-isobutyraldéhydhydrazone ;
N-(4-méthyl-6-cyclopropylpyrimidyl-2)-N-m-fluorophényl-isobutyraldéhydhydrazone ;
N-(4,6-diméthylpyrimidyl-2)-N-phényl-N'-méthylhydrazine;
N-(4,6-diméthylpyrimidyl-2)-N-phényl-N'-diméthylhydrazine;
N-(4,6-diméthylpyrimidyl-2)-N-phényl-N'-n-propylhydrazine;
N-(4,6-diméthylpyrimidyl-2)-N-phényl-N'-isobutylhydrazine;
N-(4-méthyl-6-méthoxyméthylpyrimidyl-2)-N-phényl-N'-méthylhydrazine ;
N-(4-méthyl-6-méthoxyméthylpyrimidyl-2)-N-phényl-N'-n-propylhydrazine ;
N-(4-méthyl-6-méthoxyméthylpyrimidyl-2)-N-phényl-N'-diméthylhydrazine ;
N-(4-méthyl-6-cyclopropylpyrimidyl-2)-N-phényl-N'-méthylhydrazine ;
N-(4-méthyl-6-cyclopropylpyrimidyl-2)-N-phényl-N'-isobutylhydrazine ;
N-(4-méthyl-6-cyclopropylpyrimidyl-2)-N-phényl-N'-diméthylhydrazine ;
N-(4-méthyl-6-cyclopropylpyrimidyl-2)-N-phényl-N'-diéthylhydrazine ;
N-(4-méthyl-6-cyclopropylpyrimidyl-2)-N-phényl-N'-méthyl-N'-éthylhydrazine ;
N-(4-méthyl-6-cyclopropylpyrimidyl-2)-N-p-fluorophényl-N'-éthylhydrazine ;
N-(4-méthyl-6-méthoxyméthylpyrimidyl-2)-N-m-fluorophényl-N'-isopropylhydrazine.

**12.** Produit pour combattre ou prévenir une attaque par des insectes ou des microorganismes nuisibles, caractérisé en ce qu'il contient, en tant que composant actif, avec un véhicule approprié, au moins un composé de formule I

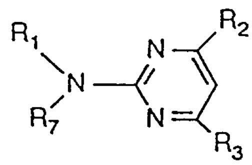

(I)

dans laquelle

$R_1$ représente un groupe phényle ou un groupe phényle portant jusqu'à trois substituants $R_4$ ;

$R_2$ représente l'hydrogène, un groupe alkyle en C1-C5, un groupe alkyle en C1-C5 substitué par $OR_5$ ou $SR_5$, un groupe cycloalkyle en C3-C6, un groupe cycloalkyle en C3-C6 portant jusqu'à trois substituants alkyle en C1-C4 ou halogéno, un groupe alcényle en C2-C5, alcynyle en C2-C5 ou formyle ;

$R_3$ représente l'hydrogène, un groupe alkyle en C1-C4, un groupe alkyle en C1-C4 substitué par des halogènes, des groupes cyano, ou les groupes $OR_5$ ou $SR_5$, un groupe cycloalkyle en C3-C6 ou un groupe cycloalkyle en C3-C6 portant jusqu'à trois substituants alkyle en C1-C4 ou halogéno ;

$R_4$ représente un halogène, un groupe alkyle en C1-C3, halogénoalkyle en C1-C2, alcoxy en C1-C3 ou halogénoalcoxy en C1-C3 ;

$R_5$ représente l'hydrogène, un groupe alkyle en C1-C5, alcényle en C3-C5, alcynyle en C3-C5 ou $(CH_2)_n$-X-alkyle en C1-C3 ;

$R_7$ représente un groupe $-NH_2$,

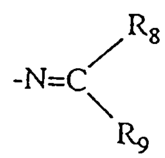

ou

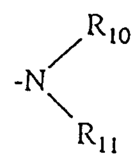

$R_8$ représente l'hydrogène, un groupe alkyle en C1-C3 ou halogénoalkyle en C1-C3 ;

$R_9$ représente l'hydrogène, un groupe alkyle en C1-C8, un groupe alkyle en C1-C3 substitué par des groupes hydroxy, $OR_{12}$, $SR_{12}$ ou $N(R_{12})_2$, un groupe cycloalkyle en C3-C6, un groupe cyclopropyle substitué par $SR_{12}$, un groupe alcényle en C3-C10, halogénoalkyle en C1-C3, 1-, 2- ou 3-pyridyle,

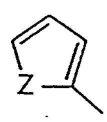

ou

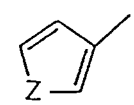

$R_8$ et $R_9$ forment ensemble et avec l'atome de carbone de $R_7$ un cycle saturé ou insaturé consistant en 4 à 7 atomes de carbone ;

$R_{10}$ représente un groupe $CH(R_8)R_9$, un groupe phényle, alcényle en C3-C5, alcynyle en C3-C5 ou cyanalkyle contenant deux ou trois atomes de carbone dans la partie alkyle ;

$R_{11}$ représente l'hydrogène, un groupe alkyle en C1-C5, alcényle en C3-C5, alcynyle en C3-C5 ou cyanalkyle contenant deux ou trois atomes de carbone dans la partie alkyle ;

$R_{12}$ représente $CH_3$ ou $C_2H_5$ ;

X représente l'oxygène ou le soufre ;

Z représente O, S, NH ou $NH_3$ ; et

n est un nombre allant de 1 à 3.

**13.** Produit selon revendication 12, caractérisé en ce qu'il contient, en tant que composant actif, au moins un composé de formule I dans laquelle $R_1$ représente un groupe phényle ou un groupe phényle portant jusqu'à trois substituants $R_4$ ;

$R_2$ représente l'hydrogène, un groupe alkyle en C1-C5, un groupe alkyle en C1-C5 substitué par $OR_5$ ou $SR_5$, un groupe cycloalkyle en C3-C6, un groupe cycloalkyle en C3-C6 portant jusqu'à trois substituants alkyle en C1-C4 ou halogéno, un groupe alcényle en C2-C5, alcynyle en C2-C5 ou formyle ;

$R_3$ représente un groupe alkyle en C1-C4, alkyle en C1-C4 substitué par des halogènes, des groupes cyano ou les groupes $OR_5$ ou $SR_5$, un groupe cycloalkyle en C3-C6 ou un groupe cycloalkyle en C3-C6 portant jusqu'à trois substituants alkyle en C1-C4 ou halogéno ;
$R_4$ représente un halogène, un groupe alkyle en C1-C3, halogénoalkyle en C1-C2, alcoxy en C1-C3 ou halogénoalcoxy en C1-C3 ;
$R_5$ représente l'hydrogène, un groupe alkyle en C1-C5, alcényle en C3-C5, alcynyle en C3-C5 ou $(CH_2)_n$-X-alkyle en C1-C3 ;
$R_7$ représente $-NH_2$ ;
X représente l'oxygène ou le soufre ;
n est un nombre allant de 1 à 3.

**14.** Produit selon la revendication 12, caractérisé en ce qu'il contient, en tant que composant actif, au moins un composé de formule I choisi parmi les suivants :
N-(4-fluorométhyl-6-cyclopropylpyrimidyl-2)-N-phénylhydrazine ;
N-(4-méthyl-6-cyclopropylpyrimidyl-2)-N-m-fluorophénylhydrazine ;
N-(4-méthyl-6-cyclopropylpyrimidyl-2)-N-p-fluorophénylhydrazine.

**15.** Produit selon revendication 12, caractérisé en ce qu'il contient, en tant que composant actif, au moins un composé de formule I choisi parmi les suivants :
N-(4-méthyl-6-cyclopropylpyrimidyl-2)-N-phénylhydrazine;
N-(4,6-diméthylpyrimidyl-2)-N-phénylhydrazine;
N-(4-méthyl-6-méthoxyméthylpyrimidyl-2)-N-phénylhydrazine.

**16.** Produit selon revendication 12, caractérisé en ce qu'il contient, en tant que composant actif, au moins un composé de formule I choisi parmi les suivants :
N-(4,6-diméthylpyrimidyl-2)-N-phényl-propionaldéhydhydrazone ;
N-(4,6-diméthylpyrimidyl-2)-N-phényl-isobutyraldéhydhydrazone ;
N-(4-méthyl-6-méthoxyméthylpyrimidyl-2)-N-phényl-isobutyraldéhydhydrazone ;
N-(4-méthyl-6-hydroxyméthylpyrimidyl-2)-N-phényl-propionaldéhydhydrazone ;
N-(4-méthyl-6-cyclopropylpyrimidyl-2)-N-phényl-propionaldéhydhydrazone ;
N-(4-méthyl-6-cyclopropylpyrimidyl-2)-N-phényl-n-bulyraldéhydhydrazone ;
N-(4-méthyl-6-cyclopropylpyrimidyl-2)-N-phényl-isobutyraldéhydhydrazone ;
N-(4-méthyl-6-cyclopropylpyrimidyl-2)-N-phényl-trichloracétaldéhydhydrazone ;
N-(4-méthyl-6-cyclopropylpyrimidyl-2)-N-p-fluorophényl-acétaldéhydhydrazone ;
N-(4-méthyl-6-cyclopropylpyrimidyl-2)-N-p-fluorophényl-isobutyraldéhydhydrazone ;
N-(4-méthyl-6-cyclopropylpyrimidyl-2)-N-m-fluorophényl-isobutyraldéhydhydrazone ;
N-(4,6-diméthylpyrimidyl-2)-N-phényl-N'-méthylhydrazine;
N-(4,6-diméthylpyrimidyl-2)-N-phényl-N'-diméthylhydrazine;
N-(4,6-diméthylpyrimidyl-2)-N-phényl-N'-n-propylhydrazine;
N-(4,6-diméthylpyrimidyl-2)-N-phényl-N'-isobutylhydrazine;
N-(4-méthyl-6-méthoxyméthylpyrimidyl-2)-N-phényl-N'-méthylhydrazine ;
N-(4-méthyl-6-méthoxyméthylpyrimidyl-2)-N-phényl-N'-n-propylhydrazine ;
N-(4-méthyl-6-méthoxyméthylpyrimidyl-2)-N-phényl-N'-diméthylhydrazine ;
N-(4-méthyl-6-cyclopropylpyrimidyl-2)-N-phényl-N'-méthylhydrazine ;
N-(4-méthyl-6-cyclopropylpyrimidyl-2)-N-phényl-N'-isobutylhydrazine ;
N-(4-méthyl-6-cyclopropylpyrimidyl-2)-N-phényl-N'-diméthylhydrazine ;
N-(4-méthyl-6-cyclopropylpyrimidyl-2)-N-phényl-N'-diéthylhydrazine ;
N-(4-méthyl-6-cyclopropylpyrimidyl-2)-N-phényl-N'-méthyl-N'-éthylhydrazine ;
N-(4-méthyl-6-cyclopropylpyrimidyl-2)-N-p-fluorophényl-N'-éthylhydrazine ;
N-(4-méthyl-6-méthoxyméthylpyrimidyl-2)-N-m-fluorophényl-N'-isopropylhydrazine.

**17.** Procédé pour combattre ou prévenir une attaque de végétaux cultivés par des insectes nuisibles ou des microorganismes phytopathogènes, caractérisé en ce que l'on applique sur les végétaux, des parties des végétaux ou leur lieu de plantation, en tant que substance active, un composé de formule I préparé selon revendication 1.

**18.** Procédé selon revendication 17, caractérisé en ce que l'on applique, en tant que substance active, un composé préparé selon une des revendications 2 à 11.

**19.** Procédé selon revendication 17, caractérisé en ce que l'on combat des mycètes phytopathogènes.

20. Procédé pour la préparation d'un produit agrochimique selon revendication 12, caractérisé en ce que l'on mélange intimement au moins un composé de formule I préparé selon revendication 1 avec des additifs et/ou agents tensio-actifs solides ou liquides appropriés.